(19) Europäisches Patentamt
European Patent Office
Office européen des brevets

(11) EP 2 406 224 B1

(12) **EUROPÄISCHE PATENTSCHRIFT**

(45) Veröffentlichungstag und Bekanntmachung des Hinweises auf die Patenterteilung:
**23.04.2014 Patentblatt 2014/17**

(21) Anmeldenummer: **10708140.8**

(22) Anmeldetag: **11.03.2010**

(51) Int Cl.:
***C07D 213/75*** (2006.01) ***C07D 409/12*** (2006.01)
***A61K 31/44*** (2006.01) ***A61K 31/4427*** (2006.01)
***A61P 25/08*** (2006.01)

(86) Internationale Anmeldenummer:
**PCT/EP2010/001508**

(87) Internationale Veröffentlichungsnummer:
**WO 2010/102810 (16.09.2010 Gazette 2010/37)**

(54) **SUBSTITUIERTE 2-MERCAPTO-3-AMINOPYRIDINE ALS KCNQ2/3 MODULATOREN**

SUBSTITUTED 2-MERCAPTO-3-AMINOPYRIDINES AS KCNQ2/3 MODULATORS

2-MERCAPTO-3-AMINOPYRIDINES SUBSTITUÉS EN TANT QUE MODULATEURS DE KCNQ2/3

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **12.03.2009 EP 09003604**

(43) Veröffentlichungstag der Anmeldung:
**18.01.2012 Patentblatt 2012/03**

(73) Patentinhaber: **Grünenthal GmbH**
**52078 Aachen (DE)**

(72) Erfinder:
- **KÜHNERT, Sven**
**52355 Düren (DE)**
- **MERLA, Beatrix**
**52078 Aachen (DE)**
- **KLESS, Achim**
**52072 Aachen (DE)**
- **BAHRENBERG, Gregor**
**52156 Monschau-Konzen (DE)**
- **SCHRÖDER, Wolfgang**
**52074 Aachen (DE)**

(56) Entgegenhaltungen:
**WO-A-2004/058704 WO-A-2006/092143**
**WO-A-2009/018466**

- **DATABASE CAPLUS [Online] CHEMICAL ABSTRACTS SERVICE, COLUMBUS, OHIO, US; 1981, TRAVEN, N. I. ET AL: "Study of nitrogen- and sulfur-containing heterocycles. 39. Reactions of 2-mercapto-3-ureido- and 2-mercapto-3-aminopyridines with .beta.-halocarbonyl compounds. Synthesis of 6-oxo derivatives of pyrido[2,3-b][1,5] thiazepines" XP002577875 gefunden im STN Database accession no. 1981:550615 & KHIMIYA GETEROTSIKLICHESKIKH SOEDINENII , (5), 634-9 CODEN: KGSSAQ; ISSN: 0453-8234, 1981,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 406 224 B1

## Beschreibung

Die Erfindung betrifft substituierte 2-Mercapto-3-aminopyridine, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Übererregbarkeit von Neuronen. Die neuronale Erregbarkeit wird entscheidend von der Aktivität von $K^+$ Kanälen beeinflusst, da diese das Ruhemembranpotential der Zelle und somit die Erregbarkeitsschwelle maßgeblich bestimmen. Heteromere $K^+$ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 $K^+$ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., J Neurosci. 2003; 23(18):7227-36).

Dementsprechend konnte für den KCNQ2/3 Agonisten Retigabine eine analgetische Wirksamkeit in präklinischen Neuropathie- und Entzündungsschmerzmodellen nachgewiesen werden (Blackburn-Munro and Jensen, Eur J Pharmacol. 2003; 460(2-3):109-16; Dost et al., Naunyn Schmfedebergs Arch Pharmacol 2004; 369(4): 382-390).

Der KCNQ2/3 $K^+$ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., Eur J Pharmacol. 2004; 487(1-3): 93-103), insbesondere von neuropathischem und inflammatorischem Schmerz dar.

Darüber hinaus ist der KCNQ2/3 $K^+$ Kanal ein geeignetes Target für die Therapie einer Vielzahl weiterer Erkrankungen wie beispielsweise Migräne (US2002/0128277), kognitive Erkrankungen (Gribkoff, Expert Opin Ther Targets 2003; 7(6): 737-748), Angstzuständen (Korsgaard et al., J Pharmacol Exp Ther. 2005, 14(1): 282-92), Epilepsie (Wickenden et al., Expert Opin Ther Pat 2004, 14(4): 457-469; Gribkoff, Expert Opin Ther Targets 2008, 12(5): 565-81; Miceli et al., Curr Opin Pharmacol 2008, 8(1): 65-74), Harninkontinenz (Streng et al., J Urol 2004; 172: 2054-2058), Abhängigkeit (Hansen et al., Eur J Pharmacol 2007, 570(1-3): 77-88), Manie/bipolare Störungen (Dencker et al., Epilepsy Behav 2008, 12(1): 49-53), Dystonie-assoziierte Dyskinesien (Richter et al., Br J Pharmacol 2006, 149(6): 747-53).

Aus dem Stand der Technik sind substituierte Pyridin-Derivate (WO 2006/092143), substituierte Naphthyridin - Derivate (WO 2009/018466), Chinazolinon - Derivate (WO 2004/058704) substituierte Heteroaryl-Verbindungen (WO 01/010380) sowie stickstoffhaltige Heteroaryl-Derivate (WO 2006/051311) bekannt, die eine Affinität zum KCNQ. Rezeptor aufweisen.

Es besteht ein Bedarf an weiteren Verbindungen mit vergleichbaren oder besseren Eigenschaften, nicht nur im Hinblick auf die Affinität an KCNQ2/3 als solche (*potency, efficacy*).

So kann es vorteilhaft sein, die metabolische Stabilität, die Löslichkeit in wässrigen Medien oder die Permeabilität der Verbindungen zu verbessern. Diese Faktoren können sich günstig auf die orale Bioverfügbarkeit auswirken oder können das PK/PD (Pharmakokinetik/Pharmakodynamik)-Profil verändern, was z.B. zu einer günstigeren Wirkdauer führen kann.

Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner kann es von Vorteil sein, wenn die Verbindungen eine hohe Selektivität gegenüber anderen Rezeptoren der KCNQ-Familie zeigen (Spezifität), beispielsweise gegenüber KCNQ1, KCNQ3/5 oder KCNQ4. Eine hohe Selektivität kann sich günstig auf das Nebenwirkungsprofil auswirken. So ist beispielsweise bekannt, dass Verbindungen, welche (auch) an KCNQ1 binden, ein hohes Risiko kardialer Nebenwirkungen mit sich bringen, weshalb eine hohe Selektivität gegenüber KCNQ1 wünschenswert sein kann. Eine hohe Selektivität kann jedoch auch gegenüber anderen Rezeptoren von Vorteil sein. Eine geringe Affinität zum hERG-Ionenkanal oder zum L-Typ Calcium-Ionenkanal (Phenylalkylamin-, Benzothiazepin-, Dihydropyridin-Bindungsstellen) kann vorteilhaft sein, da diese Rezeptoren in Zusammenhang mit dem Auftreten kardialer Nebenwirkungen gebracht werden. Insgesamt kann eine verbesserte Selektivität bezüglich der Bindung an andere endogene Proteine (d.h. z.B. Rezeptoren oder Enzyme) zu einer Verbesserung des Nebenwirkungsprofils, und dadurch zu einer verbesserten Verträglichkeit führen.

Eine Aufgabe der Erfindung bestand daher darin, neue Verbindungen zur Verfügung zu stellen, welche Vorteile gegenüber den Verbindungen des Standes der Technik aufweisen. Die Verbindungen sollten sich insbesondere als pharmakologische Wirkstoffe in Arzneimitteln eignen, vorzugsweise in Arzneimitteln zur Behandlung von Störungen oder Krankheiten, welche zumindest teilweise durch KCNQ2/3 $K^+$ Kanäle vermittelt werden.

Diese Aufgabe wird durch den Gegenstand der Patentansprüche gelöst.

Es wurde überraschend gefunden, dass sich substituierte 2-Mercapto-3-aminopyridine der nachstehend angegebenen allgemeinen Formel (1) zur Behandlung von Schmerzen eignen. Es wurde ferner überraschend gefunden, dass substituierte 2-Mercapto-3-aminopyridine der nachstehend angegebenen allgemeinen Formel (1) auch eine ausgezeichnete Affinität zum KCNQ2/3 $K^+$ Kanal aufweisen und sich daher zur Behandlung von Störungen oder Krankheiten eignen, die zumindest teilweise durch KCNQ2/3 $K^+$ Kanäle vermittelt werden. Dabei wirken die substituierten 2-Mercapto-3-aminopyridine als Modulatoren, d.h. Agonisten oder Antagonisten, des KCNQ2/3 $K^+$ Kanals.

Ein Gegenstand der Erfindung sind substituierte 2-Mercapto-3-aminopyridine der allgemeinen Formel (1)

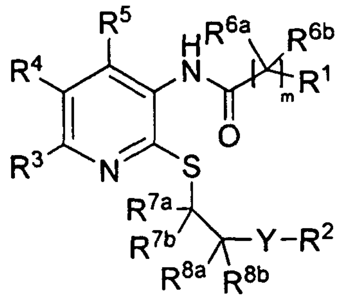

**(1)** ,

worin

m für 0, 1, 2 oder 3 steht;

$R^1$ für $C_{1-6}$-Alkyl, gesättigt oder ungesättigt, verzweigt oder unverzweigt, unsubstituiert oder einfach oder mehrfach substituiert; $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert steht;

mit der Maßgabe, dass, wenn $R^1$ Heterocyclyl bedeutet, die Bindung des Heterocyclyls an die übergeordnete allgemeine Struktur über ein Kohlenstoffatom des Heterocyclyls erfolgt;

$R^2$ für Aryl oder Heteroaryl, jeweils unsubstituiert oder einfach oder mehrfach substituiert steht;

Y ausgewählt ist aus der Gruppe bestehend aus -($CR^{9a}R^{9b}$)-, $S(=O)_2$, S(=O), -S-, -O-, C(=O);

$R^3$, $R^4$, $R^5$, $R^{6a}$, $R^{6b}$, $R^{7a}$, $R^{7b}$, $R^{8a}$, $R^{8b}$, $R^{9a}$ und $R^{9b}$ jeweils unabhängig voneinander für H; F; Cl; Br; I; $NO_2$; $CF_3$; CN; OH; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ oder C(=O)-$C_{1-6}$-Alkyl, jeweils gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, verzweigt oder unverzweigt, unsubstituiert oder einfach oder mehrfach substituiert; stehen,

wobei $R^{7a}$ mit $R^{8a}$ einen $C_{3-7}$Cycloalkylrest bilden können, der gesättigt oder ungesättigt, unsubstituiert oder einfach oder mehrfach substituiert sein kann;

worin "Alkyl substituiert" für die Substitution eines oder mehrerer Wasserstoffatome jeweils unabhängig voneinander durch F; Cl; Br; I; $NO_2$; $CF_3$; CN; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; $S(=O)_2C_{1-8}$-Alkyl; $S(=O)_2$Aryl; $S(=O)_2$Heteroaryl; $S(=O)_2OH$; $S(=O)_2O$-$C_{1-8}$-Alkyl; $S(=O)_2O$-Aryl; $S(=O)_2O$-Heteroaryl; $S(=O)_2$-NH-$C_{1-8}$-Alkyl; $S(=O)_2$-NH-Aryl; und $S(=O)_2$-NH-$C_{1-8}$-Heteroaryl; steht;

worin "Heterocyclyl substituiert" und "Cycloalkyl substituiert" für die Substitution eines oder mehrerer Wasserstoffatome jeweils unabhängig voneinander durch F; Cl; Br; I; $NO_2$; $CF_3$; =O; CN; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder

Heterocyclyl; CHO; $C(=O)C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; 0-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; und S(=O)$_2$-NH-$C_{1-8}$-Heteroaryl; steht;
worin "Aryl substituiert" und "Heteroaryl substituiert" für die Substitution eines oder mehrerer Wasserstoffatome jeweils unabhängig voneinander durch F; Cl; Br; I; $NO_2$; $CF_3$; CN; $C_{1-8}$-Alkyl; oder $C_{2-8}$-Heteroalkyl; Aryl; Heteroaryl; $C_{3-10}$-Cycloalkyl; Heterocyclyl; $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$CH_3$; C(=O)NH-$C_2H_5$; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; S(=O)$_2$-NH-$C_{1-8}$-Heteroaryl; steht;
in Form der freien Verbindungen oder Salze physiologisch verträglicher Säuren oder Basen.

Die Ausdrücke "Alkyl" bzw. "$C_{1-10}$-Alkyl", "$C_{1-8}$-Alkyl", "$C_{1-6}$-Alkyl", "$C_{1-4}$-Alkyl", "$C_{2-10}$-Alkyl", " $C_{2-8}$-Alkyl" und "$C_{4-10}$-Alkyl" umfassen im Sinne dieser Erfindung acyclische gesättigte oder ungesättigte aliphatische Kohlenwasserstoffreste, die verzweigt oder unverzweigt sowie unsubstituiert oder ein- oder mehrfach substituiert sein können, mit 1 bis 10 bzw. 1 bis 8 bzw. 1 bis 6 bzw. 1 bis 4 bzw. 2 bis 10 bzw. 2 bis 8 bzw. 4 bis 10 C-Atomen d.h. $C_{1-10}$-Alkanyle, $C_{2-10}$-Alkenyle und $C_{2-10}$-Alkinyle bzw. $C_{1-8}$-Alkanyle, $C_{2-8}$-Alkenyle und $C_{2-8}$-Alkinyle bzw. $C_{1-6}$-Alkanyle, $C_{2-6}$-Alkenyle und $C_{2-6}$-Alkinyle bzw. $C_{1-4}$-Alkanyle, $C_{2-4}$-Alkenyle und $C_{2-4}$-Alkinyle bzw. $C_{2-10}$-Alkanyle, $C_{2-10}$-Alkenyle und $C_{2-10}$-Alkinyle bzw. $C_{2-8}$-Alkanyle, $C_{2-8}$-Alkenyle und $C_{2-8}$-Alkinyle bzw. $C_{4-10}$-Alkanyle, $C_{4-10}$-Alkenyle und $C_{4-10}$-Alkinyle. Dabei weisen Alkenyle mindestens eine C-C-Doppelbindung und Alkinyle mindestens eine C-C-Dreifachbindung auf. Bevorzugt ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, n-Heptyl, n-Octyl, n-Nonyl, n-Decyl, Ethenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $-C\equiv C-CH_3$), Butenyl, Butinyl, Pentenyl, Pentinyl, Hexenyl und Hexinyl, Heptenyl, Heptinyl, Octenyl, Octinyl, Nonenyl, Noninyl, Decenyl und Decinyl umfasst.

Die Ausdrücke "Cycloalkyl" bzw. "$C_{3-10}$-Cycloalkyl" und "$C_{3-7}$-Cycloalkyl" oder bedeuten für die Zwecke dieser Erfindung cyclische aliphatische Kohlenwasserstoffe mit 3, 4, 5, 6, 7, 8, 9 oder 10 bzw. mit 3, 4, 5, 6 oder 7 Kohlenstoffatomen, wobei die Kohlenwasserstoffe gesättigt oder ungesättigt (aber nicht aromatisch), unsubstituiert oder ein- oder mehrfach substituiert sein können. Die Bindung des Cycloalkyls an die jeweilige übergeordnete allgemeine Struktur kann über jedes beliebige und mögliche Ringglied des Cycloalkyl-Restes erfolgen. Die Cycloalkyl-Reste können auch mit weiteren gesättigten, (partiell) ungesättigten, (hetero)cyclischen, aromatischen oder heteroaromatischen Ringsystemen , d.h. mit Cycloalkyl, Heterocyclyl, Aryl oder Heteroaryl kondensiert sein, die wiederum unsubstituiert oder einfach oder mehrfach substituiert sein können. Beispiele für solche Ringssyteme sind Chromanyl, Tetrahydronaphthyl und Decahydronaphthyl. Die Cycloalkyl-Reste können weiterhin einfach oder mehrfach verbrückt sein wie bspw. im Fall von Adamantyl, Bicyclo[2.2.1]heptyl oder Bicyclo[2.2.2]octyl. Bevorzugt ist Cycloalkyl aus der Gruppe ausgewählt, die Cyclopropyl, Cyclobutyl, Cyclopentyl, Cyclohexyl, Cycloheptyl, Cyclooctyl, Cyclononyl, Cyclodecyl, Adamantyl,

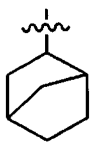 , 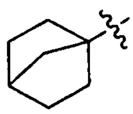 , 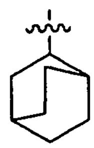 , 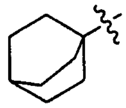 ,

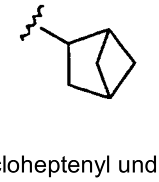 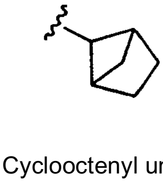 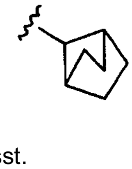 ,

Cyclopentenyl, Cyclohexenyl, Cycloheptenyl und Cyclooctenyl umfasst.

Der Begriff "Heterocyclyl" oder "Heterocycloalkyl" umfasst aliphatische gesättigte oder ungesättigte (aber nicht aromatische) Cycloalkyle mit drei bis zehn, d.h. 3, 4, 5, 6, 7, 8, 9 oder 10 Ringgliedern, in denen mindestens ein, ggf. auch zwei oder drei Kohlenstoffatome durch ein Heteroatom oder eine Heteroatomgruppe jeweils unabhängig voneinander ausgewählt aus der Gruppe bestehend aus O, S, N, NH und N($C_{1-8}$-Alkyl), vorzugsweise N($CH_3$) ersetzt sind, wobei die Ringglieder unsubstituiert oder ein- oder mehrfach substituiert sein können. Die Bindung des Heterocyclyls an die übergeordnete allgemeine Struktur kann über jedes beliebige und mögliche Ringglied des Heterocyclyl-Restes erfolgen. Die Heterocyclyl-Reste können auch mit weiteren gesättigten, (partiell) ungesättigten (hetero)cyclischen oder aromatischen oder heteroaromatischen Ringsystemen, d.h. mit Cycloalkyl, Heterocyclyl, Aryl oder Heteroaryl kondensiert sein, die wiederum unsubstituiert oder einfach oder mehrfach substituiert sein können. Bevorzugt sind Heterocyclyl-Reste aus der Gruppe umfassend Azetidinyl, Aziridinyl, Azepanyl, Azocanyl, Diazepanyl, Dithiolanyl, Dihydrochinolinyl, Dihydropyrrolyl, Dioxanyl, Dioxolanyl, Dihydroindenyl Dihydropyridinyl, Dihydrofuranyl, Dihydroisochinolinyl, Dihydroindolinyl, Dihydroisoindolyl, Imidazolidinyl, Isoxazolidinyl, Morpholinyl, Oxiranyl, Oxetanyl, Pyrrolidinyl, Piperazinyl, Piperidinyl, Pyrazolidinyl, Pyranyl, Tetrahydropyrrolyl, Tetrahydropyranyl, Tetrahydrochinolinyl, Tetrahydroisochinolinyl, Tetrahydroindolinyl, Tetrahydrofuranyl, Tetrahydropyridinyl, Tetrahydrothiophenyl, Tetrahydropyridoindolyl, Tetrahydronaphthyl, Tetrahydrocarbolinyl, Tetrahydroisoxazolopyridinyl, Thiazolidinyl und Thiomorpholinyl.

Der Begriff "Aryl" bedeutet im Sinne dieser Erfindung aromatische Kohlenwasserstoffe mit bis zu 14 Ringgliedern, u.a. Phenyle und Naphthyle. Jeder Aryl-Rest kann unsubstituiert oder einfach oder mehrfach substituiert vorliegen, wobei die ArylSubstituenten gleich oder verschieden und in jeder beliebigen und möglichen Position des Aryls sein können. Die Bindung des Aryls an die übergeordnete allgemeine Struktur kann über jedes beliebige und mögliche Ringglied des ArylRestes erfolgen. Die Aryl-Reste können auch mit weiteren gesättigten, (partiell) ungesättigten, (hetero)cyclischen, aromatischen oder heteroaromatischen Ringsystemen kondensiert sein, d.h. mit Cycloalkyl, Heterocyclyl, Aryl oder Heteroaryl, die wiederum unsubstituiert oder einfach oder mehrfach substituiert sein können. Beispiele für kondensierte Aryl-Reste sind Benzodioxolanyl und Benzodioxanyl. Bevorzugt ist Aryl aus der Gruppe ausgewählt, die Phenyl, 1-Naphthyl und 2-Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

Der Begriff "Heteroaryl" steht für einen 5- oder 6-gliedrigen cyclischen aromatischen Rest, der mindestens 1, ggf. auch 2, 3, 4 oder 5 Heteroatome, enthält, wobei die Heteroatome jeweils unabhängig voneinander ausgewählt sind aus der Gruppe S, N und O und der Heteroaryl-Rest unsubstituiert oder ein- oder mehrfach substituiert sein kann; im Falle der Substitution am Heteroaryl können die Substituenten gleich oder verschieden sein und in jeder beliebigen und möglichen Position des Heteroaryls sein. Die Bindung an die übergeordnete allgemeine Struktur kann über jedes beliebige und mögliche Ringglied des Heteroaryl-Restes erfolgen. Das Heteroaryl kann auch Teil eines bi- oder polycyclischen Systems mit bis zu 14 Ringgliedern sein, wobei das Ringsystem mit weiteren gesättigten, (partiell) ungesättigten, (hetero)cyclischen oder aromatischen oder heteroaromatischen Ringen gebildet werden kann, d.h. mit Cycloalkyl, Heterocyclyl, Aryl oder Heteroaryl, die wiederum unsubstituiert oder einfach oder mehrfach substituiert sein können. Es ist bevorzugt, dass der Heteroaryl-Rest ausgewählt ist aus der Gruppe, die Benzofuranyl, Benzoimidazolyl, Benzothienyl, Benzothiadiazolyl, Benzothiazolyl, Benzotriazolyl, Benzooxazolyl, Benzooxadiazolyl, Chinazolinyl, Chinoxalinyl, Carbazolyl, Chinolinyl, Dibenzofuranyl, Dibenzothienyl, Furyl (Furanyl), Imidazolyl, Imidazothiazolyl, Indazolyl, Indolizinyl, Indolyl, Isochinolinyl, Isoxazoyl, Isothiazolyl, Indolyl, Naphthyridinyl, Oxazolyl, Oxadiazolyl, Phenazinyl, Phenothiazinyl, Phtalazinyl, Pyrazolyl, Pyridyl (2-Pyridyl, 3-Pyridyl, 4-Pyridyl), Pyrrolyl, Pyridazinyl, Pyrimidinyl, Pyrazinyl, Purinyl, Phenazinyl, Thienyl (Thiophenyl), Triazolyl, Tetrazolyl, Thiazolyl, Thiadiazolyl und Triazinyl umfasst. Besonders bevorzugt sind Furyl, Pyridyl und Thienyl.

Im Zusammenhang mit "Alkyl" versteht man unter dem Begriff "ein- oder mehrfach substituiert" im Sinne dieser Erfindung die ein- oder mehrfache, z.B. zwei-, drei- oder vierfache Substitution eines oder mehrerer Wasserstoffatome jeweils unabhängig voneinander durch Substituenten ausgewählt aus der Gruppe aus F; Cl; Br; I; $NO_2$; $CF_3$; CN; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl, $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$ ; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2$$C_{1-8}$-Alkyl; S(=O)$_2$Aryl; S(=O)$_2$Heteroaryl; S(=O)$_2$OH; S(=O)$_2$O.$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; und S(=O)$_2$-NH-$C_{1-8}$-Heteroaryl; wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise dreifach am gleichen C-Atom wie im Falle von $CF_3$ oder $CH_2CF_3$ oder an verschiedenen Stellen wie im Falle von CH(OH)-CH=CH-$CHCl_2$. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Alkyl" Substituenten sind ausgewählt aus der Gruppe aus F; Cl; Br; I; $NO_2$; $CH_2CF_3$; $CF_3$; CN; $C_{1-8}$-Alkyl; $C_{2-8}$-Heteroalkyl; Phenyl; Naphthyl; Pyridyl; Thienyl; Furyl; $C_{3-10}$-Cycloalkyl; Heterocyclyl; $C_{1-8}$-Alkyl oder $C_{2-8}$-Heteroalkyl verbrücktes Phenyl, Naphthyl, Pyridyl, Thienyl, Furyl, $C_{3-10}$-Cycloalkyl oder Heterocyclyl; CHO; C(=O)$C_{1-8}$-Alkyl, $CO_2H$; C(=O)O-$C_{1-8}$-Alkyl; $CONH_2$; C(=O)NH-$C_{1-8}$-Alkyl; C(=O)N($C_{1-8}$-Alkyl)$_2$; OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Phenyl; O-Heteroaryl; O-C(=O)$C_{1-8}$-Alkyl; $NH_2$ ; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; SH; S-$C_{1-8}$-Alkyl; $SCF_3$; S-Benzyl; S-Phenyl; S-Heteroaryl; S(=O)$_2C_{1-8}$-Alkyl;; S(=O)$_2$OH; S(=O)$_2$O-$C_{1-8}$-Alkyl; S(=O)$_2$-NH-$C_{1-8}$-Alkyl.

Im Zusammenhang mit "Heterocyclyl substituiert" und "Cycloalkyl substituiert" versteht man unter dem Begriff "ein- oder mehrfach substituiert" im Sinne dieser Erfindung die ein- oder mehrfache, z.B. zwei-, drei- oder vierfache Substitution eines oder mehrerer Wasserstoffatome jeweils unabhängig voneinander durch Substituenten ausgewählt aus der Gruppe aus F; Cl; Br; I; $NO_2$; $CF_3$; =O; CN; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; und S(=O)$_2$-NH-$C_{1-8}$-Heteroaryl; wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise zweifach am gleichen C-Atom wie im Falle von 1,1-Difluorcyclohexyl oder an verschiedenen Stellen wie im Falle von 1,2-Difluorcyclohexyl. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Heterocyclyl-" und "Cycloalkyl-" Substituenten sind ausgewählt aus der Gruppe aus F; Cl; Br; I; $NO_2$; $CH_2CF_3$; $CF_3$; CN; $C_{1-8}$-Alkyl; $C_{2-8}$-Heteroalkyl; Phenyl; Naphthyl; Pyridyl; Thienyl; Furyl; $C_{3-10}$-Cycloalkyl; Heterocyclyl; $C_{1-8}$-Alkyl oder $C_{2-8}$-Heteroalkyl verbrücktes Phenyl, Naphthyl, Pyridyl, Thienyl, Furyl, $C_{3-10}$-Cycloalkyl oder Heterocyclyl; CHO; C(=O)$C_{1-8}$-Alkyl; $CO_2H$; C(=O)O-$C_{1-8}$-Alkyl, $CONH_2$; C(=O)NH-$C_{1-8}$-Alkyl; C(=O)N($C_{1-8}$-Alkyl)$_2$; OH; =O; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Phenyl; O-Heteroaryl; 0-C(=O)$C_{1-8}$-Alkyl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; SH; S-$C_{1-8}$-Alkyl; $SCF_3$; S-Benzyl; S-Phenyl; S-Heteroaryl; S(=O)$_2C_{1-8}$-Alkyl;; S(=O)$_2$OH; S(=O)$_2$O-$C_{1-8}$-Alkyl; S(=O)$_2$-NH-$C_{1-8}$-Alkyl.

Im Zusammenhang mit "Aryl" und "Heteroaryl" versteht man im Sinne dieser Erfindung unter "ein- oder mehrfach substituiert" die ein- oder mehrfache, z.B. zwei-, drei- oder vierfache Substitution eines oder mehrerer Wasserstoffatome des Ringsystems jeweils unabhängig voneinander durch Substituenten ausgewählt aus der Gruppe aus F; Cl; Br; I; $NO_2$; $CF_3$; CN; $C_{1-8}$-Alkyl; oder $C_{2-8}$-Heteroalkyl; Aryl; Heteroaryl; $C_{3-10}$-Cycloalkyl; Heterocyclyl; $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$CH_3$; C(=O)NH-$C_2H_5$; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; S(=O)$_2$-NH-$C_{1-8}$-Heteroaryl; an einem oder ggf. verschiedenen Atomen, wobei ein Substituent ggf. seinerseits wiederum einfach oder mehrfach substituiert sein kann. Die Mehrfachsubstitution erfolgt mit dem gleichen oder mit unterschiedlichen Substituenten.

Bevorzugte "Aryl-" und "Heteroaryl-" Substituenten sind F; Cl; Br; I; $NO_2$; $CH_2CF_3$; $CF_3$; CN; $C_{1-8}$-Alkyl; $C_{2-8}$-Heteroalkyl; Phenyl; Naphthyl; Pyridyl; Thienyl; Furyl; $C_{3-10}$-Cycloalkyl; Heterocyclyl; $C_{1-8}$-Alkyl oder $C_{2-8}$-Heteroalkyl verbrücktes Phenyl, Naphthyl, Pyridyl, Thienyl, Furyl, $C_{3-10}$-Cycloalkyl oder Heterocyclyl; CHO; C(=O)$C_{1-8}$-Alkyl; $CO_2H$; C(=O)O-$C_{1-8}$-Alkyl; $CONH_2$; C(=O)NH-$CH_3$; C(=O)NH-$C_2H_5$; C(=O)N($C_{1-8}$-Alkyl)$_2$; OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Phenyl; O-Heteroaryl; O-C(=O)$C_{1-8}$-Alkyl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; SH; S-$C_{1-8}$-Alkyl; $SCF_3$; S-Benzyl; S-Phenyl; S-Heteroaryl; S(=O)$_2C_{1-8}$-Alkyl;; S(=O)$_2$OH; S(=O)$_2$O-$C_{1-8}$-Alkyl; S(=O)$_2$-NH-$C_{1-8}$-Alkyl.

Die erfindungsgemäßen Verbindungen sind durch Substituenten definiert, beispielsweise durch $R^1$, $R^2$ und $R^3$ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Gene-

ration). Ist beispielsweise $R^1$ = Aryl (Substituent der 1. Generation), so kann Aryl seinerseits substituiert sein, z.B. mit $C_{1-8}$-Alkyl (Substituent der 2. Generation). Es ergibt sich daraus die funktionelle Gruppe Aryl-$C_{1-8}$-Alkyl. $C_{1-8}$-Alkyl kann dann seinerseits erneut substituiert sein, z.B. mit Cl (Substituent der 3. Generation). Es ergibt sich daraus dann insgesamt die funktionelle Gruppe Aryl-$C_{1-8}$-Alkyl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform bspw. im Fall der allgemeinen Formel (1) die funktionellen Gruppen für $R^1$ bis $R^5$ jeweils ggf. substituiert sein, die jeweiligen Substituenten können dann ihrerseits jedoch nicht erneut substituiert sein.

In einigen Fällen sind die erfindungsgemäßen Verbindungen durch Substituenten definiert, die einen Aryl- oder Heteroaryl-Rest darstellen oder tragen, jeweils unsubstituiert oder einfach oder mehrfach substituiert oder die zusammen mit dem oder den sie verbindenden Kohlenstoff- oder Heteroatom(en) als Ringglied oder als Ringgliedern einen Ring bilden, beispielsweise ein Aryl oder Heteroaryl, jeweils unsubstituiert oder einfach oder mehrfach substituiert. Sowohl diese Aryl- oder Heteroaryl-Reste als auch die so gebildeten aromatischen Ringsysteme können ggf. mit $C_{3-10}$-Cycloalkyl oder Heterocyclyl, jeweils gesättigt oder ungesättigt, kondensiert sein, d.h. mit einem $C_{3-10}$-Cycloalkyl wie Cyclopentyl oder einem Heterocyclyl wie Morpholinyl, wobei die so kondensierten $C_{3-10}$-Cycloalkyl- oder Heterocyclyl-Reste ihrerseits jeweils unsubstituiert oder einfach oder mehrfach substituiert sein können.

In einigen Fällen sind die erfindungsgemäßen Verbindungen durch Substituenten definiert, die einen $C_{3-10}$-Cycloalkyl- oder Heterocyclyl-Rest darstellen oder tragen, jeweils unsubstituiert oder einfach oder mehrfach substituiert oder die zusammen mit dem oder den sie verbindenden Kohlenstoff- oder Heteroatom(en) als Ringglied oder als Ringgliedern einen Ring bilden, beispielsweise ein $C_{3-10}$-Cycloalkyl oder Heterocyclyl, jeweils unsubstituiert oder einfach oder mehrfach substituiert. Sowohl diese $C_{3-10}$-Cycloalkyl oder Heterocyclyl-Reste als auch die gebildeten aliphatischen Ringsysteme können ggf. mit Aryl oder Heteroaryl kondensiert sein, d.h. mit einem Aryl wie Phenyl oder einem Heteroaryl wie Pyridyl, wobei die so kondensierten Aryl- oder Heteroaryl-Reste ihrerseits jeweils unsubstituiert oder einfach oder mehrfach substituiert sein können.

Im Rahmen der vorliegenden Erfindung bezeichnet das in Formeln verwendete Symbol

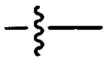

eine Verknüpfung eines entsprechenden Restes an die jeweilige übergeordnete allgemeine Struktur.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure, Phosphorsäure und/oder Asparaginsäure. Besonders bevorzugt sind die Zitronensäure und die Salzsäure.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

In einer bevorzugten Ausführungsform der erfindungsgemäßen Verbindungen steht m für 0 oder 1, besonders bevorzugt für 1.

In einer ebenfalls bevorzugten Ausführungsform der erfindungsgemäßen Verbindungen steht der Rest $R^1$ für

$C_{1-6}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CF_3$, CN, OH, $OCF_3$, C(=O)-OH, SH, S-$C_{1-8}$-Alkyl, $SCF_3$, $S(=O)_2OH$, $NH_2$, $C_{1-8}$-Alkyl, O-$C_{1-8}$-Alkyl, NH-$C_{1-8}$-Alkyl, N($C_{1-8}$-Alkyl)$_2$, wobei die vorstehenden Alkyl-Reste jeweils wiederum gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus F, Cl, Br, I, O-$C_{1-8}$-Alkyl, OH und $OCF_3$;

$C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CH_2CF_3$, $CF_3$, CN, CHO, $CO_2H$, SH, $SCF_3$, $S(=O)_2OH$, OH, =O, $OCF_3$,$NH_2$, C(=O)-$NH_2$, $C_{1-8}$-Alkyl, $C_{2-8}$-Heteroalkyl, NH-$C_{1-8}$-Alkyl, N($C_{1-8}$-Alkyl)$_2$, O-$C_{1-8}$-Alkyl, C(=O)$C_{1-8}$-Alkyl, C(=O)O-$C_{1-8}$-Alkyl, O-C(=O)$C_{1-8}$-Alkyl, C(=O)NH-$C_{1-8}$-Alkyl, C(=O)N($C_{1-8}$-Alkyl)$_2$, NH-C(=O)$C_{1-8}$-Alkyl, N($C_{1-8}$-Alkyl)-C(=O)$C_{1-8}$-Alkyl, N(C(=O)$C_{1-8}$-Alkyl)$_2$, S-$C_{1-8}$-Alkyl, $S(=O)_2O$-$C_{1-8}$-Alkyl, Benzyl, Phenyl, Pyridyl, Thienyl und Furyl; wobei die vorstehenden Alkyl- und Heteroalkyl-Reste jeweils gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus O-$C_{1-4}$-Alkyl und OH; und wobei Benzyl, Phenyl, Pyridyl, Thienyl und Furyl jeweils unsubstituiert oder einfach oder mehrfach substituiert sein können mit einem oder mehreren Substituenten jeweils unabhängig voneinander ausgewählt aus der Gruppe bestehend aus F, Cl, Br, I, CN, $C_{1-4}$-Alkyl, O-$C_{1-4}$-Alkyl $CF_3$, OH und $OCF_3$;

Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CH_2CF_3$, $CF_3$, CN, CHO, $CO_2H$, SH, $SCF_3$, $S(=O)_2OH$, OH, $OCF_3$,$NH_2$, C(=O)-$NH_2$, $C_{1-8}$-Alkyl, $C_{2-8}$-Heteroalkyl, NH-$C_{1-8}$-Alkyl, N($C_{1-8}$-Alkyl)$_2$, O-$C_{1-8}$-Alkyl, C(=O)$C_{1-8}$-Alkyl, C(=O)O-$C_{1-8}$-Alkyl, O-C(=O)$C_{1-8}$-Alkyl, C(=O)NH-$C_{1-8}$-Alkyl, C(=O)N($C_{1-8}$-Alkyl)$_2$, NH-C(=O)$C_{1-8}$-Alkyl, N($C_{18}$-Alkyl)-C(=O)$C_{1-8}$-Alkyl, N(C(=O)$C_{1-8}$-Alkyl)$_2$, S-$C_{1-8}$-Alkyl, $S(=O)_2O$-$C_{1-8}$-Alkyl, $C_{3-10}$-Cycloalkyl und Heterocyclyl, Benzyl, Phenyl, Pyridyl, Thienyl und Furyl; wobei die vorstehenden Alkyl- und Heteroalkyl-Reste jeweils gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus O-$C_{1-4}$-Alkyl und OH; wobei $C_{3-10}$-Cycloalkyl oder Heterocyclyl, jeweils gesättigt oder ungesättigt, unsubstituiert oder einfach oder mehrfach substituiert sein können mit einem oder mehreren Substituenten jeweils unabhängig voneinander ausgewählt aus der Gruppe bestehend aus O-$C_{1-4}$-Alkyl, =O und OH; und wobei Benzyl, Phenyl, Pyridyl, Thienyl und Furyl jeweils unsubstituiert oder einfach oder mehrfach substituiert sein können mit einem oder mehreren Substituenten jeweils unabhängig voneinander ausgewählt aus der Gruppe bestehend aus F, Cl, Br, I, CN, $C_{1-4}$-Alkyl, O-$C_{1-4}$-Alkyl, $CF_3$, OH und $OCF_3$;

In einer weiteren bevorzugten Ausführungsform steht der Rest $R^1$ für Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CH_2CF_3$, $CF_3$, CN, CHO, $CO_2H$, SH, $SCF_3$, $S(=O)_2OH$, OH, $OCF_3$,$NH_2$, C(=O)-$NH_2$, $C_{1-8}$-Alkyl, $C_{2-8}$-Heteroalkyl, NH-$C_{1-8}$-Alkyl, N($C_{1-8}$-Alkyl)$_2$, O-$C_{1-8}$-Alkyl, C(=O)$C_{1-8}$-Alkyl, C(=O)O-$C_{1-8}$-Alkyl, O-C(=O)$C_{1-8}$-Alkyl, C(=O)NH-$C_{1-8}$-Alkyl, C(=O)N($C_{1-8}$-Alkyl)$_2$, NH-C(=O)$C_{1-8}$-Alkyl, N($C_{1-8}$-Alkyl)-C(=O)$C_{1-8}$-Alkyl, N(C(=O)$C_{1-8}$-Alkyl)$_2$, S-$C_{1-8}$-Alkyl, $S(=O)_2O$-$C_{1-8}$-Alkyl, $C_{3-10}$-Cycloalkyl und Heterocyclyl, Benzyl, Phenyl, Pyridyl, Thienyl und Furyl; wobei die vorstehenden Alkyl- und Heteroalkyl-Reste jeweils gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus O-$C_{1-4}$-Alkyl und OH; wobei $C_{3-10}$-Cycloalkyl oder Heterocyclyl jeweils gesättigt oder ungesättigt, unsubstituiert oder einfach oder mehrfach substituiert sein können mit einem oder mehreren Substituenten jeweils unabhängig voneinander ausgewählt aus der Gruppe bestehend aus O-$C_{1-4}$-Alkyl, =O und OH; und wobei Benzyl, Phenyl, Pyridyl, Thienyl und Furyl jeweils unsubstituiert oder einfach oder mehrfach substituiert sein können mit einem oder mehreren Substituenten jeweils unabhängig voneinander ausgewählt aus der Gruppe bestehend aus F, Cl, Br, I, CN, $C_{1-4}$-Alkyl, O-$C_{1-4}$-Alkyl, $CF_3$, OH und $OCF_3$;

Vorzugsweise steht der Rest $R^1$ für Phenyl, Naphthyl, Biphenyl, Benzofuranyl, Benzothienyl, Furyl, Imidazolyl, Indolyl, Oxazolyl, Oxadiazolyl, Pyrimidinyl, Pyridazinyl, Pyrazinyl, Pyrrolyl, Pyrazolyl, Pyridyl, Thienyl, Thiazolyl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CF_3$, CN, $CH_2CF_3$, C(=O)-OH, SH, $SCF_3$, $S(=O)_2OH$, $OCF_3$, OH, $NH_2$, C(=O)$NH_2$, $C_{1-8}$-Alkyl, O-$C_{1-8}$-Alkyl, NH-$C_{1-8}$-Alkyl, N($C_{1-8}$-Alkyl)$_2$, wobei Alkyl jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus O-Methyl und OH sein kann, C(=O)$C_{1-8}$-Alkyl, C(=O)O-$C_{1-8}$-Alkyl, O-C(=O)-$C_{1-8}$-Alkyl, C(=O)NH-$C_{1-8}$-Alkyl, C(=O)N($C_{1-8}$-Alkyl)$_2$, NH-C(=O)$C_{1-8}$-Alkyl, N($C_{1-8}$-Alkyl)-C(=O)$C_{1-8}$-Alkyl, N(C(=O)$C_{1-8}$-Alkyl)$_2$, S-$C_{1-8}$-Alkyl, $S(=O)_2O$-$C_{1-8}$-Alkyl, wobei Alkyl jeweils gesättigt oder ungesättigt, verzweigt oder unverzweigt, unsubstituiert sein kann, Cyclopentyl, Cyclohexyl, Adamantyl, Pyrrolidinyl, Piperidinyl, 4-Methylpiperazinyl, Piperazinyl oder Morpholinyl, jeweils unsubstituiert, Benzyl, Phenyl oder Pyridyl, wobei Benzyl, Phenyl oder Pyridyl jeweils unsubstituiert oder einfach, zweifach oder dreifach substituiert sind mit einem, zwei oder drei Substituenten jeweils unabhängig voneinander ausgewählt aus der Gruppe bestehend aus F, Cl, Br, I, CN, $C_{1-8}$-Alkyl, O-$C_{1-8}$-Alkyl, $CF_3$,

OH und $OCF_3$;

Besonders bevorzugt steht $R^1$ für Phenyl, Pyridyl oder Thienyl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CF_3$, CN, Methyl, Ethyl, n-Propyl, iso-Propyl, Cyclopropyl, n-Butyl, sec.-Butyl, tert.-Butyl, $CH_2CF_3$, C(=O)-Methyl, C(=O)-Ethyl, C(=O)-OH, C(=O)-O-Methyl, C(=O)-O-Ethyl, C(=O)-$NH_2$, C(=O)-N(Methyl)$_2$, C(=O)-N(Ethyl)$_2$, C(=O)-NH-Methyl, C(=O)-NH-Ethyl, C(=O)-N(Methyl)(Ethyl), OH, O-Methyl, O-Ethyl, O-$(CH_2)_2$-O-$CH_3$, O-$(CH_2)_2$-OH, $OCF_3$, O-C(=O)-Methyl, O-C(=O)-Ethyl, $NR^aR^b$, wobei $R^a$ und $R^b$ jeweils unabhängig voneinander ausgewählt sind aus der Gruppe bestehend aus H, Methyl, Ethyl, $(CH_2)_2$-O-$CH_3$ und $(CH_2)_2$-OH oder $R^a$ und $R^b$ gemeinsam mit dem sie verbindenden Stickstoffatom ein Pyrrolidinyl, Piperidinyl, 4-Methylpiperazinyl oder Morpholinyl bilden, NHC(=O)-Methyl, NHC(=O)-Ethyl, SH, $SCF_3$, S-Methyl, S-Ethyl, S(=O)$_2$OH, S(=O)$_2$O-Methyl, Benzyl, Phenyl, Pyridyl, wobei Benzyl, Phenyl, Pyridyl jeweils unsubstituiert oder einfach, zweifach oder dreifach substituiert sind mit einem, zwei oder drei Substituenten jeweils unabhängig voneinander ausgewählt aus der Gruppe bestehend aus F, Cl, Br, I, CN, Methyl, Ethyl, $CF_3$, OH, O-Methyl und $OCF_3$;

Ganz besonders bevorzugt steht $R^1$ für Phenyl, Pyridyl oder Thienyl, jeweils unsubstituiert oder einfach oder zweifach oder dreifach substituiert mit einem, zwei oder drei Substituenten jeweils unabhängig voneinander ausgewählt aus der Gruppe bestehend aus F, Cl, Br, I, $NO_2$, $CF_3$, CN, Methyl, Ethyl, C(=O)-Methyl, OH, O-Methyl, O-$(CH_2)_2$-O-$CH_3$, $OCF_3$, O-C(=O)-Methyl, $NH_2$, NH-C(=O)-Methyl, N(Methyl)$_2$, Morpholinyl, S-Methyl, $SCF_3$, Benzyl und Phenyl, jeweils unsubstituiert;

In einer weiteren bevorzugten Ausführungsform steht m für die Zahl 1, wobei $R^1$ die Bedeutung gemäß einer der vorstehend genannten Ausführungsformen hat, bevorzugt jedoch ein Thienyl-, Phenyl-, ein $C_{3-8}$ Alkylrest, der gesättigt oder ungesättigt , unsubstituiert oder ein- oder mehrfach substituiert sein kann. oder ein mono- oder bicyclischer $C_{3-8}$-Cycloalkylrest ist, der gesättigt oder ungesättigt (aber nicht aromatisch), unsubstituiert oder ein- oder mehrfach substituiert sein kann.

Bevorzugte dieser Cycloalkylreste sind Cyclopentyl, Cyclohexyl, Cycloheptyl, Cyclooctyl, Adamantyl, Dihydropyranyl, Tetrahydrofuranyl oder

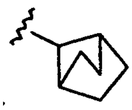

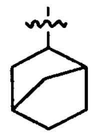

In einer weiteren bevorzugten Ausführungsform steht m für die Zahl 2, wobei $R^1$ die Bedeutung gemäß einer der vorstehend genannten Ausführungsformen hat, bevorzugt jedoch für Phenyl-, Cycloalkyl- oder Alkyl- steht.

In einer weiteren bevorzugten Ausführungsform steht m für die Zahl 0, wobei $R^1$ für Aryl- oder Heteroaryl- steht.

In einer bevorzugten Ausführungsform stehen $R^{6a}$ und $R^{6b}$ jeweils unabhängig voneinander für H; F; Cl; Br; I; Methyl; Ethyl; n-Propyl; iso-Propyl; n-Butyl; sec.-Butyl; tert.-Butyl; OH; O-Methyl; O-Ethyl; O-$(CH_2)_2$-O-$CH_3$; oder O-$(CH_2)_2$-OH;

In einer weiteren bevorzugten Ausführungsform steht der Rest $R^2$ für Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH;

In einer weiteren bevorzugten Ausführungsform stehen die Reste $R^{7a}$,$R^{7b}$, $R^{8a}$, $R^{8b}$, $R^{9a}$ und $R^{9b}$ jeweils unabhängig voneinander für H; F; Cl; Br; I; $NO_2$; $CF_3$; CN; OH; $OCF_3$; $NH_2$; $C_{1-4}$-Alkyl, O-$C_{1-4}$-Alkyl, NH-$C_{1-4}$-Alkyl, N($C_{1-4}$-Alkyl)$_2$, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, O-$C_{1-4}$-Alkyl, OH und $OCF_3$; $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, =O, O-$C_{1-4}$-Alkyl, $OCF_3$, $NH_2$, NH-$C_{1-4}$-Alkyl, und N($C_{1-4}$-Alkyl)$_2$; stehen;

Vorzugsweise stehen $R^{7a}$, $R^{7b}$, $R^{8a}$, $R^{8b}$, $R^{9a}$ und $R^{9b}$ jeweils unabhängig voneinander für H; F; Cl; Br; I; $NO_2$;

$CF_3$; $CH_2CF_3$; CN; OH; $OCF_3$, $NH_2$; $C_{1-4}$-Alkyl, O-$C_{1-4}$-Alkyl, 0-$C_{1-4}$-Alkyl-OH, O-$C_{1-4}$-Alkyl-O-$CH_3$, NH-$C_{1-4}$-Alkyl, N($C_{1-4}$-Alkyl)$_2$, jeweils gesättigt oder ungesättigt, verzweigt oder unverzweigt, unsubstituiert; $C_{3-10}$-Cycloalkyl, gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, O-$C_{1-4}$-Alkyl;

Besonders bevorzugt stehen $R^{7a}$, $R^{7b}$ , $R^{8a}$, $R^{8b}$, $R^{9a}$ und $R^{9b}$ jeweils unabhängig voneinander für für H; F; Cl; Br; I; $NO_2$; $CF_3$; CN; Methyl; Ethyl; n-Propyl; iso-Propyl; Cyclopropyl; n-Butyl; sec.-Butyl; tert.-Butyl; $CH_2CF_3$; OH; O-Methyl; O-Ethyl; 0-$(CH_2)_2$-O-$CH_3$; O-$(CH_2)_2$-OH; $OCF_3$; $NH_2$; NH-Methyl; N(Methyl)$_2$; NH-Ethyl; N(Ethyl)$_2$; oder N(Methyl)(Ethyl);

Ganz besonders bevorzugt stehen $R^{7a}$, $R^{7b}$, $R^{8a}$ und $R^{8b}$ jeweils unabhängig voneinander für H oder Methyl.

Für den Fall, dass $R^{7a}$ mit $R^{8a}$ einen Cycloalkylrest bildet, so ist dieser bevorzugt 3 bis 6, besonders bevorzugt 6-gliedrig und kann gesättigt oder ungesättigt, unsubstituiert oder einfach oder mehrfach substituiert sein.

In einer weiteren bevorzugten Ausführungsform steht Y für-($CR^{9a}R^{9b}$)-, wobei $R^{9a}$ und $R^{9b}$ unabhängig voneinander für Wasserstoff oder Halogen stehen, besonders bevorzugt ist $R^{9a}$ = $R^{9b}$ = Wasserstoff oder Halogen, ganz besonders bevorzugt $R^{9a}$ = $R^{9b}$ = Fluor oder Wasserstoff.

In einer weiteren bevorzugten Ausführungsform stehen die Reste $R^3$, $R^4$ und $R^5$ jeweils unabhängig voneinander für H; F; Cl; Br; I; $NO_2$; $CF_3$; CN; OH; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ oder C(=O)-$C_{1-6}$-Alkyl, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH und O-$C_{1-4}$-Alkyl; $C_{3-7}$-Cycloalkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, =O und O-$C_{1-4}$-Alkyl; $NR^aR^b$, wobei $R^a$ und $R^b$ gemeinsam mit dem sie verbindenden Stickstoffatom ein Heterocyclyl, gesättigt oder ungesättigt, unsubstituiert oder einfach oder mehrfach substituiert mit $C_{1-4}$-Alkyl-, bilden.

Vorzugsweise stehen die Reste $R^3$, $R^4$ und $R^5$ jeweils unabhängig voneinander für H; F; Cl; Br; I; $NO_2$; $CF_3$; CN; OH; $OCF_3$; SH; $SCF_3$; Methyl; Ethyl; n-Propyl; iso-Propyl; Butyl; sec.-Butyl; tert.-Butyl; $CH_2CF_3$; O-Methyl; O-Ethyl; O-n-Propyl; O-iso-Propyl; 0-Butyl; O-sec.-Butyl; O-tert.-Butyl; O-$(CH_2)_2$-O-Methyl; O-$(CH_2)_2$-OH; O-(C=O)-Methyl; O-(C=O)-Ethyl; S-Methyl; S-Ethyl; Cyclopropyl; Cyclobutyl; $NR^aR^b$, wobei $R^a$ und $R^b$ jeweils unabhängig voneinander ausgewählt sind aus der Gruppe bestehend aus H, Methyl, Ethyl, $(CH_2)_2$-O-Methyl, $(CH_2)_2$-OH, (C=O)-Methyl, (C=O)-Ethyl oder $R^a$ und $R^b$ gemeinsam mit dem sie verbindenden Stickstoffatom ein Pyrrolidinyl, Piperidinyl, 4-Methylpiperazinyl oder Morpholinyl bilden.

Besonders bevorzugt stehen die Reste $R^3$, $R^4$ und $R^5$ jeweils unabhängig voneinander für H; F; Cl; Br; I; Methyl; Ethyl; n-Propyl, iso-Propyl; Cyclopropyl; CN; $CF_3$; O-Methyl; $OCF_3$; S-Methyl; $SCF_3$.

Ganz besonders bevorzugt stehen die Reste $R^3$, $R^3$ und $R^5$ jeweils unabhängig voneinander für H; F; Cl; Methyl; Ethyl; $CF_3$; insbesondere für H.

Andere bevorzugte Ausführungsformen der erfindungsgemäßen Verbindungen der allgemeinen Formel (1) weisen eine der allgemeinen Formeln (2a) oder (2b) auf:

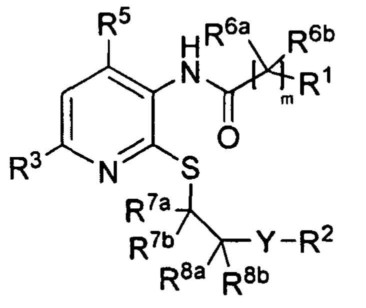

(2a)

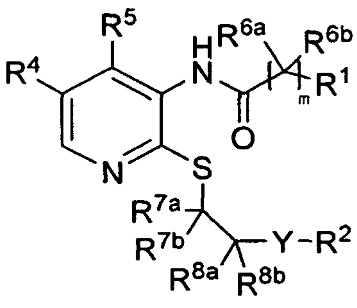

(2b)

Insbesondere bevorzugt sind Verbindungen aus der Gruppe

1 N-[2-[2-(Benzolsulfonyl)-ethylsulfanyl]-pyridin-3-yl]-2-cyclohexyl-acetamid;

2 2-Cyclohexyl-N-[2-[2-[[3-(trifluormethyl)phenyt]sulfonyl]-ethylsulfanyl]-pyridin-3-yl]-acetamid;

**3** 2-Thiophen-2-yl-N-[2-[2-[[3-(trifluormethyl)phenyl]sulfonyl]-ethylsulfanyl]-pyridin-3-yl]-acetamid;

**4** N-[2-[2-(Benzolsulfonyl)-ethylsulfanyl]-pyridin-3-yl]-benzamid;
**5** N-[2-[2-(Benzolsulfonyl)-ethylsulfanyl]-pyridin-3-yl]-3,4-difluor-benzamid;
**6** N-[2-[2-(Benzolsulfonyl)-ethylsulfanyl]-pyridin-3-yl]-3-cyclohexyl-propionamid;
**7** N-[2-[2-(Benzolsulfonyl)-ethylsulfanyl]-pyridin-3-yl]-2-thiophen-2-yl-acetamid;
**8** N-[2-[2-(Benzolsulfonyl)-ethylsulfanyl]-pyridin-3-yl]-2-(3,5-dimethyl-phenyl)-propionamid;
**9** 2-(2-Methoxyphenyl)-N-[2-[2-[[3-(trifluormethyl)phenyl]sulfonyl]-ethylsulfanyl]-pyridin-3-yl]-acetamid;
**10** 2-(4-Methoxyphenyl)-N-[2-[2-[[3-(trifluormethyl)phenyl]sulfonyl]-ethylsulfanyl]-pyridin-3-yl]-acetamid;
**11** 2-(3-Methoxyphenyl)-N-[2-[2-[[3-(trifluormethyl)phenyl]sulfonyl]-ethylsulfanyl]-pyridin-3-yl]-acetamid
**12** 2-(2-Hydroxyphenyl)-N-[2-[2-[[3-(trifluormethyl)phenyl]sulfonyl]-ethylsulfanyl]-pyridin-3-yl]-acetamid;
**13** 2-(4-Hydroxyphenyl)-N-[2-[2-[[3-(trifluormethyl)phenyl]sulfonyl]-ethylsulfanyl]-pyridin-3-yl]-acetamid;
**14** 2-Cyclopentyl-N-[2-[2-[[3-(trifluormethyl)phenyl]sulfonyl]-ethylsulfanyl]-pyridin-3-yl]-acetamid;
**18** N-[2-[2-[[3-(Trifluormethyl)phenyl]sulfonyl]-ethylsulfanyl]-pyridin-3-yl]-thiophen-2-carbonsäure amid;
**19** N-[2-[2-[[3-(Trifluormethyl)phenyl]sulfonyl]-ethylsulfanyl]-pyridin-3-yl]-cyclohexancarbonsäure amid;
**20** 2-Thiophen-2-yl-N-[2-[2-[[3-(trifluormethyl)phenyl]sulfanyl]-ethylsulfanyl]-pyridin-3-yl]-acetamid;
**21** N-[2-(2-Phenylsulfanyl-ethylsulfanyl)-pyridin-3-yl]-2-thiophen-2-yl-acetamid;
**22** 2-Thiophen-2-yl-N-[2-[2-[3-(trifluormethyl)-phenoxy]-ethylsulfanyl]-pyridin-3-yl]-acetamid;
**23** 2-Thiophen-2-yl-N-[2-[3-[3-(trifluormethyl)phenyl]-propylsulfanyl]-pyridin-3-yl]-acetamid;
**24** 2-Naphthalen-2-yl-N-[2-[2-[[3-(trifluormethyl)phenyl]sulfonyl]-ethylsulfanyl]-pyridin-3-yl]-acetamid;
**25** 4-Phenyl-N-[2-[2-[[3-(trifluormethyl)phenyl]sulfonyl]-ethylsulfanyl]-pyridin-3-yl]-butyramid;
**26** 3-Thiophen-2-yl-N-[2-[2-[[3-(trifluormethyl)phenyl]sulfonyl]-ethylsulfanyl]-pyridin-3-yl]-propionamid;
**27** 3-Phenyl-N-[2-[2-[[3-(trifluormethyl)phenyl]sulfonyl]-ethylsulfanyl]-pyridin-3-yl]-propionamid;
**28** 3-Cyclopentyl-N-[2-[2-[[3-(trifluormethyl)phenyl]sulfonyl]-ethylsulfanyl]-pyridin-3-yl]-propionamid;
**29** 2-Cyclohexyl-N-[2-[2-[[3-(trifluormethyl)phenyl]sulfanyl]-ethylsulfanyl]-pyridin-3-yl]-acetamid;
**31** ((E)-3-(4-Fluorphenyl)-N-[2-[2-[[3-(trifluormethyl)phenyl]sulfonyl]-ethylsulfanyl]-pyridin-3-yl]-acrylamid:
**33** N-[2-(3-Phenyl-propylsulfanyl)-pyridin-3-yl]-2-thiophen-2-yl-acetamid;
**35** 6-Chlor-N-[2-[2-[[3-(trifluormethyl)phenyl]sulfonyl]-ethylsulfanyl]-pyridin-3-yl]-pyridin-3-carbonsäure amid;
**36** 2-Pyridin-4-yl-N-[2-[2-[[3-(trifluormethyl)phenyl]sulfonyl]-ethylsulfanyl]-pyridin-3-yl]-acetamid;
**37** 2-(3-Hydroxyphenyl)-N-[2-[2-[[3-(trifluormethyl)phenyl]sulfonyl]-ethylsulfanyl]-pyridin-3-yl]-acetamid;
**38** N-[2-[2-[[3-(Trifluormethyl)phenyl]sulfonyl]-ethylsulfanyl]-pyridin-3-yl]-tetrahydro-pyran-3-carbonsäure amid;
**39** 2-Tetrahydro-pyran-2-yl-N-[2-[2-[[3-(trifluormethyl)phenyl]sulfonyl]-ethylsulfanyl]-pyridin-3-yl]-acetamid;
**40** 2-Tetrahydro-pyran-4-yl-N-[2-[2-[[3-(trifluormethyl)phenyl]sulfonyl]-ethylsulfanyl]-pyridin-3-yl]-acetamid;
**41** 3-Cyclohexyl-N-[2-[2-[[3-(trifluormethyl)phenyl]sulfonyl]-ethylsulfanyl]-pyridin-3-yl]-propionamid;
**44** 2-Cyclohexyl-N-[2-(2-phenylsulfanyl-ethylsulfanyl)-pyridin-3-yl]-acetamid;
**45** N-[2-(2-Phenoxy-ethylsulfanyl)-pyridin-3-yl]-2-thiophen-2-yl-acetamid;
**46** 2-Pyridin-3-yl-N-[2-[2-[[3-(trifluormethyl)phenyl]sulfonyl]-ethylsulfanyl]-pyridin-3-yl]-acetamid;
**47** 3-Hydroxy-N-[2-[2-[[3-(trifluormethyl)phenyl]sulfonyl]-ethylsulfanyl]-pyridin-3-yl]-benzamid;
**48** N-[2-[2-[[3-(Trifluormethyl)phenyl]sulfonyl]-ethylsulfanyl]-pyridin-3-yl]-tetrahydro-pyran-2-carbonsäure amid;
**49** 2-Cycloheptyl-N-[2-[2-[[3-(trifluormethyl)phenyl]sulfonyl]-ethylsulfanyl]-pyridin-3-yl]-acetamid;
**50** 4-Fluor-2-methoxy-N-[2-[2-[[3-(trifluormethyl)phenyl]sulfonyl]-ethylsulfanyl]-pyridin-3-yl]-benzamid;
**51** 4-Fluor-2-hydroxy-N-[2-[2-[[3-(trifluormethyl)phenyl]sulfonyl]-ethylsulfanyl]-pyridin-3-yl]-benzamid;
**52** 2-(1,2,3,4-Tetrahydro-naphthalen-2-yl)-N-[2-[2-[[3-(trifluormethyl)phenyl]sulfonyl]-ethylsulfanyl]-pyridin-3-yl]-acetamid;
**53** 2-Hydroxy-N-[2-[2-[[3-(trifluormethyl)phenyl]sulfonyl]-ethylsulfanyl]-pyridin-3-yl]-benzamid;
**55** 2-(3-Oxo-cyclohexyl)-N-[2-[2-[[3-(trifluormethyl)phenyl]sulfonyl]-ethylsulfanyl]-pyridin-3-yl]-acetamid;
**56** N-[4-Methyl-2-[2-[[3-(trifluormethyl)phenyl]sulfonyl]-ethylsulfanyl]-pyridin-3-yl]-2-thiophen-2-yl-acetamid;
**57** 3-(1,2,3,4-Tetrahydro-naphthalen-2-yl)-N-[2-[2-[[3-(trifluormethyl)phenyl]sulfonyl]-ethylsulfanyl]-pyridin-3-yl]-propionamid;
**58** 3-Cycloheptyl-N-[2-[2-[[3-(trifluormethyl)phenyl]sulfonyl]-ethylsulfanyl]-pyridin-3-yl]-propionamid;
**59** 2-(Benzo[b]thiophen-2-yl)-N-[2-[2-[[3-(trifluormethyl)phenyl]sulfonyl]-ethylsulfanyl]-pyridin-3-yl]-acetamid;
**60** 2-Pyridin-2-yl-N-[2-[2-[[3-(trifluormethyl)phenyl]sulfonyl]-ethylsulfanyl]-pyridin-3-yl]-acetamid
**61** N-[2-[2-[(4-Fluorphenyl)sulfanyl]-ethylsulfanyl]-pyridin-3-yl]-3,3-dimethyl-butyramid;
**62** 2-(5-Bicyclo[2.2.1]heptanyl)-N-[2-[2-[[3-(trifluormethyl)phenyl]sulfonyl]-ethylsulfanyl]-pyridin-3-yl]-acetamid;
**63** N-[2-[2-(4-Fluor-phenoxy)-ethylsulfanyl]-pyridin-3-yl]-3,3-dimethyl-butyramid
**64** 3,4-Difluor-N-[2-[2-(4-fluor-phenoxy)-ethylsulfanyl]-pyridin-3-yl]-benzamid;
**65** 3,4-Difluor-N-[2-[3-(4-fluorphenyl)-propylsulfanyl]-pyridin-3-yl]-benzamid;
**66** N-[2-[3-(4-Fluorphenyl)-propylsulfanyl]-pyridin-3-yl]-3,3-dimethyl-butyramid;
**67** 2-Cyctoheptyl-N-[2-[2-[(4-fluorphenyl)sulfonyl]-ethyfsulfanyl]-pyridin-3-yl]-acetamid;
**68** 2-Cyclohexyl-2,2-difluor-N-[2-[2-[[3-(trifluormethyl)phenyl]sulfonyl]-ethylsulfanyl]-pyridin-3-yl]-acetamid;

oder deren physiologisch verträgliche Salze.

Die erfindungsgemäßen substituierten 2-Mercapto-3-aminopyridine sowie jeweils die entsprechenden Säuren, Basen, Salze und Solvate eignen sich als pharmazeutische Wirkstoffe in Arzneimitteln.

Ein weiterer Gegenstand der Erfindung ist daher ein Arzneimittel enthaltend wenigstens ein erfindungsgemäßes substituiertes 2-Mercapto-3-aminopyridin der allgemeinen Formel (1), worin die Reste $R^1$-$R^5$ die oben angegebene Bedeutung haben sowie ggf. einen oder mehrere pharmazeutisch verträgliche Hilfsstoffe.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfindungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Diese erfindungsgemäßen Arzneimittel eignen sich zur Beeinflussung von KCNQ2/3-Kanälen und üben eine agonistische oder antagonistische, insbesondere eine agonistische Wirkung aus.

Bevorzugt eignen sich die erfindungsgemäßen Arzneimittel zur Behandlung von Störungen oder Krankheiten, die zumindest teilweise durch KCNQ2/3-Kanäle vermittelt werden.

Bevorzugt eignet sich die erfindungsgemäßen Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulärem Schmerz.

Weiterhin eignen sich die erfindungsgemäßen Arzneimittel besonders bevorzugt zur Behandlung von Epilepsie.

Ein weiterer Gegenstand der Erfindung ist die Verwendung wenigstens eines erfindungsgemäßen substituierten 2-Mercapto-3-aminopyridins sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3-Kanäle vermittelt werden.

Bevorzugt ist die Verwendung wenigstens eines erfindungsgemäßen substituierten 2-Mercapto-3-aminopyridins sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt ist die Verwendung wenigstens eines erfindungsgemäßen substituierten 2-Mercapto-3-aminopyridins sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz.

Weiterhin besonders bevorzugt ist die Verwendung wenigstens eines erfindungsgemäßen substituierten 2-Mercapto-3-aminopyridins sowie ggf. eines oder mehrerer pharmazeutisch verträglicher Hilfsstoffe zur Herstellung eines Arzneimittels zur Behandlung von Epilepsie.

Ein weiterer Gegenstand der Erfindung ist wenigstens ein erfindungsgemäßes substituiertes 2-Mercapto-3-aminopyridin sowie ggf. ein oder mehrere pharmazeutisch verträgliche Hilfsstoffe zur Behandlung von Störungen oder Krankheiten, die zumindest teilweise durch KCNQ2/3-Kanäle vermittelt werden.

Ein weiterer Gegenstand der Erfindung ist wenigstens ein erfindungsgemäßes substituiertes 2-Mercapto-3-aminopyridin sowie ggf. ein oder mehrere pharmazeutisch verträgliche Hilfsstoffe zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt ist wenigstens ein erfindungsgemäßes substituiertes 2-Mercapto-3-aminopyridin sowie ggf. ein oder mehrere pharmazeutisch verträgliche Hilfsstoffe zur Behandlung von Schmerz, ganz besonders bevorzugt von chronischem Schmerz, neuropathischem Schmerz, inflammatorischem Schmerz und muskulärem Schmerz.

Besonders bevorzugt ist auch wenigstens ein erfindungsgemäßes substituiertes 2-Mercapto-3-aminopyridin sowie ggf. ein oder mehrere pharmazeutisch verträgliche Hilfsstoffe zur Behandlung von Epilepsie.

Die Wirksamkeit gegen Schmerz kann beispielsweise im Bennett- bzw. Chung-Modell gezeigt werden (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363). Die Wirksamkeit gegen Epilepsie kann beispielsweise im DBA/2 Maus Modell (De Sarro et al., Naunyn-Schmiedeberg's Arch. Pharmacol. 2001, 363, 330-336) nachgewiesen werden.

Bevorzugt weisen die erfindungsgemäßen substituierten 2-Mercapto-3-aminopyridine einen $EC_{50}$-Wert von höchstens 10 μM oder höchstens 6 μM auf, bevorzugter höchstens 5 μM oder höchstens 4 μM, noch bevorzugter höchstens 3 μM oder höchstens 2 μM, am bevorzugtesten höchstens 1 μM oder höchstens 0,7 μM und insbesondere höchstens 0,6 μM oder höchstens 0,4 μM. Methoden zur Bestimmung des $EC_{50}$-Wertes sind dem Fachmann bekannt. Bevorzugt erfolgt die Bestimmung des $EC_{50}$-Wertes fluorimetrisch, besonders bevorzugt wie unter "Pharmakologische Experimente" beschrieben.

Ein weiterer Gegenstand der Erfindung sind Verfahren zur Herstellung der erfindungsgemäßen substituierten 2-Mercapto-3-aminopyridine.

Die in den nachstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

**Allgemeines Reaktionsschema**

**Schema 1:**

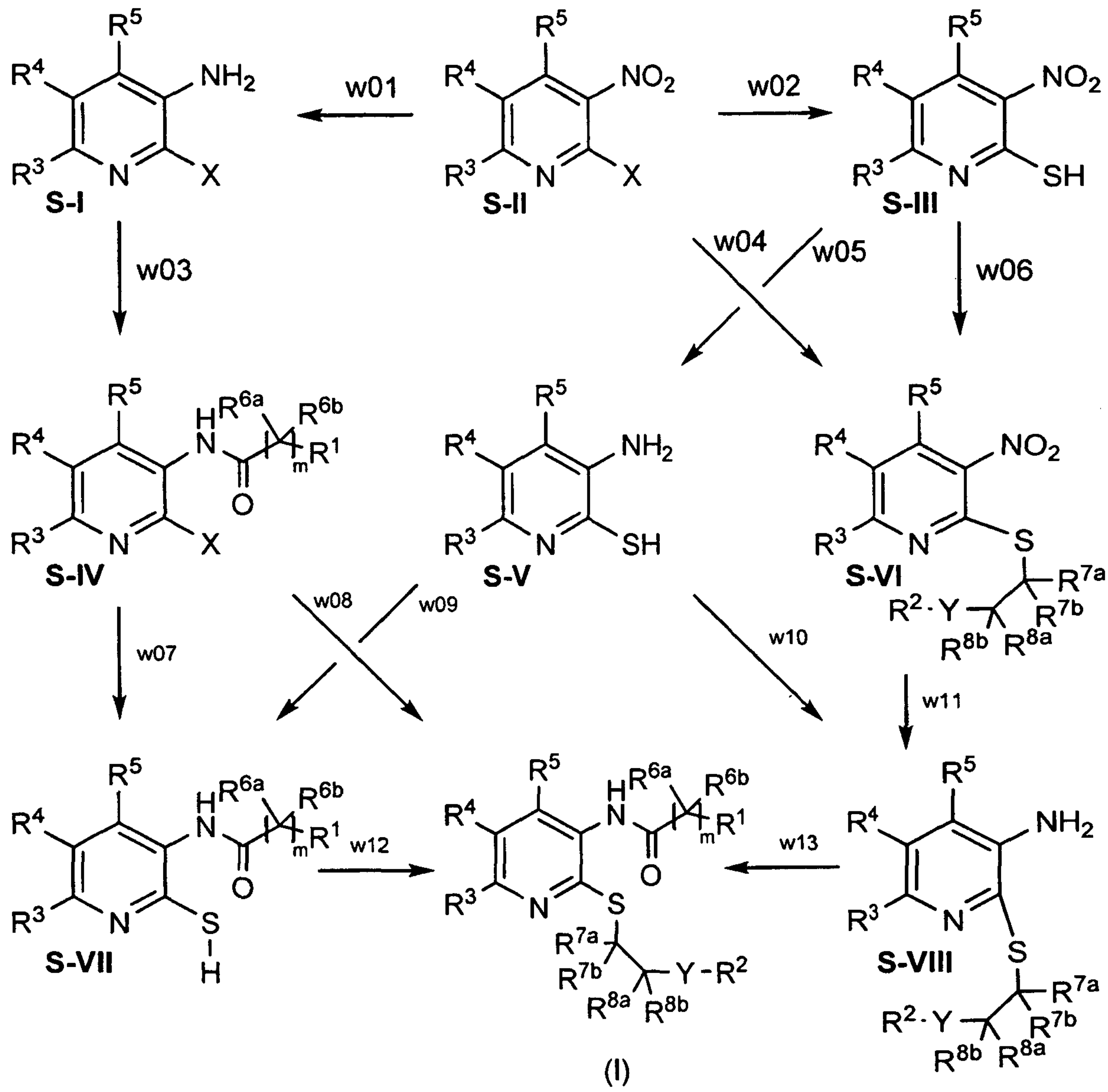


In den Schritten **w01**, **w05** und **w11** können die Nitro-Gruppen der Verbindungen **S-II** und **S-VI** mittels dem Fachmann geläufigen Reduktionsmethoden wie bspw. in Gegenwart von Metallen in saurer Lösung oder durch katalytische Hydrierung in die entsprechenden Amine **S-I**, **S-V** und **S-VIII** überführt werden.

In den Schritten **w02** und **w07** können die 2-Halogen-pyridine **S-II** und **S-IV**, worin X für Halogen, bevorzugt für F oder Chlor steht, mittels dem Fachmann bekannten Methoden wie bspw. durch Substitution mit einem Thiol, beispielsweise 3-Mercaptopropansäuremethylester, zunächst in den entsprechenden Thioether überführt werden, welcher im Anschluss, ggf. in Gegenwart einer Säure oder einer Base, zum Thiol **S-III** oder **S-VII** gespalten werden kann.

In den Schritten **w03**, **w09** und **w13** können die Amine **S-I**, **S-V** und **S-VIII** in die entsprechenden Amide **S-IV**, **S-VII**, und **I** überführt werden. Dies kann bspw. jeweils durch Reaktion mit einem Säurechlorid $R^1$-C(=O)-Cl mittels dem Fachmann geläufigen Methoden, ggf. in Gegenwart einer Base oder durch Reaktion mit einer Säure $R^1$-C(=O)-OH in Gegenwart eines geeigneten Kopplungsreagenz, beispielsweise HATU oder CDI, ggf. unter Zusatz einer Base, erreicht werden.

In den Schritten **w04** und **w08** können die Thioether **I** und **S-VI** ausgehend von den 2-Halogen-pyridinen **S-II** und **S-IV**, worin X jeweils für Halogen, bevorzugt für Fluor oder Chlor steht, mittels dem Fachmann geläufigen Methoden, bspw. durch Reaktion mit dem entsprechenden Thiol $R^2$-SH in einer ipso-Substitution, ggf. in Gegenwart einer Base, gebildet werden.

In den Schritten **w06**, **w10** und **w12** können die Thiole **S-III**, **S-V** und **S-VII** in die entsprechenden Thioether **S-VI**, **S-VIII** und I mittels dem Fachmann geläufigen Methoden, bspw. durch Umsetzung mit einem Alkylhalogenids $R^2$-Hal ggf. in Gegenwart einer Base, überführt werden.

Die dem Fachmann geläufigen Methoden zur Durchführung der Reaktionsschritte **w01** bis **w13** sind den Standardwerken der Organischen Chemie zu entnehmen wie bspw. J. March, Advanced Organic Chemistry, Wiley & Sons, 6th edition, 2007; F. A. Carey, R. J. Sundberg, Advanced Organic Chemistry, Parts A and B, Springer, 5th edition, 2007); Autorenkollektiv, Compendium of Organic Synthetic Methods, Wiley & Sons. Darüber hinaus können weitere Methoden sowie Literaturverweise von den gängigen Datenbanken wie bspw. der Reaxys® Datenbank der Firma Elsevier, Amsterdam, NL oder der SciFinder® Datenbank der American Chemical Society, Washington, US, ausgegeben werden.

## Beschreibung der Synthesen

### Abkürzungen

| | |
|---|---|
| AcOH | Essigsäure |
| aq. | wässrig |
| d | Tage |
| BOP | 1-Benzotriazolyloxy-tris-(dimethylamino)-phosphonium hexafluorophosphat |
| Brine | gesättigte Natriumchlorid-Lösung |
| DCC | N,N'-Dicyclohexylcarbodiimid |
| DCM | Dichlormethan |
| DIPEA | N,N-Diisopropylethylamin |
| DMF | N,N-Dimethylformamid |
| DMAP | 4-Dimethylamino-pyridin |
| EDC | N-(3-Dimethylaminopropyl)-N'-ethyl-carbodiimid |
| EE | Ethylacetat |
| Ether | Diethylether |
| EtOH | Ethanol |
| ges. | gesättigt |
| h | Stunde(n) |
| HATU | O-(7-Aza-Benzotriazol-1-yl)-N,N,N',N'-tetramethyluroniumhexafluorphosphat |
| HBTU | O-(Benzotriazol-1-yl)-N,N,N',N'-tetramethyluroniom hexafluorophosphat |
| HOAt | 1-Hydroxy-7-azabenzotriazol |
| Lsg. | Lösung |
| LG | Abgangsgruppe |
| m/z | Verhältnis Masse zur Ladung |
| MeCN | Acetonitril |
| MeOH | Methanol |
| min | Minuten |
| MS | Massenspektrometrie |
| N/A | nicht verfügbar |
| $NEt_3$ | Triethylamin |
| RG | Retigabine |
| RT | Raumtemperatur 23 ± 7 °C |
| SC | Säulenchromatographie auf Kieselgel |
| THF | Tetrahydrofuran |
| vv | Volumenverhältnis |

Alle nicht explizit beschriebenen Ausgangsstoffe waren entweder kommerziell verfügbar (Anbieter können beispielsweise in der Symyx® Available Chemicals Database der Firma MDL, San Ramon, US recherchiert werden) oder deren Synthese ist in der Fachliteratur bereits exakt beschrieben (Experimentelle Vorschriften können beispielweise in der Reaxys® Datenbank der Firma Elsevier, Amsterdam, NL recherchiert werden) oder können nach den dem Fachmann bekannten Methoden hergestellt werden.

Als stationäre Phase für die Säulenchromathographie (SC) wurde Kieselgel 60 (0.040 - 0.063 mm) eingesetzt.

Die analytische Charakterisierung aller Zwischenprodukte und Beispielverbindungen erfolgte mittels $^1$H-NMR-Spektroskopie. Zusätzlich wurden für alle Beispielverbindungen und ausgewählte Zwischenprodukte massenspektrometrische Untersuchungen (MS, Angabe m/z für $[M+H]^+$) durchgeführt.

**Synthese der Zwischenprodukte**

**Synthese des Zwischenprodukts VPF-001: [2-[2-[[3-(Trifluormethyl)-phenyl]sulfonyl]-ethylsulfanyl]-pyridin-3-yl]-amin**

a) Synthese von 3-Nitro-2-[2-[[3-(trifluormethyl)phenyl]sulfonyl]-ethylsulfanyl]-pyridin Eine Lösung von 1,56 g (10,0 mmol) 3-Nitro-2-mercapto-pyridin in Aceton (50 ml) wurde bei RT mit 4,15 g (30,0 mmol) Kaliumcarbonat und 2,59 g (9,5 mmol) (1-(3-Chlorethylsulfonyl)-3-(trifluormethyl)benzol versetzt und anschließend 16 h auf 60 °C erhitzt. Dann wurde im Vakuum eingeengt und er Rückstand wurde mit Wasser und EE aufgenommen. Die Phasen wurden getrennt und die organische Phase wurde mit Wasser und Brine gewaschen, über $Na_2SO_4$ getrocknet, filtriert und im Vakuum eingeengt. Das Rohprodukt wurde mehrmals mit Hexan gewaschen wobei 2,35 g (6,0 mmol, 63%) 3-Nitro-2-[2-[[3-(trifluormethyl)phenyl]sulfonyl]-ethylsulfanyl]-pyridin erhalten wurden.

b) Synthese von [2-[2-[[3-(Trifluormethyl)-phenyl]sulfonyl]-ethylsulfanyl]-pyridin-3-yl]-amin
Eine Lösung von 1,96 g (5,0 mmol) 3-Nitro-2-[2-[[3-(trifluormethyl)phenyl]sulfonyl]-ethyl-sulfanyl]-pyridin in Ethanol (45 ml) wurde mit 1,31 g (20 mmol) Zink versetzt und auf 0 °C abgekühlt. Bei dieser Temperatur wurden 200 ml (0.2 M, 40 mmol) einer aq. $NH_4Cl$-Lsg zugegeben und anschließend wurde 30 min auf 90 °C erhitzt. Dann wurde im Vakuum eingeengt und der Rückstand wurde mit Wasser aufgenommen und mit $NaHCO_3$ neutralisiert. Danach wurde mit EE extrahiert und die organische Phase wurde über $Na_2SO_4$ getrocknet, filtriert und im Vakuum eingeengt. Durch SC (Hexan / EE 4:1) des Rückstands wurden 1,38 g (3,8 mmol, 76%) [2-[2-[[3-(Trifluormethyl)-phenyl]sulfonyl]-ethylsulfanyl]-pyridin-3-yl]-amin erhalten.

**Synthese des Zwischenprodukts VPF-002: [2-[2-(Benzolsulfonyl)-ethylsulfanyl]-pyridin-3-yl]-amin**

a) Synthese von 2-[2-(Benzolsulfonyl)-ethylsulfanyl]-3-nitro-pyridin Eine Lösung von 3,0 g (14,8 mmol) 2-(Phenylsulfonyl)ethanthiol in DMF (35 ml) wurde mit 1,8 g (16,0 mmol) Kalium-tert.-butylat versetzt und 30 min bei RT gerührt. Anschließend wurden 2,5 g (15,8 mmol) 2-Chlor-3-nitro-pyridin zugegeben und es wurde 1 h bei RT weiter gerührt. Dann wurde mit einem Brine/EE-gemisch (1:1 w) verdünnt und weitere 30 min bei RT gerührt. Anschließend wurde die organische Phase abgetrennt, mit Wasser und Brine gewaschen, über $Na_2SO_4$ getrocknet, filtriert und im Vakuum eingeengt. Durch SC (Hexan / EE 6:1) des Rückstands wurden 2,2 g (6,8 mmol, 46%) 2-[2-(Benzolsulfonyl)-ethylsulfanyl]-3-nitro-pyridin erhalten.

b) Synthese von 2-[2-(Benzolsulfonyl)-ethylsulfanyl]-pyridin-3-yl]-amin Eine Lösung von 2,2 g (6,8 mmol) 2-[2-(Benzolsulfonyl)-ethylsulfanyl]-3-nitro-pyridin in MeOH (34 ml) wurde mit 5.5 ml konz. aq. Salzsäure und 1,1 g (19,7 mmol) Eisenspähne versetzt und dann 45 min auf 60 °C erhitzt. Anschließend wurde mit MeOH verdünnt, durch Kieselgur filtriert und das Filtrat im Vakuum eingeengt. Der Rückstand wurde mit Wasser aufgenommen und es wurde mit EE extrahiert. Die organische Phase wurde mit Wasser und Brine gewaschen, über $Na_2SO_4$ getrocknet, filtriert und im Vakuum eingeengt. Durch SC (Hexan / EE 3:1) des Rückstands wurden 1,5 g (5,1 mmol, 75%) 2-[2-(Benzolsulfonyl)-ethylsulfanyl]-pyridin-3-yl]-amin erhalten.

**Synthese des Zwischenprodukts VPF-006: [2-(2-Phenylsulfanyl-ethylsulfanyl)-pyridin-3-yl]-amin**

a) Synthese von 3-Nitro-2-(2-phenylsulfanyl-ethylsulfanyl)-pyridin Eine Lösung von 1,56 g (10,0 mmol) 2-Mercapto-3-nitro-pyridin in DMF (30 ml) wurde mit 3,44 g (20,0 mmol) (2-Chlorethyl)(phenyl)sulfan und 5,53 g (40,0 mmol) Kaliumcarbonat versetzt und 4 h auf 60 °C erhitzt. Anschließend wurde mit EE verdünnt und mit Brine gewaschen. Die organische Phase wurde abgetrennt, über $Na_2SO_4$ getrocknet, filtriert und im Vakuum eingeengt. Durch SC (Hexan / EE 9:1) des Rückstands wurden 1,93 g (6,6 mmol, 66%) 3-Nitro-2-(2-phenylsulfanyl-ethylsulfanyl)-pyridin erhalten.

b) Synthese von [2-(2-Phenylsulfanyl-ethylsulfanyl)-pyridin-3-yl]-amin Ausgehend von 1,46 g (5,0 mmol) 3-Nitro-2-(2-phenylsulfanyl-ethylsulfanyl)-pyridin wurden nach dem für Vorstufe VPF-001 Schritt b) beschriebenen Verfahren 878 mg (3,4 mmol, 67%) [2-(2-Phenylsulfanyl-ethylsulfanyl)-pyridin-3-yl]-amin erhalten.

## Synthese weiterer Zwischenprodukte

Die Synthese weiterer Zwischenprodukte erfolgte nach den bereits beschriebenen Verfahren. In Tabelle 1 ist angegeben, welche Verbindung nach welchem Verfahren hergestellt wurde. Dem Fachmann ist dabei ersichtlich, welche Ausgangsstoffe und Reagenzien jeweils eingesetzt wurden.

**Tabelle 1:**

| Zwischenprodukt | Chemische Bezeichnung | Herstellung analog Zwischenprodukt | Ausbeute [%] |
|---|---|---|---|
| **VPF-007** | [2-[2-[[3-(Trifluormethyl)phenyl]sulfanyl]-ethylsulfanyl]-pyridin-3-yl]-amin | VPF-006 | 38 (2 Stufen) |
| **VPF-008** | [2-(3-Phenyl-propylsulfanyl)-pyridin-3-yl]-amin | VPF-006 | 34 (2 Stufen) |
| **VPF-009** | [2-[3-[3-(Trifluormethyl)phenyl)-propylsulfanyl]-pyridin-3-yl]-amin | VPF-006 | 46 (2 Stufen) |
| **VPF-010** | [2-(2-Phenoxy-ethylsuifanyl)-pyridin-3-yl]-amin | VPF-006 | 47 (2 Stufen) |
| **VPF-011** | [2-[2-[3-(Trifluormethyl)-phenoxy]-ethylsulfanyl]-pyridin-3-yl]-amin | VPF-006 | 44 (2 Stufen) |
| **VPF-012** | [4-Methyl-2-[2-[[3-(trifluormethyl)-phenyl]sulfonyl]-ethylsulfanyl]-pyridin-3-yl]-amin | VPF-001 | 44 (2 Stufen) |
| **VPF-013** | [2-[2-[(4-Fluorphenyl)sulfanyl]-ethylsulfanyl]-pyridin-3-yl]-amin | VPF-001 | 20 (2 Stufen) |
| **VPF-014** | [2-[2-(4-Fluor-phenoxy)-ethylsulfanyl]-pyridin-3-yl]-amin | VPF-006 | 56 (2 Stufen) |
| **VPF-015** | [2-[3-(4-Fluorphenyl)-propylsulfanyl]-pyridin-3-yl]-amin | VPF-006 | 48 (2 Stufen) |
| **VPF-016** | [2-[2-[(4-Fluorphenyl)sulfonyl]-ethylsulfanyl]-pyridin-3-yl]-amin | VPF-001 | 53 (2 Stufen) |

## Synthese der Beispielverbindungen

### Synthese der Beispielverbindung 1: N-[2-[2-(Benzolsulfonyl)-ethylsulfanyl]-pyridin-3-yl]-2-cyclohexyl-acetamid

Eine Lösung von 294 mg (1,0 mmol) [2-[2-(Benzolsulfonyl)-ethylsulfanyl]-pyridin-3-yl]-amin (VPF-002) in Dioxan (10 ml) wurde mit 336 mg (4,0 mmol) $NaHCO_3$ versetzt und 10 min bei RT gerührt. Anschließend wurden 321 mg (2,0 mmol) 2-Cyclohexylacetylchlorid bei 5 °C zugegeben und es wurde weitere 16 h bei RT gerührt. Dann wurde mit EE verdünnt und durch Kieselgur filtriert. Das Filtrat wurde mit einer ges. aq. $NaHCO_3$-Lsg, Wasser und Brine gewaschen, über $Na_2SO_4$ getrocknet, filtriert und im Vakuum eingeengt. Durch SC (Hexan / EE 6:1) mit dem Rückstand wurden 368 mg (0,9 mmol, 88%) N-[2-[2-(Benzolsulfonyl)-ethyl-sulfanyl]-pyridin-3-yl]-2-cyclohexyl-acetamid erhalten. MS: m/z 419,1 $[M+H]^+$.

### Synthese der Beispielverbindung 9: 2-(2-Methoxyphenyl)-N-[2-[2-[[3-(trifluormethyl)-phenyl]sulfonyl]-ethylsulfanyl]-pyridin-3-yl]-acetamid

Eine Lösung von 582 mg (3,5 mmol) 2-(2-Methoxyphenyl)essigsäure in DCM (10 ml) wurde mit 1,7 g (4,0 mmol) HATU und 1,36 ml (8,0 mmol) DIPEA versetzt, auf 0 °C abgekühlt. Nach 15 min Rühren wurde bei 0 °C eine Lösung von 725 mg (2,0 mmol) [2-[2-[[3-(Trifluor-methyl)phenyl]sulfonyl]-ethylsulfanyl]-pyridin-3-yl]-amin (VPF-001) in DCM (6 ml) zugegeben. Danach wurde 16 h bei RT gerührt. Dann wurde mit DCM verdünnt und nacheinander mit einer ges. aq. $NH_4Cl$-Lsg, einer ges. aq. $NaHCO_3$-Lsg und Brine gewaschen. Die organische Phase wurde über $Na_2SO_4$ getrocknet, filtriert und im Vakuum eingeengt. Durch SC (Hexan / EE 7:3) mit dem Rückstand wurden 827 mg (1,6 mmol, 81%) 2-(2-methoxyphenyl)-N-[2-[2-[[3-(trifluormethyl)-phenyl]sulfonyl]-ethylsulfanyl]-pyridin-3-yl]-acetamid erhalten. MS: m/z 511,1 $[M+H]^+$.

### Synthese der Beispielverbindung 12: 2-(2-Hydroxyphenyl)-N-[2-[2-[[3-(trifluormethyl)-phenyl]sulfonyl]-ethylsulfanyl]-pyridin-3-yl]-acetamid

Eine Lösung von 511 mg (1,0 mmol) 2-(2-methoxyphenyl)-N-[2-[2-[[3-(trifluormethyl)-phenyl]sulfonyl]-ethylsulfanyl]-pyridin-3-yl]-acetamid (Beispielverbindung 9) in DCM (9 ml) wurde bei 10 °C mit 214 μl (2,5 mmol) $BBr_3$ versetzt und 2 h bei dieser Temperatur gerührt.

Dann wurde mit Wasser gequencht und mit DCM extrahiert. Die organische Phase wurde mit einer ges. aq. $NaHCO_3$-Lsg und Brine gewaschen, über $Na_2SO_4$ getrocknet, filtriert und im Vakuum eingeengt. Durch SC (Hexan / EE 3:2) mit dem Rückstand wurden 338 mg (0,7 mmol, 68%) 2-(2-hydroxyphenyl)-N-[2-[2-[[3-(trifluormethyl)-phenyl]sulfonyl]-ethylsulfanyl]-pyridin-3-yl]-acetamid erhalten. MS: m/z 497,1 $[M+H]^+$.

### Synthese der Beispielverbindung 14: 2-Cyclopentyl-N-[2-[2-[[3-(trifluormethyl)-phenyl]sulfonyl]-ethylsulfanyl]-pyridin-3-yl]-acetamid

Eine Lösung von 192 mg (1,5 mmol) 2-Cyclopentylessigsäure in DCM (10 ml) wurde mit 254 μl (3,0 mmol) Oxalylchlorid versetzt und 3 h bei RT gerührt. Danach wurde im Vakuum eingeengt und der Rückstand wurde mit Dioxan (6 ml) aufgenommen. Diese Lösung wurde bei 0 °C zu einer Lösung von 362 mg (1,0 mmol) [2-[2-[[3-(Trifluormethyl)phenyl]sulfonyl]-ethylsulfanyl]-pyridin-3-yl]-amin (VPF-001) in Dioxan (5 ml) gegeben. Anschließend wurde die Reaktionslösung mit 420 mg (5,0 mmol) $NaHCO_3$ versetzt und 16 h bei RT gerührt. Dann wurde im Vakuum eingeengt und der Rückstand wurde mit Wasser aufgenommen. Dann wurde mit EE extrahiert und die organische Phase wurde mit einer ges. aq. $NaHCO_3$-Lsg und Brine gewaschen, über $Na_2SO_4$ getrocknet, filtriert und im Vakuum eingeengt. Durch SC (Hexan / EE 3:1) mit dem Rückstand wurden 231 mg (0,5 mmol, 54%) 2-Cyclopentyl-N-[2-[2-[[3-(trifluormethyl)-phenyl]sulfonyl]-ethylsulfanyl]-pyridin-3-yl]-acetamid erhalten. MS: m/z 473,1 $[M+H]^+$.

### Synthese der Beispielverbindung 21: N-[2-(2-Phenylsulfanyl-ethylsulfanyl)-pyridin-3-yl]-2-thiophen-2-yl-acetamid

Eine Lösung von 262 mg (1,0 mmol) [2-(2-Phenylsulfanyl-ethylsulfanyl)-pyridin-3-yl]-amin (VPF-006) in Dioxan (25 ml) wurde bei 0 °C mit 420 mg (5,0 mmol) $NaHCO_3$ und 402 mg (2,5 mmol) 2-(Thiophen-2-yl)essigsäurechlorid versetzt und anschließend 16 h bei RT gerührt. Dann wurde mit EE verdünnt und mit Wasser und Brine gewaschen. Die organische Phase wurde über $Na_2SO_4$ getrocknet, filtriert und im Vakuum eingeengt. Durch SC (Hexan / EE 9:1) mit dem Rückstand wurden 186 mg (0,5 mmol, 48%) N-[2-(2-Phenylsulfanyl-ethylsulfanyl)-pyridin-3-yl]-2-thiophen-2-yl-acetamid erhalten. MS: m/z 387,1 $[M+H]^+$.

**Synthese der Beispielverbindung 50: 4-Fluor-2-methoxy-N-[2-[2-[[3-(trifluormethyl)-phenyl]sulfonyl]-ethylsulfanyl]-pyridin-3-yl]-benzamid**

Eine Lösung von 510 mg (3,0 mmol) 4-Fluor-2-methoxybenzoesäure in Thionylchlorid (3 ml) wurde 2 h bei RT gerührt. Danach wurde im Vakuum eingeengt und der Rückstand wurde mit Dioxan (15 ml) aufgenommen. Dann wurde mit einer Lösung von 1,09 g (3,0 mmol) [2-[2-[[3-(Trifluormethyl)phenyl]sulfonyl]-ethylsulfanyl]-pyridin-3-yl]-amin (VPF-001) in Dioxan (15 ml) und mit 1,06 g (10,0 mmol) $Na_2CO_3$ versetzt und 16 h bei RT gerührt. Anschließend wurde im Vakuum eingeengt und de Rückstand wurde mit Wasser aufgenommen und mit EE extrahiert. Die organische Phase wurde mit einer ges. aq. $NaHCO_3$-Lsg und Brine gewaschen, über $Na_2SO_4$ getrocknet, filtriert und im Vakuum eingeengt. Durch Kristallisaton (DCM / Hexan) des Rückstand wurden 895 mg (1,7 mmol, 58%) 4-Fluor-2-methoxy-N-[2-[2-[[3-(trifluormethyl)-phenyl]sulfonyl]-ethylsulfanyl]-pyridin-3-yl]-benzamid erhalten. MS: m/z 514,1 $[M+H]^+$.

**Synthese der Beispielverbindung 51: 4-Fluor-2-hydroxy-N-[2-[2-[[3-(trifluormethyl)-phenyl]sulfonyl]-ethylsulfanyl]-pyridin-3-yl]-benzamid**

Eine Lösung von 515 mg (1,0 mmol) 4-Fluor-2-methoxy-N-[2-[2-[[3-(trifluormethyl)-phenyl]sulfonyl]-ethylsulfanyl]-pyridin-3-yl]-benzamid (Beispielverbindung 50) in DCM (5 ml) wurde auf -78 °C abgekühlt. Bei dieser Temperatur wurden 5 ml (1 M in DCM, 5,0 mmol) $BBr_3$ zugegeben und es wurde 1 h bei -78 °C gerührt. Anschließend wurde die Reaktionslösung auf eine Eis-Wasser-mischung gegossen und es wurde mit $NaHCO_3$ auf pH 8 gestellt. Dann wurde mit DCM extrahiert und die organische Phase wurde mit Wasser und Brine gewaschen, über $Na_2SO_4$ getrocknet, filtriert und im Vakuum eingeengt. Als Rückstand wurde 290 mg (0,6 mmol, 58%) 4-Fluor-2-hydroxy-N-[2-[2-[[3-(trifluormethyl)-phenyl]sulfonyl]-ethylsulfanyl]-pyridin-3-yl]-benzamid erhalten. MS: m/z 501,0 $[M+H]^+$.

**Synthese der Beispielverbindung 55: 2-(3-Oxo-cyclohexyl)-N-[2-[2-[[3-(trifluormethyl)phenyl]sulfonyl]-ethylsulfanyl]-pyridin-3-yl]-acetamid**

Eine Lösung von 550 mg (35.2 mmol) 2-(3-oxocyclohexyl)essigsäure in DCM (7 ml) wurde bei 0 °C mit 560 µl (4.23 mmol) 1-Chloro-N,N,2-trimethylprop-1-en-1-amin versetzt. Nach 15 min Rühren bei 0°C wurde mit 1.5 g (4.23 mmol) [2-[2-[[3-(Trifluor-methyl)phenyl]sulfonyl]-ethylsulfanyl]-pyridin-3-yl]-amin (VPF-001) versetzt. Anschließend wurde die Reaktionslösung weitere 16 h bei 0°C gerührt. Danach wurde mit Wasser (30 ml) verdünnt und mit DCM (3 x 50 ml) extrahiert. Die vereinten organischen Phasen wurde mit Wasser und Brine gewaschen, über $Na_2SO_4$ getrocknet, filtriert und im Vakuum eingeengt. Nach SC (Hexan / EE 6:4) des Rückstands wurden 220 mg (0,44 mmol, 13%) 2-(3-Oxo-cyclohexyl)-N-[2-[2-[[3-(trifluormethyl)phenyl]sulfonyl]-ethylsulfanyl]-pyridin-3-yl]-acetamid erhalten. MS: m/z 501,1 $[M+H]^+$.

**Synthese der Beispielverbindung 60: 2-Pyridin-2-yl-N-[2-[2-[[3-(trifluormethyl)phenyl]sulfonyl]-ethylsulfanyl]-pyridin-3-yl]-acetamid**

Eine Lösung von 362 mg (1.0 mmol)) [2-[2-[[3-(Trifluormethyl)phenyl]sulfonyl]-ethylsulfanyl]-pyridin-3-yl]-amin (VPF-001) in Xylol (4 ml) wurde mit 302 mg (2.0 mmol) Methyl 2-(pyridin-2-yl)acetat versetzt und anschließend 7h auf 150°C erhitzt. Nach Abkühlen auf RT wurde mit EtOAc verdünnt und die Phasen wurden getrennt. Die organische Phase wurde mit Wasser und Brine gewaschen, über $Na_2SO_4$ getrocknet, filtriert und im Vakuum eingeengt. Nach SC (Hexan / EE 6:4) mit dem Rückstand wurden 154 mg (0,32 mmol, 32%) 2-Pyridin-2-yl-N-[2-[2-[[3-(trifluormethyl)phenyl]sulfonyl]-ethylsulfanyl]-pyridin-3-yl]-acetamid erhalten. MS: m/z 482,1 $[M+H]^+$.

**Synthese weiterer Beispielverbindungen**

Die Synthese weiterer Beispielverbindungen erfolgte nach den bereits beschriebenen Verfahren. In Tabelle 2 ist angegeben, welche Verbindung nach welchem Verfahren hergestellt wurde. Dem Fachmann ist dabei ersichtlich, welche Ausgangsstoffe und Reagenzien jeweils eingesetzt wurden.

**Tabelle 2:**

| Beispiel | Chemische Bezeichnung | Herstellung analog Beispiel | Ausbeute [%] | MS m/z $[M+H]^+$ |
|---|---|---|---|---|
| **2** | 2-Cyclohexyl-N-[2-[2-[[3-(trifluormethyl)phenyl]-sulfonyl]-ethylsulfanyl]-pyridin-3-yl]-acetamid | 1 | 89 | 487,1 |
| **3** | 2-Thiophen-2-yl-N-[2-[2-[[3-(trifluormethyl)phenyl]-sulfonyl]-ethylsulfanyl]-pyridin-3-yl]-acetamid | 1 | 57 | 487 |
| **4** | N-[2-[2-(Benzolsulfonyl)-ethylsulfanyl]-pyridin-3-yl]-benzamid | 1 | 92 | 399,1 |
| **5** | N-[2-[2-(Benzolsulfonyl)-ethylsulfanyl]-pyridin-3-yl]-3,4-difluor-benzamid | 1 | 53 | 435,1 |
| **6** | N-[2-[2-(Benzolsulfonyl)-ethylsulfanyl]-pyridin-3-yl]-3-cyclohexyl-propionamid | 1 | 50 | 433,2 |
| **7** | N-[2-[2-(Benzolsulfonyl)-ethylsulfanyl]-pyridin-3-yl]-2-thiophen-2-yl-acetamid | 1 | 56 | 419 |
| **8** | N-[2-[2-(Benzolsulfonyl)-ethylsulfanyl]-pyridin-3-yl]-2-(3,5-dimethyl-phenyl)-propionamid | 1 | 42 | 455,1 |
| **10** | 2-(4-Methoxyphenyl)-N-[2-[2-[[3-(trifluor-methyl)phenyl]-sulfonyl]-ethylsulfanyl]-pyridin-3-yl]-acetamid | 9 | 83 | 511,1 |
| **11** | 2-(3-Methoxyphenyl)-N-[2-[2-[[3-(trifluormethyl)-phenyl]sulfonyl]-ethylsulfanyl]-pyridin-3-yl]-acetamid | 9 | 71 | 511,1 |
| **13** | 2-(4-Hydroxyphenyl)-N-[2-[2-[[3-(trifluormethyl)phenyl]sulfonyl]-ethylsulfanyl]-pyridin-3-yl]-acetamid | 12 | 56 | 497,1 |
| **18** | N-[2-[2-[[3-(Trifluormethyl)phenyl]sulfonyl]-ethylsulfanyl]-pyridin-3-yl]-thiophen-2-carbonsäure amid | 14 | 58 | 473 |
| **19** | N-[2-[2-[[3-(Trifluormethyl)phenyl]sulfonyl]-ethylsulfanyl]-pyridin-3-yl]-cyclohexancarbonsäure amid | 14 | 60 | 473,1 |
| **20** | 2-Thiophen-2-yl-N-[2-[2-[[3-(trifluormethyl)phenyl]sulfanyl]-ethylsulfanyl]-pyridin-3-yl]-acetamid | 21 | 72 | 455 |
| **22** | 2-Thiophen-2-yl-N-[2-[2-[3-(trifluormethyl)-phenoxy]-ethylsulfanyl]-pyridin-3-yl]-acetamid | 21 | 63 | 439,1 |
| **23** | 2-Thiophen-2-yl-N-[2-[3-[3-(trifluormethyl)phenyl]-propylsulfanyl]-pyridin-3-yl]-acetamid | 21 | 72 | 437,1 |
| **24** | 2-Naphthalin-2-yl-N-[2-[2-[[3-(trifluormethyl)phenyl]sulfonyl]-ethylsulfanyl]-pyridin-3-yl]-acetamid | 14 | 73 | 531,1 |
| **25** | 4-Phenyl-N-[2-[2-[[3-(trifluormethyl)phenyl]sulfonyl]-ethylsulfanyl]-pyridin-3-yl]-butyramid | 14 | 72 | 509,1 |
| **26** | 3-Thiophen-2-yl-N-[2-[2-[[3-(trifluormethyl)phenyl]sulfonyl]-ethylsulfanyl]-pyridin-3-yl]-propionamid | 14 | 57 | 501,1 |
| **27** | 3-Phenyl-N-[2-[2-[[3-(trifluormethyl)phenyl]sulfonyl]-ethylsulfanyl]-pyridin-3-yl]-propionamid | 14 | 53 | 495,1 |
| **28** | 3-Cyclopentyl-N-[2-[2-[[3-(trifluormethyl)phenyl]sulfonyl]-ethylsulfanyl]-pyridin-3-yl]-propionamid | 14 | 75 | 487,1 |
| **29** | 2-Cyclohexyl-N-[2-[2-[[3-(trifluormethyl)phenyl]sulfanyl]-ethylsulfanyl]-pyridin-3-yl]-acetamid | 21 | 44 | 455,1 |
| **31** | (E)-3-(4-Fluorphenyl)-N-[2-[2-[[3-(trifluormethyl)phenyl]sulfonyl]-ethylsulfanyl]-pyridin-3-yl]-acrylamid | 14 | 63 | 511,1 |

(fortgesetzt)

| **Beispiel** | **Chemische Bezeichnung** | **Herstellung analog Beispiel** | **Ausbeute [%]** | **MS m/z $[M+H]^+$** |
|---|---|---|---|---|
| **33** | N-[2-(3-Phenyl-propylsulfanyl)-pyridin-3-yl]-2-thiophen-2-yl-acetamid | 21 | 62 | 369,1 |
| **35** | 6-Chlor-N-[2-[2-[[3-(trifluormethyl)phenyl]sulfonyl]-ethylsulfanyl]-pyridin-3-yl]-pyridin-3-carbonsäure amid | 14 | 77 | 502 |
| **36** | 2-Pyridin-4-yl-N-[2-[2-[[3-(trifluormethyl)phenyl]sulfonyl]-ethylsulfanyl]-pyridin-3-yl]-acetamid | 14 | 44 | 482,1 |
| **37** | 2-(3-Hydroxyphenyl)-N-[2-[2-[[3-(trifluormethyl)phenyl]sulfonyl]-ethylsulfanyl]-pyridin-3-yl]-acetamid | 12 | 58 | 497,1 |
| **38** | N-[2-[2-[[3-(Trifluormethyl)phenyl]sulfonyl]-ethylsulfanyl]-pyridin-3-yl]-tetrahydro-pyran-3-carbonsäure amid | 14 | 65 | 475,1 |
| **39** | 2-Tetrahydro-pyran-2-yl-N-[2-[2-[[3-(trifluormethyl)phenyl]sulfonyl]-ethylsulfanyl]-pyridin-3-yl]-acetamid | 14 | 58 | 489,1 |
| **40** | 2-Tetrahydro-pyran-4-yl-N-[2-[2-[[3-(trifluormethyl)phenyl]sulfonyl]-ethylsulfanyl]-pyridin-3-yl]-acetamid | 14 | 61 | 489,1 |
| **41** | 3-Cyclohexyl-N-[2-[2-[[3-(trifluormethyl)phenyl]sulfonyl]-ethylsulfanyl]-pyridin-3-yl]-propionamid | 14 | 77 | 501,1 |
| **44** | 2-Cyclohexyl-N-[2-(2-phenylsulfanyl-ethylsulfanyl)-pyridin-3-yl]-acetamid | 21 | 27 | 387,1 |
| **45** | N-[2-(2-Phenoxy-ethylsulfanyl)-pyridin-3-yl]-2-thiophen-2-yl-acetamid | 21 | 66 | 371,1 |
| **46** | 2-Pyridin-3-yl-N-[2-[2-[[3-(trifluormethyl)phenyl]sulfonyl]-ethylsulfanyl]-pyridin-3-yl]-acetamid | 14 | 55 | 482,1 |
| **47** | 3-Hydroxy-N-[2-[2-[[3-(trifluormethyl)phenyl]sulfonyl]-ethylsulfanyl]-pyridin-3-yl]-benzamid | 14 | 52 | 483,1 |
| **48** | N-[2-[2-[[3-(Trifluormethyl)phenyl]sulfonyl]-ethylsulfanyl]-pyridin-3-yl]-tetrahydro-pyran-2-carbonsäure amid | 14 | 45 | 475,1 |
| **49** | 2-Cycloheptyl-N-[2-[2-[[3-(trifluormethyl)phenyl]sulfonyl]-ethylsulfanyl]-pyridin-3-yl]-acetamid | 14 | 64 | 501,1 |
| **52** | 2-(1,2,3,4-Tetrahydro-naphthalen-2-yl)-N-[2-[2-[[3-(trifluormethyl)phenyl]sulfonyl]-ethylsulfanyl]-pyridin-3-yl]-acetamid | 14 | 58 | 535,1 |
| **53** | 2-Hydroxy-N-[2-[2-[[3-(trifluormethyl)phenyl]sulfonyl]-ethylsulfanyl]-pyridin-3-yl]-benzamid | 14 | 31 | 483,1 |
| **56** | N-[4-Methyl-2-[2-[[3-(trifluormethyl)phenyl]sulfonyl]-ethylsulfanyl]-pyridin-3-yl]-2-thiophen-2-yl-acetamid | 1 | 49 | 501,1 |
| **57** | 3-(1,2,3,4-Tetrahydro-naphthalen-2-yl)-N-[2-[2-[[3-(trifluormethyl)phenyl]sulfonyl]-ethylsulfanyl]-pyridin-3-yl]-propionamid | 14 | 52 | 549,1 |
| **58** | 3-Cycloheptyl-N-[2-[2-[[3-(trifluormethyl)phenyl]sulfonyl]-ethylsulfanyl]-pyridin-3-yl]-propionamid | 14 | 47 | 515,2 |

(fortgesetzt)

| Beispiel | Chemische Bezeichnung | Herstellung analog Beispiel | Ausbeute [%] | MS m/z $[M+H]^+$ |
|---|---|---|---|---|
| **59** | 2-(Benzo[b]thiophen-2-yl)-N-[2-[2-[[3-(trifluormethyl)phenyl]sulfonyl]-ethylsulfanyl]-pyridin-3-yl]-acetamid | 14 | 54 | 537,1 |
| **61** | N-[2-[2-[(4-Fluorphenyl)sulfanyl]-ethylsulfanyl]-pyridin-3-yl]-3,3-dimethyl-butyramid | 1 | 74 | 379,1 |
| **62** | 2-(5-Bicyclo[2.2.1]heptanyl)-N-[2-[2-[[3-(trifluormethyl)phenyl]sulfonyl]-ethylsulfanyl]-pyridin-3-yl]-acetamid | 14 | 55 | 499,1 |
| **63** | N-[2-[2-(4-Fluor-phenoxy)-ethylsulfanyl]-pyridin-3-yl]-3,3-dimethyl-butyramid | 1 | 35 | 363,1 |
| **64** | 3,4-Difluor-N-[2-[2-(4-fluor-phenoxy)-ethylsulfanyl]-pyridin-3-yl]-benzamid | 1 | 55 | 405,1 |
| **65** | 3,4-Difluor-N-[2-[3-(4-fluorphenyl)-propylsulfanyl]-pyridin-3-yl]-benzamid | 1 | 60 | 403,1 |
| **66** | N-[2-[3-(4-Fluorphenyl)-propylsulfanyl]-pyridin-3-yl]-3,3-dimethyl-butyramid | 1 | 48 | 361,2 |
| **67** | 2-Cycloheptyl-N-[2-[2-[(4-fluorphenyl)sulfonyl]-ethylsulfanyl]-pyridin-3-yl]-acetamid | 14 | 18 | 451,1 |
| **68** | 2-Cyclohexyl-2,2-difluor-N-[2-[2-[[3-(trifluormethyl)phenyl]sulfonyl]-ethylsulfanyl]-pyridin-3-yl]-acetamid | 55 | 11 | 523,1 |

**Pharmakologische Experimente**

**Fluoreszenzassay unter Verwendung eines 'voltage sensitive dyes'**

Humane, KCNQ2/3-Kanäle exprimierende CHO-K1-Zellen werden in Zellkultur-flaschen (z.B. 80 $cm^2$ TC flasks, Nunc) mit DMEM-high glucose (Sigma Aldrich, D7777) inklusive 10% FCS (PAN Biotech, z.B. 3302-P270521) oder alternativ MEM Alpha Medium (1x, flüssig, Invitrogen, #22571), 10 % Fetal Calf Serum (FCS) (Invitrogen, #10270-106, hitzeinaktiviert) und den notwendigen Selektionsantibiotika bei 37°C, 5 % $CO_2$ und 95 % Luftfeuchtigkeit adhärent kultiviert.

Vor Aussaat für die Messungen werden die Zellen mit einem 1 x DPBS-Puffer ohne $Ca^{2+}/Mg^{2+}$ (z.B. Invitrogen, #14190-094) gewaschen und mittels Accutase (PAA Laboratories, #L11-007) vom Boden des Kulturgefäßes abgelöst (Inkubation mit Accutase für 15 min bei 37°C). Die Bestimmung der dann vorliegenden Zellzahl wird mit einem CASY™ cell counter (Modell TCC, Schärfe System) durchgeführt, um anschließend je nach Dichteoptimierung für die individuelle Zelllinie 20.000 - 30.000 Zellen/well/100 µl des beschriebenen Nährmediums auf die 96 well-Messplatten des Typs Corning™ CellBIND™ (Flat Clear Bottom Black Polystyrene Microplates, #3340) auszubringen. Danach erfolgt eine einstündige Inkubation bei Raumtemperatur ohne Begasung oder Regelung der Luftfeuchtigkeit, gefolgt von einer 24stündigen Inkubation bei 37°C, 5 % $CO_2$ und 95 % Luftfeuchtigkeit.

Der spannungssensitive Fluoreszenzfarbstoff aus dem Membrane Potential Assay Kit (Red™ Bulk format part R8123 for FLIPR, MDS Analytical Technologies™) wird vorbereitet, indem der Inhalt eines Gefäßes *Membrane Potential Assay Kit Red Component A* in 200 ml Extrazellulären Puffers (ES-Puffer, 120 mM NaCl, 1 mM KCl, 10 mM HEPES, 2 mM $CaCl_2$, 2 mM $MgCl_2$, 10 mM Glucose; pH 7,4) gelöst wird. Nach Abnahme des Nährmediums werden die Zellen mit 200 µl ES-Puffer gewaschen, anschließend mit 100 µl der oben angesetzten Farbstofflösung überschichtet und 45 min bei Raumtemperatur unter Lichtverschluss inkubiert.

Die Fluoreszenzmessungen werden mit einem BMG Labtech FLUOstar™-, BMG Labtech NOVOstar™- oder BMG Labtech POLARstar™-Instrumentdurchgeführt (525 nm Exication, 560 nm Emission, Bottom Read mode). Nach der Farbstoff-Inkubation werden 50 µl der zu testenden Substanzen in den gewünschten Konzentrationen oder 50 µl ES-Puffer zwecks Kontrolle in separate Cavitäten der Messplatte gegeben und 30 min bei Raumtemperatur unter Abschirmung von Licht inkubiert. Anschließend misst man für 5 min die Fluoreszenzintensität des Farbstoffs und ermittelt so zu einem festgelegten und gleichbleibenden Zeitpunkt den Fluoreszenzwert $F_1$ eines jeden wells. Daraufhin erfolgt Zugabe von 15 µl einer 100 mM KCl-Lösung (Endkonzentration 92 mM) in jedes well. Die Veränderung der Fluoreszenz wird anschließend so lange gemessen, bis alle relevanten Messwerte erhalten sind (vornehmlich 5-30 min). Zu einem festgelegten Zeitpunkt nach KCl-Applikation wird ein Fluoreszenzwert $F_2$ ermittelt, in diesem Falle zum Zeitpunkt des Fluoreszenzpeaks.

Zur Berechnung wird die Fluoreszenzintensität $F_2$ mit der Fluoreszenzintensität $F_1$ verglichen und daraus die agonistische Aktivität der Zielverbindung auf den Kaliumkanal ermittelt. $F_2$ und $F_1$ werden hierfür wie folgt verrechnet:

$$\left(\frac{F_2 - F_1}{F_1}\right) \times 100 = \frac{\Delta F}{F}(\%)$$

Um zu ermitteln, ob eine Substanz eine agonistische Aktivität besitzt, kann z.B. $\frac{\Delta F}{F}$ mit $\left(\frac{\Delta F}{F}\right)_K$ von Kontrollzellen verglichen werden. $\left(\frac{\Delta F}{F}\right)_K$ wird ermittelt, indem man dem Reaktionsansatz anstatt der zu testenden Substanz lediglich die Pufferlösung zusetzt, den Wert $F_{1K}$ der Fluoreszenzintensität bestimmt, die Kaliumionen wie oben beschrieben zugibt und einen Wert $F_{2K}$ der Fluoreszenzintensität misst. Dann werden $F_{2K}$ und $F_{1K}$ wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Unabhängig vom Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist. Kalkulationen von $EC_{50}$-Werten werden mit Hilfe der Software 'Prism v4.0' (GraphPad Software™) durchgeführt.

**Formalintest Maus**

Der Formalin Test (Dubuisson, D. and Dennis, S.G., 1977, Pain, 4, 161 - 174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustem erfasst wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 20 µl und einer Konzentration von 1 % subkutan in die dorsale Seite der rechten Hinterpfote jedes Tieres appliziert. Die spezifischen Verhaltensänderungen, wie Anheben, Schütteln oder Lecken der Pfote (score 3, Dubuisson & Dennis, 1977), werden im Beobachtungszeitraum von 21 bis 24 min nach Formalininjektion beobachtet und registriert. Das Verhalten der Tiere nach Substanzgabe (n = 10 pro Substanzdosierung) wurde mit einer Kontrollgruppe, welcher Vehikel appliziert wurde (n=10) verglichen.

Basierend auf der Quantifizierung des Schmerzverhaltens wurde die Substanzwirkung im Formalin-Test als Änderung gegenüber der Kontrolle in Prozent ermittelt. Die Berechnungen der $ED_{50}$ ($ED_{50}$ = mittlere effektive Dosis) erfolgten mittels Regressionsanalyse nach der Methode von Litchfield and Wilcoxon (Litchfield, J.T., Wilcoxon, J.J., 1949, J. Pharmacol. Exp. Ther. 96, 99 - 113). Der Applikationszeitpunkt der Verbindung vor der Formalin-Injektion war bei intravenöser Applikation 5 min und bei oraler Applikation 30 min.

**Pharmakologische Daten**

In Tabelle 3 sind die Ergebnisse aus den zuvor beschriebenen pharmakologischen Modellen zusammengefasst.

Tabelle 3

| Bsp-Nr. | Fluoriemtrie % Efficacy (I/EC50, Retigabine =100%) | Fluoriemtrie EC50 [nM] | Fluoriemtrie IC50 [nM] | Formalin Test Maus IV Effekt @ Dosis [mg/kg] |
|---|---|---|---|---|
| **1** | 172 | 95 | | |
| **2** | 142 | 27 | | |
| **3** | 164 | 31 | | 87% @ 4.64 |
| **4** | 55 | | | |
| **5** | 129 | 469 | | |
| **6** | 225 | 76 | | |
| **7** | 125 | 424 | | |

(fortgesetzt)

| Bsp-Nr. | Fluoriemtrie % Efficacy (I/EC50, Retigabine =100%) | Fluoriemtrie EC50 [nM] | Fluoriemtrie IC50 [nM] | Formalin Test Maus IV Effekt @ Dosis [mg/kg] |
|---|---|---|---|---|
| **8** | -68 | | 322 | |
| **9** | -45 | | 192 | |
| **10** | 74 | 55 | | |
| **11** | 109 | 588 | | |
| **12** | 141 | 168 | | |
| **13** | 46 | | | |
| **14** | 167 | 45 | | |
| **18** | 43 | 457 | | |
| **19** | -43 | | | |
| **20** | 97 | 180 | | |
| **21** | 152 | 83 | | |
| **22** | 155 | 732 | | |
| **23** | 165 | 157 | | |
| **24** | 84 | 153 | | |
| **25** | 170 | 71 | | |
| **26** | 82 | 123 | | |
| **27** | 112 | 208 | | |
| **28** | 152 | 277 | | |
| **29** | 108 | 312 | | |
| **31** | 109 | 841 | | |
| **33** | 106 | 141 | | |
| **35** | 43 | | | |
| **36** | 38 | | | |
| **37** | 31 | | | |
| **38** | 35 | | | |
| **39** | 139 | 363 | | |
| **40** | 52 | | | |
| **41** | 127 | 99 | | |
| **44** | 80 | 153 | | |
| **45** | 58 | | | |
| **46** | 50 | | | |
| **47** | 95 | 265 | | |
| **48** | 93 | 174 | | |
| **49** | 108 | 19 | | |
| **50** | | | | |
| **51** | 32 | 106 | | |

(fortgesetzt)

| Bsp-Nr. | Fluoriemtrie % Efficacy (I/EC50, Retigabine =100%) | Fluoriemtrie EC50 [nM] | Fluoriemtrie IC50 [nM] | Formalin Test Maus IV Effekt @ Dosis [mg/kg] |
|---|---|---|---|---|
| **52** | 59 | 208 | | |
| **53** | 27,45 | | | |
| **55** | 84,85 | 3600 | | |
| **56** | 98,1 | 348,5 | | |
| **57** | 134 | 56,5 | | |
| **58** | 167,4 | 47,5 | | |
| **59** | 138,55 | 925 | | |
| **60** | 101 | 1025 | | |
| **61** | 128,55 | 27,5 | | |
| **62** | 168,4 | 23,5 | | |
| **63** | 119 | 546 | | |
| **64** | 49 | 200 | | |
| **65** | 148 | 480 | | |
| **66** | 176 | 184 | | |
| **67** | 114 | 18 | | |
| **68** | 79 | 22 | | |

**Patentansprüche**

1. Substituierte 2-Mercapto-3-aminopyridine der allgemeinen Formel (1),

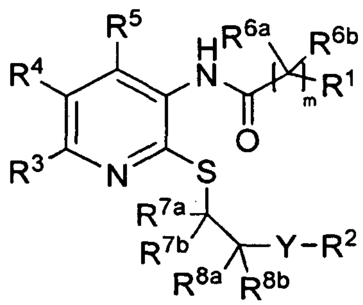

**(1)**

worin
$R^1$ für $C_{1-6}$-Alkyl, gesättigt oder ungesättigt, verzweigt oder unverzweigt, unsubstituiert oder einfach oder mehrfach substituiert; $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert steht;
mit der Maßgabe, dass, wenn $R^1$ Heterocyclyl bedeutet, die Bindung des Heterocyclyls an die übergeordnete allgemeine Struktur über ein Kohlenstoffatom des Heterocyclyls erfolgt;
$R^2$ für Aryl oder Heteroaryl, jeweils unsubstituiert oder einfach oder mehrfach substituiert steht;
Y ausgewählt ist aus der Gruppe bestehend aus -($CR^{9a}R^{9b}$)-, $SO_2$, S(=O), -S-, -O-, C(=O);
$R^3$, $R^4$, $R^5$, $R^{6a}$, $R^{6b}$, $R^{7a}$, $R^{7b}$, $R^{8a}$, $R^{8b}$, $R^{9a}$ und $R^{9b}$ jeweils unabhängig voneinander für H; F; Cl; Br; I; $NO_2$; $CF_3$;

CN; OH; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ oder C(=O)-$C_{1-6}$-Alkyl, jeweils gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, verzweigt oder unverzweigt, unsubstituiert oder einfach oder mehrfach substituiert; stehen,
wobei $R^{7a}$ mit $R^{8a}$ einen $C_{3-7}$-Cycloalkylrest bilden können, der gesättigt oder ungesättigt, unsubstituiert oder einfach oder mehrfach substituiert sein kann;
worin "Alkyl substituiert" für die Substitution eines oder mehrerer Wasserstoffatome jeweils unabhängig voneinander durch F; Cl; Br; I; $NO_2$; $CF_3$; CN; $C_{1-8}$-Alkyl; $C_{2-8}$-Heteroalkyl; Aryl; Heteroaryl; $C_{3-10}$-Cycloalkyl; Heterocyclyl; über $C_{18}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; und S(=O)$_2$-NH-$C_{1-8}$-Heteroaryl; steht;
worin "Heterocyclyl substituiert" und "Cycloalkyl substituiert" für die Substitution eines oder mehrerer Wasserstoffatome jeweils unabhängig voneinander durch F; Cl; Br; I; $NO_2$; $CF_3$; =O; CN; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl;
C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$. NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; und S(=O)$_2$-NH-$C_{1-8}$-Heteroaryl; steht;
worin "Aryl substituiert" und "Heteroaryl substituiert" für die Substitution eines oder mehrerer Wasserstoffatome jeweils unabhängig voneinander durch F; Cl; Br; I; $NO_2$; $CF_3$; CN; $C_{1-8}$-Alkyl; oder $C_{2-8}$-Heteroalkyl; Aryl; Heteroaryl; $C_{3-10}$-Cycloalkyl; Heterocyclyl; $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$CH_3$; C(=O)NH-$C_2H_5$; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$ ; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2C_{1-8}$-Alkyl; S(=O)$_2$Aryl; S(=O)$_2$Heteroaryl; S(=O)$_2$OH; S(=O)$_2$O-$C_{1-8}$-Alkyl; S(=)O$_2$O-Aryl; S(=O)$_2$O-Heteroaryl; S(=O)$_2$-NH-$C_{1-8}$-Alkyl; S(=O)$_2$-NH-Aryl; S(=O)$_2$-NH-$C_{1-8}$-Heteroaryl; steht.
in Form der freien Verbindungen oder Salze physiologisch verträglicher Säuren oder Basen.

2. Substituierte Aminopyridine gemäß Anspruch 1, **dadurch gekennzeichnet, dass** $R^1$ für Phenyl, Pyridyl oder Thienyl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CF_3$, CN, Methyl, Ethyl, n-Propyl, iso-Propyl, Cyclopropyl, n-Butyl, sec.-Butyl, tert.-Butyl, $CH_2CF_3$, C(=O)-Methyl, C(=O)-Ethyl, C(=O)-OH, C(=O)-O-Methyl, C(=O)-O-Ethyl, C(=O)-$NH_2$, C(=O)-N(Methyl)$_2$, C(=O)-N(Ethyl)$_2$, C(=O)-NH-Methyl, C(=O)-NH-Ethyl, C(=O)-N(Methyl)(Ethyl), OH, O-Methyl, 0-Ethyl, O-$(CH_2)_2$-O-$CH_3$, O-$(CH_2)_2$-OH, $OCF_3$, O-C(=O)-Methyl, O-C(=O)-Ethyl, $NR^aR^b$, wobei $R^a$ und $R^b$ jeweils unabhängig voneinander ausgewählt sind aus der Gruppe bestehend aus H, Methyl, Ethyl, $(CH_2)_2$-O-$CH_3$ und $(CH_2)_2$-OH oder $R^a$ und $R^b$ gemeinsam mit dem sie verbindenden Stickstoffatom ein Pyrrolidinyl, Piperidinyl, 4-Methylpiperazinyl oder Morpholinyl bilden, NHC(=O)-Methyl, NHC(=O)-Ethyl, SH, $SCF_3$, S-Methyl, S-Ethyl, S(=O)$_2$OH, S(=O)$_2$O-Methyl, Benzyl, Phenyl, Pyridyl, wobei Benzyl, Phenyl, Pyridyl jeweils unsubstituiert oder einfach, zweifach oder dreifach substituiert sind mit einem, zwei oder drei Substituenten jeweils unabhängig voneinander ausgewählt aus. der Gruppe bestehend aus F, Cl, Br, I, CN, Methyl, Ethyl, $CF_3$, OH, O-Methyl und $OCF_3$.

3. Substituierte Aminopyridine gemäß Anspruch 1 oder 2, **dadurch gekennzeichnet, dass** $R^1$ für Phenyl, Pyridyl oder

Thienyl steht, jeweils unsubstituiert oder einfach oder zweifach oder dreifach substituiert mit einem, zwei oder drei Substituenten jeweils unabhängig voneinander ausgewählt aus der Gruppe bestehend aus F, Cl, Br, I, $NO_2$, $CF_3$, CN, Methyl, Ethyl, C(=O)-Methyl, OH, O-Methyl, O-$(CH_2)_2$-O-$CH_3$, $OCF_3$, O-C(=O)-Methyl, $NH_2$, NH-C(=O)-Methyl, N(Methyl)$_2$, Morpholinyl, S-Methyl, $SCF_3$, Benzyl und Phenyl.

4. Substituierte Aminopyridine gemäß einem der Ansprüche 1 bis 3, **dadurch gekennzeichnet, dass** $R^2$ für Aryl oder Heteroaryl, jeweils unsubstituiert oder einfach oder mehrfach substituiert unsubstituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH.

5. Substituierte Aminopyridine gemäß einem der Ansprüche 1 bis 4, **dadurch gekennzeichnet, dass** der Rest $R^2$ für Aryl oder Heteroaryl steht, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH.

6. Substituierte Aminopyridine gemäß einem der Ansprüche 1 bis 5, **dadurch gekennzeichnet, dass** $R^{7a}$ , $R^{7b}$, $R^{8a}$, $R^{8b}$, $R^{9a}$ und $R^{9b}$ jeweils unabhängig voneinander für H; F; Cl; Br; I; $NO_2$; $CF_3$; $CH_2CF_3$; CN; OH; $OCF_3$, $NH_2$; $C_{1-4}$-Alkyl, O-$C_{1-4}$-Alkyl, O-$C_{1-4}$-Alkyl-OH, O-$C_{1-4}$-Alkyl-O-$CH_3$, NH-$C_{1-4}$-Alkyl, N($C_{1-4}$-Alkyl)$_2$, jeweils gesättigt oder ungesättigt, verzweigt oder unverzweigt, unsubstituiert; $C_{3-10}$-Cycloalkyl, gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, O-$C_{1-4}$-Alkyl.

7. Substituierte Aminopyridine gemäß einem der Ansprüche 1 bis 6, **dadurch gekennzeichnet, dass** $R^{7a}$ mit $R^{8a}$ einen 6-gliedrigen Cycloalkylrest bildet, der gesättigt oder ungesättigt, unsubstituiert oder einfach oder mehrfach substituiert sein kann.

8. Substituierte Aminopyridine gemäß einem der Ansprüche 1 bis 7, **dadurch gekennzeichnet, dass** m = 0 oder 1 ist.

9. Substituierte Aminopyridine gemäß einem der Ansprüche 1 bis 8, **dadurch gekennzeichnet, dass** steht Y für-(C$R^{9a}R^{9b}$)-.

10. Substituierte Aminopyridine gemäß einem der Ansprüche 1 bis 8, **dadurch gekennzeichnet, dass** $R^3$, $R^4$ und $R^5$ jeweils unabhängig voneinander für H; F; Cl; Br; I; $NO_2$; $CF_3$; CN; OH; $OCF_3$; SH; $SCF_3$; Methyl; Ethyl; n-Propyl; iso-Propyl; Butyl; sec.-Butyl; tert.-Butyl; $CH_2CF_3$; O-Methyl; O-Ethyl; O-n-Propyl; O-iso-Propyl; O-Butyl; O-sec.-Butyl; O-tert.-Butyl; O-$(CH_2)_2$-O-Methyl; O-$(CH_2)_2$-OH; O-(C=O)-Methyl; O-(C=O)-Ethyl; S-Methyl; S-Ethyl; Cyclopropyl; Cyclobutyl; N$R^aR^b$, wobei $R^a$ und $R^b$ jeweils unabhängig voneinander ausgewählt sind aus der Gruppe bestehend aus H, Methyl, Ethyl, $(CH_2)_2$-O-Methyl, $(CH_2)_2$-OH, (C=O)-Methyl, (C=O)-Ethyl oder $R^a$ und $R^b$ gemeinsam mit dem sie verbindenden Stickstoffatom ein Pyrrolidinyl, Piperidinyl, 4-Methylpiperazinyl oder Morpholinyl bilden.

11. Substituierte Aminopyridine gemäß einem der Ansprüche 1 bis 10, **dadurch gekennzeichnet, dass**
m für die Zahl 0 und $R^1$ für Aryl- oder Heteroaryl- steht;
m für die Zahl 1 und $R^1$ für Thienyl-, Phenyl-, einen $C_{3-8}$ Alkylrest, der gesättigt oder ungesättigt , unsubstituiert oder ein- oder mehrfach substituiert sein kann, oder einen mono- oder bicyclischen $C_{3-8}$-Cycloalkylrest steht, der gesättigt oder ungesättigt (aber nicht aromatisch), unsubstituiert oder ein- oder mehrfach substituiert sein kann; oder
m für die Zahl 2 und $R^1$ für Phenyl-, Cycloalkyl- oder Alkyl- steht.

12. Substituierte Aminopyridine gemäß einem der Ansprüche 1 bis 11 ausgewählt aus der Gruppe

**1** N-[2-[2-(Benzolsulfonyl)-ethylsulfanyl]-pyridin-3-yl]-2-cyclohexyl-acetamid;
**2** 2-Cyclohexyl-N-[2-[2-[[3-(trifluormethyl)phenyl]sulfonyl]-ethylsulfanyl]-pyridin-3-yl]-acetamid;

**3** 2-Thiophen-2-yl-N-[2-[2-[[3-(trifluormethyl)phenyl]sulfonyl]-ethylsulfanyl]-pyridin-3-yl]-acetamid;
**4** N-[2-[2-(Benzolsulfonyl)-ethylsulfanyl]-pyridin-3-yl]-benzamid;
**5** N-[2-[2-(Benzolsulfonyl)-ethylsulfanyl]-pyridin-3-yl]-3,4-difluor-benzamid;
**6** N-[2-[2-(Benzolsulfonyl)-ethylsulfanyl]-pyridin-3-yl]-3-cyclohexyl-propionamid;
**7** N-[2-[2-(Benzolsulfonyl)-ethylsulfanyl]-pyridin-3-yl]-2-thiophen-2-yl-acetamid;
**8** N-[2-[2-(Benzolsulfonyl)-ethylsulfanyl]-pyridin-3-yl]-2-(3,5-dimethyl-phenyl)-propionamid;
**9** 2-(2-Methoxyphenyl)-N-[2-[2-[[3-(trifluormethyl)phenyl]sulfonyl]-ethylsulfanyl]-pyridin-3-yl]-acetamid;
**10** 2-(4-Methoxyphenyl)-N-[2-[2-[[3-(trifluormethyl)phenyl]sulfonyl]-ethylsulfanyl]-pyridin-3-yl]-acetamid;
**11** 2-(3-Methoxyphenyl)-N-[2-[2-[[3-(trifluormethyl)phenyl]sulfonyl]-ethylsulfanyl]-pyridin-3-yl]-acetamid
**12** 2-(2-Hydroxyphenyl)-N-[2-[2-[[3-(trifluormethyl)phenyl]sulfonyl]-ethylsulfanyl]-pyridin-3-yl]-acetamid;
**13** 2-(4-Hydroxyphenyl)-N-[2-[2-[[3-(trifluormethyl)phenyl]sulfonyl]-ethylsulfanyl]-pyridin-3-yl]-acetamid;
**14** 2-Cyclopentyl-N-[2-[2-[[3-(trifluormethyl)phenyl]sulfonyl]-ethylsulfanyl]-pyridin-3-yl]acetamid;
**18** N-[2-[2-[[3-(Trifluormethyl)phenyl]sulfonyl]-ethylsulfanyl]-pyridin-3-yl]-thiophen-2-carbonsäure amid;
**19** N-[2-[2-[[3-(Trifluormethyl)phenyl]sulfonyl]-ethylsulfanyl]-pyridin-3-yl]-cyclohexancarbonsäure amid;
**20** 2-Thiophen-2-yl-N-[2-[2-[[3-(trifluormethyl)phenyl]sulfanyl]-ethylsulfanyl]-pyridin-3-yl]-acetamid;
**21** N-[2-(2-Phenylsulfanyl-ethylsulfanyl)-pyridin-3-yl]-2-thiophen-2-yl-acetamid;
**22** 2-Thiophen-2-yl-N-[2-[2-[3-(trifluormethyl)-phenoxy]-ethylsulfanyl]-pyridin-3-yl]-acetamid;
**23** 2-Thiophen-2-yl-N-[2-[3-[3-(trifluormethyl)phenyl]-propylsulfanyl]-pyridin-3-yl]-acetamid;
**24** 2-Naphthalen-2-yl-N-[2-[2-[[3-(trifluormethyl)phenyl]sulfonyl]-ethylsulfanyl]-pyridin-3-yl]-acetamid;
**25** 4-Phenyl-N-[2-[2-[[3-(trifluormethyl)phenyl]sulfonyl]-ethylsulfanyl]-pyridin-3-yl]-butyramid;
**26** 3-Thiophen-2-yl-N-[2-[2-[[3-(trifluormethyl)phenyl]sulfonyl]-ethylsulfanyl]-pyridin-3-yl]-propionamid;
**27** 3-Phenyl-N-[2-[2-[[3-(trifluormethyl)phenyl]sulfonyl]-ethylsulfanyl]-pyridin-3-yl]-propionamid;
**28** 3-Cyclopentyl-N-[2-[2-[[3-(trifluormethyl)phenyl]sulfonyl]-ethylsulfanyl]-pyridin-3-yl]-propionamid;
**29** 2-Cyclohexyl-N-[2-[2-[[3-(trifluormethyl)phenyl]sulfanyl]-ethylsulfanyl]-pyridin-3-yl]-acetamid;
**31** ((E)-3-(4-Fluorphenyl)-N-[2-[2-[[3-(trifluormethyl)phenyl]sulfonyl]-ethylsulfanyl]-pyridin-3-yl]-acrylamid:
**33** N-[2-(3-Phenyl-propylsulfanyl)-pyridin-3-yl]-2-thiophen-2-yl-acetamid;
**35** 6-Chlor-N-[2-[2-[[3-(trifluormethyl)phenyl]sulfonyl]-ethylsulfanyl]-pyridin-3-yl]-pyridin-3-carbonsäure amid;
**36** 2-Pyridin-4-yl-N-[2-[2-[[3-(trifluormethyl)phenyl]sulfonyl]-ethylsulfanyl]-pyridin-3-yl]-acetamid;
**37** 2-(3-Hydroxyphenyl)-N-[2-[2-[[3-(trifluormethyl)phenyl]sulfonyl]-ethylsulfanyl]-pyridin-3-yl]-acetamid;
**38** N-[2-[2-[[3-(Trifluormethyl)phenyl]sulfonyl]-ethylsulfanyl]-pyridin-3-yl]-tetrahydro-pyran-3-carbonsäure amid;
**39** 2-Tetrahydro-pyran-2-yl-N-[2-[2-[[3-(trifluormethyl)phenyl]sulfonyl]-ethylsulfanyl]-pyridin-3-yl]-acetamid;
**40** 2-Tetrahydro-pyran-4-yl-N-[2-[2-[[3-(trifluormethyl)phenyl]sulfonyl]-ethylsulfanyl]-pyridin-3-yl]-acetamid;
**41** 3-Cyclohexyl-N-[2-[2-[[3-(trifluormethyl)phenyl]sulfonyl]-ethylsulfanyl]-pyridin-3-yl]-propionamid;
**44** 2-Cyclohexyl-N-[2-(2-phenylsulfanyl-ethylsulfanyl)-pyridin-3-yl]-acetamid;
**45** N-[2-(2-Phenoxy-ethylsulfanyl)-pyridin-3-yl]-2-thiophen-2-yl-acetamid;
**46** 2-Pyridin-3-yl-N-[2-[2-[[3-(trifluormethyl)phenyl]sulfonyl]-ethylsulfanyl]-pyridin-3-yl]-acetamid;
**47** 3-Hydroxy-N-[2-[2-[[3-(trifluormethyl)phenyl]sulfonyl]-ethylsulfanyl]-pyridin-3-yl]-benzamid;
**48** N-[2-[2-[[3-(Trifluormethyl)phenyl]sulfonyl]-ethylsulfanyl]-pyridin-3-yl]-tetrahydro-pyran-2-carbonsäure amid;
**49** 2-Cycloheptyl-N-[2-[2-[[3-(trifluormethyl)phenyl]sulfonyl]-ethylsulfanyl]-pyridin-3-yl]-acetamid;
**50** 4-Fluor-2-methoxy-N-[2-[2-[[3-(trifluormethyl)phenyl]sulfonyl]-ethylsulfanyl]-pyridin-3-yl]-benzamid;
**51** 4-Fluor-2-hydroxy-N-[2-[2-[[3-(trifluormethyl)phenyl]sulfonyl]-ethylsulfanyl]-pyridin-3-yl]-benzamid;
**52** 2-(1,2,3,4-Tetrahydro-naphthalen-2-yl)-N-[2-[2-[[3-(trifluormethyl)phenyl]sulfonyl]-ethylsulfanyl]-pyridin-3-yl]-acetamid;
**53** 2-Hydroxy-N-[2-[2-[[3-(trifluormethyl)phenyl]sulfonyl]-ethylsulfanyl]-pyridin-3-yl]-benzamid;
**55** 2-(3-Oxo-cyclohexyl)-N-[2-[2-[[3-(trifluormethyl)phenyl]sulfonyl]-ethylsulfanyl]-pyridin-3-yl]-acetamid
**56** N-[4-Methyl-2-[2-[[3-(trifluormethyl)phenyl]sulfonyl]-ethylsulfanyl]-pyridin-3-yl]-2-thiophen-2-yl-acetamid;
**57** 3-(1,2,3,4-Tetrahydro-naphthalen-2-yl)-N-[2-[2-[[3-(trifluormethyl)phenyl]sulfonyl]-ethylsulfanyl]-pyridin-3-yl]-propionamid;
**58** 3-Cycloheptyl-N-[2-[2-[[3-(trifluormethyl)phenyl]sulfonyl]-ethylsulfanyl]-pyridin-3-yl]-propionamid;
**59** 2-(Benzo[b]thiophen-2-yl)-N-[2-[2-[[3-(trifluormethyl)phenyl]sulfonyl]-ethylsulfanyl]-pyridin-3-yl]-acetamid;
**60** 2-Pyridin-2-yl-N-[2-[2-[[3-(trifluormethyl)phenyl]sulfonyl]-ethylsulfanyl]-pyridin-3-yl]-acetamid
**61** N-[2-[2-[(4-Fluorphenyl)sulfanyl]-ethylsulfanyl]-pyridin-3-yl]-3,3-dimethyl-butyramid;
**62** 2-(5-Bicyclo[2.2.1]heptanyl)-N-[2-[2-[[3-(trifluormethyl)phenyl]sulfonyl]-ethylsulfanyl]-pyridin-3-yl]-acetamid;
**63** N-[2-[2-(4-Fluor-phenoxy)-ethylsulfanyl]-pyridin-3-yl]-3,3-dimethyl-butyramid
**64** 3,4-Difluor-N-[2-[2-(4-fluor-phenoxy)-ethylsulfanyl]-pyridin-3-yl]-benzamid;
**65** 3,4-Difluor-N-[2-[3-(4-fluorphenyl)-propylsulfanyl]-pyridin-3-yl]-benzamid;

**66** N-[2-[3-(4-Fluorphenyl)-propylsulfanyl]-pyridin-3-yl]-3,3-dimethyl-butyramid;
**67** 2-Cycloheptyl-N-[2-[2-[(4-fluorphenyl)sulfonyl]-ethylsulfanyl]-pyridin-3-yl]-acetamid;
**68** 2-Cyclohexyl-2,2-difluor-N-[2-[2-[[3-(trifluormethyl)phenyl]sulfonyl]-ethylsulfanyl]-pyridin-3-yl]-acetamid;

oder deren physiologisch verträgliche Salze.

**13.** Arzneimittel enthaltend wenigstens ein substituiertes Aminopyridin gemäß einem der Ansprüche 1 bis 12, in Form eines einzelnen Stereoisomers oder deren Mischung, der freien Verbindungen und/oder ihrer physiologisch verträglichen Salze, sowie ggf. geeignete Zusatz- und/ oder Hilfsstoffe und/oder gegebenenfalls weiterer Wirkstoffe.

**14.** Verwendung wenigstens eines substituierten Aminopyridins gemäß einem der Ansprüche 1 bis 12, jeweils in Form eines einzelnen Stereoisomers oder deren Mischung, der freien Verbindung und/oder ihrer physiologisch verträglichen Salze, zur Herstellung eines Arzneimittels zur Behandlung von Schmerz, Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

**15.** Ein substituiertes Aminopyridin gemäß einem der Ansprüche 1 bis 12 in Form eines einzelnen Stereoisomers oder deren Mischung, der freien Verbindung und/oder ihrer physiologisch verträglichen Salze zur Behandlung von Schmerz, Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

**Claims**

**1.** Substituted 2-mercapto-3-aminopyridines having the general formula (1),

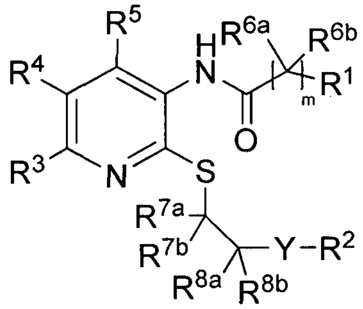

(1)

wherein
$R^1$ stands for $C_{1-6}$ alkyl, saturated or unsaturated, branched or unbranched, unsubstituted or mono- or polysubstituted; $C_{3-10}$ cycloalkyl or heterocyclyl, each saturated or unsaturated, unsubstituted or mono- or polysubstituted; aryl or heteroaryl, each unsubstituted or mono- or polysubstituted;
with the proviso that if $R^1$ denotes heterocyclyl, the binding of the heterocyclyl to the higher-order general structure takes place via a carbon atom in the heterocyclyl;
$R^2$ stands for aryl or heteroaryl, each unsubstituted or mono- or polysubstituted;
Y is selected from the group consisting of -($CR^{9a}R^{9b}$)-, $SO_2$, S(=O), -S-, -O-, C(=O);
$R^3$, $R^4$, $R^5$, $R^{6a}$, $R^{6b}$, $R^{7a}$, $R^{7b}$, $R^{8a}$, $R^{8b}$, $R^{9a}$ and $R^{9b}$ each stand independently of one another for H; F; Cl; Br; I; $NO_2$; $CF_3$; CN; OH; $OCF_3$; SH; $SCF_3$; $NH_2$; $C_{1-6}$ alkyl, O-$C_{1-6}$ alkyl, O-C(=O)-$C_{1-6}$ alkyl, S-$C_{1-6}$ alkyl, NH($C_{1-6}$ alkyl), N($C_{1-6}$ alkyl)$_2$, NH-C(=O)-$C_{1-6}$ alkyl, N(C(=O)-$C_{l-6}$ alkyl)$_2$ or C(=O)-$C_{1-6}$ alkyl, each saturated or unsaturated, branched or unbranched, unsubstituted or mono- or polysubstituted; $C_{3-7}$ cycloalkyl or heterocyclyl, each saturated or unsaturated, branched or unbranched, unsubstituted or mono- or polysubstituted;
wherein $R^{7a}$ can form with $R^{8a}$ a $C_{3-7}$ cycloalkyl radical, which can be saturated or unsaturated, unsubstituted or mono- or polysubstituted;
wherein "alkyl-substituted" stands for the substitution of one or more hydrogen atoms each independently of one

another by F; Cl; Br; I; $NO_2$; $CF_3$; CN; $C_{1-8}$ alkyl; $C_{2-8}$ heteroalkyl; aryl; heteroaryl; $C_{3-10}$ cycloalkyl; heterocyclyl; $C_{1-8}$ alkyl- or $C_{2-8}$ heteroalkyl-bridged aryl, heteroaryl, $C_{3-10}$ cycloalkyl or heterocyclyl; CHO; C(=O)$C_{1-8}$ alkyl; C(=O)aryl; C(=O)heteroaryl; $CO_2H$; C(=O)O-$C_{1-8}$ alkyl; C(=O)O-aryl; C(=O)O-heteroaryl; $CONH_2$; C(=O)NH-$C_{1-8}$ alkyl; C(=O)N($C_{1-8}$ alkyl)$_2$; C(=O)NH-aryl; C(=O)N(aryl)$_2$; C(=O)NH-heteroaryl; C(=O)N(heteroaryl)$_2$; C(=O)N($C_{1-8}$ alkyl)(aryl); C(=O)N($C_{1-8}$ alkyl)(heteroaryl); C(=O)N(heteroaryl)(aryl); OH; O-$C_{1-8}$ alkyl; $OCF_3$; O-($C_{1-8}$ alkyl)-OH; O-($C_{1-8}$ alkyl)-O-$C_{1-8}$ alkyl; O-benzyl; O-aryl; O-heteroaryl; O-C(=O)$C_{1-8}$ alkyl; O-C(=O)aryl; O-C(=O)heteroaryl; $NH_2$; NH-$C_{1-8}$ alkyl; N($C_{1-8}$ alkyl)$_2$; NH-C(=O)$C_{1-8}$ alkyl; N($C_{1-8}$ alkyl)-C(=O)$C_{1-8}$ alkyl; N(C(=O)$C_{1-8}$ alkyl)$_2$; NH-C(=O)-aryl; NH-C(=O)-heteroaryl; SH; S-$C_{1-8}$ alkyl; $SCF_3$; S-benzyl; S-aryl; S-heteroaryl; S(=O)$_2$$C_{1-8}$ alkyl; S(=O)$_2$aryl; S(=O)$_2$heteroaryl; S(=O)$_2$OH; S(=O)$_2$O-$C_{1-8}$ alkyl; S(=O)$_2$O-aryl; S(=O)$_2$O-heteroaryl; S(=O)$_2$-NH-$C_{1-8}$ alkyl; S(=O)$_2$-NH-aryl; and S(=O)$_2$-NH-$C_{1-8}$-heteroaryl;

wherein "heterocyclyl-substituted" and "cycloalkyl-substituted" stands for the substitution of one or more hydrogen atoms each independently of one another by F; Cl; Br; I; $NO_2$; $CF_3$; =O; CN; $C_{1-8}$ alkyl; $C_{2-8}$ heteroalkyl; aryl; heteroaryl; $C_{3-10}$ cycloalkyl; heterocyclyl; $C_{1-8}$ alkyl- or $C_{2-8}$ heteroalkyl-bridged aryl, heteroaryl, $C_{3-10}$ cycloalkyl or heterocyclyl; CHO; C(=O)$C_{1-8}$ alkyl; C(=O)aryl; C(=O)heteroaryl; $CO_2H$; C(=O)O-$C_{1-8}$ alkyl; C(=O)O-aryl; C(=O)O-heteroaryl; $CONH_2$; C(=O)NH-$C_{1-8}$ alkyl; C(=O)N($C_{1-8}$ alkyl)$_2$; C(=O)NH-aryl; C(=O)N(aryl)$_2$; C(=O)NH-heteroaryl; C(=O)N(heteroaryl)$_2$; C(=O)N($C_{1-8}$ alkyl)(aryl); C(=O)N($C_{1-8}$ alkyl)(heteroaryl); C(=O)N(heteroaryl)(aryl); OH; O-$C_{1-8}$ alkyl; $OCF_3$; O-($C_{1-8}$ alkyl)-OH; O-($C_{1-8}$ alkyl)-O-$C_{1-8}$ alkyl; O-benzyl; O-aryl; O-heteroaryl; O-C(=O)$C_{1-8}$ alkyl; O-C(=O)aryl; O-C(=O)heteroaryl; $NH_2$; NH-$C_{1-8}$ alkyl; N($C_{1-8}$ alkyl)$_2$; NH-C(=O)$C_{1-8}$ alkyl; N($C_{1-8}$ alkyl)-C(=O)$C_{1-8}$ alkyl; N(C(=O)$C_{1-8}$ alkyl)$_2$; NH-C(=O)-aryl; NH-C(=O)-heteroaryl; SH; S-$C_{1-8}$ alkyl; $SCF_3$; S-benzyl; S-aryl; S-heteroaryl; S(=O)$_2$$C_{1-8}$ alkyl; S(=O)$_2$ aryl; S(=O)$_2$ heteroaryl; S(=O)$_2$OH; S(=O)$_2$O-$C_{1-8}$ alkyl; S(=O)$_2$O-aryl; S(=O)$_2$O-heteroaryl; S(=O)$_2$-NH-$C_{1-8}$ alkyl; S(=O)$_2$-NH-aryl; and S(=O)$_2$-NH-$C_{1-8}$ heteroaryl;

wherein "aryl-substituted" and "heteroaryl-substituted" stands for the substitution of one or more hydrogen atoms each independently of one another by F; Cl; Br; I; $NO_2$; $CF_3$; CN; $C_{1-8}$ alkyl; or $C_{2-8}$ heteroalkyl; aryl; heteroaryl; $C_{3-10}$ cycloalkyl; heterocyclyl; $C_{1-8}$ alkyl- or $C_{2-8}$ heteroalkyl-bridged aryl, heteroaryl, $C_{3-10}$ cycloalkyl or heterocyclyl; CHO; C(=O)$C_{1-8}$ alkyl; C(=O)aryl; C(=O)heteroaryl; $CO_2H$; C(=O)O-$C_{1-8}$ alkyl; C(=O)O-aryl; C(=O)O-heteroaryl; $CONH_2$; C(=O)NH-$CH_3$; C(=O)NH-$C_2H_5$; C(=O)N($C_{1-8}$ alkyl)$_2$; C(=O)NH-aryl; C(=O)N(aryl)$_2$; C(=O)NH-heteroaryl; C(=O)N(heteroaryl)$_2$; C(=O)N($C_{1-8}$ alkyl)(aryl); C(=O)N($C_{1-8}$ alkyl)(heteroaryl); C(=O)N(heteroaryl)(aryl); OH; O-$C_{1-8}$ alkyl; $OCF_3$; O-($C_{1-8}$ alkyl)-OH; O-($C_{1-8}$ alkyl)-O-$C_{1-8}$ alkyl; O-benzyl; O-aryl; O-heteroaryl; O-C(=O)$C_{1-8}$ alkyl; O-C(=O)aryl; O-C(=O)heteroaryl; $NH_2$; NH-$C_{1-8}$ alkyl; N($C_{1-8}$ alkyl)$_2$; NH-C(=O)$C_{1-8}$ alkyl; N($C_{1-8}$ alkyl)-C(=O)$C_{1-8}$ alkyl; N(C(=O)$C_{1-8}$ alkyl)$_2$; NH-C(=O)-aryl; NH-C(=O)-heteroaryl; SH; S-$C_{1-8}$ alkyl; $SCF_3$; S-benzyl; S-aryl; S-heteroaryl; S(=O)$_2$$C_{1-8}$ alkyl; S(=O)$_2$aryl; S(=O)$_2$heteroaryl; S(=O)$_2$OH; S(=O)$_2$O-$C_{1-8}$ alkyl; S(=O)$_2$O-aryl; S(=O)$_2$O-heteroaryl; S(=O)$_2$-NH-$C_{1-8}$ alkyl; S(=O)$_2$-NH-aryl; S(=O)$_2$-NH-$C_{1-8}$ heteroaryl;

in the form of the free compounds or salts of physiologically acceptable acids or bases.

**2.** Substituted aminopyridines according to claim 1, **characterised in that** $R^1$ stands for phenyl, pyridyl or thienyl, each unsubstituted or mono- or polysubstituted with one or more substituents each selected independently of one another from the group consisting of F, Cl, Br, I, $NO_2$, $CF_3$, CN, methyl, ethyl, n-propyl, isopropyl, cyclopropyl, n-butyl, sec-butyl, tert-butyl, $CH_2CF_3$, C(=O)-methyl, C(=O)-ethyl, C(=O)-OH, C(=O)-O-methyl, C(=O)-O-ethyl, C(=O)-$NH_2$, C(=O)-N(methyl)$_2$, C(=O)-N(ethyl)$_2$, C(=O)-NH-methyl, C(=O)-NH-ethyl, C(=O)-N(methyl)(ethyl), OH, O-methyl, O-ethyl, O-($CH_2$)$_2$-O-$CH_3$, O-($CH_2$)$_2$-OH, $OCF_3$, O-C(=O)-methyl, O-C(=O)-ethyl, $NR^aR^b$, wherein $R^a$ and $R^b$ are each selected independently of one another from the group consisting of H, methyl, ethyl, ($CH_2$)$_2$-O-$CH_3$ and ($CH_2$)$_2$-OH or $R^a$ and $R^b$ together with the nitrogen atom linking them form a pyrrolidinyl, piperidinyl, 4-methylpiperazinyl or morpholinyl, NHC(=O)-methyl, NHC(=O)-ethyl, SH, $SCF_3$, S-methyl, S-ethyl, S(=O)$_2$OH, S(=O)$_2$O-methyl, benzyl, phenyl, pyridyl, wherein benzyl, phenyl, pyridyl are each unsubstituted or mono-, di- or trisubstituted with one, two or three substituents each selected independently of one another from the group consisting of F, Cl, Br, I, CN, methyl, ethyl, $CF_3$, OH, O-methyl and $OCF_3$.

**3.** Substituted aminopyridines according to claim 1 or 2, **characterised in that** $R^1$ stands for phenyl, pyridyl or thienyl, each unsubstituted or mono- or di- or trisubstituted with one, two or three substituents each selected independently of one another from the group consisting of F, Cl, Br, I, $NO_2$, $CF_3$, CN, methyl, ethyl, C(=O)-methyl, OH, O-methyl, O-($CH_2$)$_2$-O-$CH_3$, $OCF_3$, O-C(=O)-methyl, $NH_2$, NH-C(=O)-methyl, N(methyl)$_2$, morpholinyl, S-methyl, $SCF_3$, benzyl and phenyl.

**4.** Substituted aminopyridines according to one of claims 1 to 3, **characterised in that** $R^2$ stands for aryl or heteroaryl, each unsubstituted or mono- or polysubstituted with one or more substituents, each selected independently of one another from the group consisting of F, Cl, Br, I, $NO_2$, CN, OH, O-$C_{1-8}$ alkyl, $OCF_3$, $C_{1-8}$ alkyl, C(=O)-OH, $CF_3$, $NH_2$, NH($C_{1-8}$ alkyl), N($C_{1-8}$ alkyl)$_2$, SH, S-$C_{1-8}$ alkyl, $SCF_3$, S(=O)$_2$OH, benzyl, phenyl, pyridyl and thienyl, wherein benzyl, phenyl, pyridyl, thienyl can each be unsubstituted or mono- or polysubstituted with one or more substituents selected

independently of one another from the group consisting of F, Cl, Br, 1, $NO_2$, CN, OH, O-$C_{1-8}$ alkyl, $OCF_3$, $C_{1-8}$ alkyl, C(=O)-OH, $CF_3$, $NH_2$, NH($C_{1-8}$ alkyl), N($C_{1-8}$ alkyl)$_2$, SH, S-$C_{1-8}$ alkyl, $SCF_3$, S(=O)$_2$OH.

**5.** Substituted aminopyridines according to one of claims 1 to 4, **characterised in that** the radical $R^2$ stands for aryl or heteroaryl, each unsubstituted or mono-or polysubstituted with one or more substituents, each selected independently of one another from the group consisting of F, Cl, Br, I, $NO_2$, CN, OH, O-$C_{1-8}$ alkyl, $OCF_3$, $C_{1-8}$ alkyl, C(=O)-OH, $CF_3$, $NH_2$, NH($C_{1-8}$ alkyl), N($C_{1-8}$ alkyl)$_2$, SH, S-$C_{1-8}$ alkyl, $SCF_3$, S(=O)$_2$OH, benzyl, phenyl, pyridyl and thienyl, wherein benzyl, phenyl, pyridyl, thienyl can each be unsubstituted or mono- or polysubstituted with one or more substituents selected independently of one another from the group consisting of F, Cl, Br, I, $NO_2$, CN, OH, O-$C_{1-8}$ alkyl, $OCF_3$, $C_{1-8}$ alkyl, C(=O)-OH, $CF_3$, $NH_2$, NH($C_{1-8}$ alkyl), N($C_{1-8}$ alkyl)$_2$, SH, S-$C_{1-8}$ alkyl, $SCF_3$, S(=O)$_2$OH.

**6.** Substituted aminopyridines according to one of claims 1 to 5, **characterised in that** $R^{7a}$, $R^{7b}$, $R^{8a}$, $R^{8b}$, $R^{9a}$ and $R^{9b}$ each stand independently of one another for H; F; Cl; Br; I; $NO_2$; $CF_3$; $CH_2CF_3$; CN; OH; $OCF_3$, $NH_2$; $C_{1-4}$ alkyl, O-$C_{1-4}$ alkyl, O-$C_{1-4}$ alkyl-OH, O-$C_{1-4}$ alkyl-O-$CH_3$, NH-$C_{1-4}$ alkyl, N($C_{1-4}$ alkyl)$_2$, each saturated or unsaturated, branched or unbranched, unsubstituted; $C_{3-10}$ cycloalkyl, saturated or unsaturated, unsubstituted or mono- or polysubstituted with one or more substituents each selected independently of one another from the group consisting of F, Cl, Br, I, $C_{1-4}$ alkyl, OH, O-$C_{1-4}$ alkyl.

**7.** Substituted aminopyridines according to one of claims 1 to 6, **characterised in that** $R^{7a}$ forms with $R^{8a}$ a 6-membered cycloalkyl radical, which can be saturated or unsaturated, unsubstituted or mono- or polysubstituted.

**8.** Substituted aminopyridines according to one of claims 1 to 7, **characterised in that** m = 0 or 1.

**9.** Substituted aminopyridines according to one of claims 1 to 8, **characterised in that** Y stands for -($CR^{9a}R^{9b}$)-.

**10.** Substituted aminopyridines according to one of claims 1 to 8, **characterised in that** $R^3$, $R^4$ and $R^5$ each stand independently of one another for H; F; Cl; Br; I; $NO_2$; $CF_3$; CN; OH; $OCF_3$; SH; $SCF_3$; methyl; ethyl; n-propyl; isopropyl; butyl; sec-butyl; tert-butyl; $CH_2CF_3$; O-methyl; O-ethyl; O-n-propyl; O-isopropyl; 0-butyl; O-sec-butyl; O-tert-butyl; O-($CH_2$)$_2$-O-methyl; O-($CH_2$)$_2$-OH; O-(C=O)-methyl; O-(C=O)-ethyl; S-methyl; S-ethyl; cyclopropyl; cyclobutyl; $NR^aR^b$, wherein $R^a$ and $R^b$ are each selected independently of one another from the group consisting of H, methyl, ethyl, ($CH_2$)$_2$-O-methyl, ($CH_2$)$_2$-OH, (C=O)-methyl, (C=O)-ethyl or $R^a$ and $R^b$ together with the nitrogen atom linking them form a pyrrolidinyl, piperidinyl, 4-methylpiperazinyl or morpholinyl.

**11.** Substituted aminopyridines according to one of claims 1 to 10, **characterised in that**
m stands for the number 0 and $R^1$ stands for aryl or heteroaryl;
m stands for the number 1 and $R^1$ stands for thienyl, phenyl, a $C_{3-8}$ alkyl radical, which can be saturated or unsaturated, unsubstituted or mono- or polysubstituted, or a monocyclic or bicyclic $C_{3-8}$ cycloalkyl radical, which can be saturated or unsaturated (but not aromatic), unsubstituted or mono- or polysubstituted; or
m stands for the number 2 and $R^1$ stands for phenyl, cycloalkyl or alkyl.

**12.** Substituted aminopyridines according to one of claims 1 to 11, selected from the group comprising

**1** N-[2-[2-(Benzenesulfonyl)-ethylsulfanyl]-pyridin-3-yl]-2-cyclohexyl acetamide;
**2** 2-Cyclohexyl-N-[2-[2-[[3-(trifluoromethyl)phenyl]sulfonyl]-ethylsulfanyl]-pyridin-3-yl] acetamide;
**3** 2-Thiophen-2-yl-N-[2-[2-[[3-(trifluoromethyl)phenyl]sulfonyl]-ethylsulfanyl]-pyridin-3-yl] acetamide;
**4** N-[2-[2-(Benzenesulfonyl)-ethylsulfanyl]-pyridin-3-yl] benzamide;
**5** N-[2-[2-(Benzenesulfonyl)-ethylsulfanyl]-pyridin-3-yl]-3,4-difluorobenzamide;
**6** N-[2-[2-(Benzenesulfonyl)-ethylsulfanyl]-pyridin-3-yl]-3-cyclohexyl propionamide;
**7** N-[2-[2-(Benzenesulfonyl)-ethylsulfanyl]-pyridin-3-yl]-2-thiophen-2-yl acetamide;
**8** N-[2-[2-(Benzenesulfonyl)-ethylsulfanyl]-pyridin-3-yl]-2-(3,5-dimethylphenyl) propionamide;
**9** 2-(2-Methoxyphenyl)-N-[2-[2-[[3-(trifluoromethyl)phenyl]sulfonyl]-ethylsulfanyl]-pyridin-3-yl] acetamide;
**10** 2-(4-Methoxyphenyl)-N-[2-[2-[[3-(trifluoromethyl)phenyl]sulfonyl]-ethylsulfanyl]-pyridin-3-yl] acetamide;
**11** 2-(3-Methoxyphenyl)-N-[2-[2-[[3-(trifluoromethyl)phenyl]sulfonyl]-ethylsulfanyl]-pyridin-3-yl] acetamide
**12** 2-(2-Hydroxyphenyl)-N-[2-[2-[[3-(trifluoromethyl)phenyl]sulfonyl]-ethylsulfanyl]-pyridin-3-yl] acetamide;
**13** 2-(4-Hydroxyphenyl)-N-[2-[2-[[3-(trifluoromethyl)phenyl]sulfonyl]-ethylsulfanyl]-pyridin-3-yl] acetamide;
**14** 2-Cyclopentyl-N-[2-[2-[[3-(trifluoromethyl)phenyl]sulfonyl]-ethylsulfanyl]-pyridin-3-yl] acetamide;
**18** N-[2-[2-[[3-(Trifluoromethyl)phenyl]sulfonyl]-ethylsulfanyl]-pyridin-3-yl]-thiophene-2-carboxylic acid amide;
**19** N-[2-[2-[[3-(Trifluoromethyl)phenyl]sulfonyl]-ethylsulfanyl]-pyridin-3-yl]-cyclohexane carboxylic acid amide;

**20** 2-Thiophen-2-yl-N-[2-[2-[[3-(trifluoromethyl)phenyl]sulfanyl]-ethylsulfanyl]-pyridin-3-yl] acetamide;
**21** N-[2-(2-Phenylsulfanyl-ethylsulfanyl)-pyridin-3-yl]-2-thiophen-2-yl acetamide;
**22** 2-Thiophen-2-yl-N-[2-[2-[3-(trifluoromethyl)-phenoxy]-ethylsulfanyl]-pyridin-3-yl] acetamide;
**23** 2-Thiophen-2-yl-N-[2-[3-[3-(trifluoromethyl)phenyl]-propylsulfanyl]-pyridin-3-yl] acetamide;
**24** 2-Naphthalen-2-yl-N-[2-[2-[[3-(trifluoromethyl)phenyl]sulfonyl]-ethylsulfanyl]-pyridin-3-yl] acetamide;
**25** 4-Phenyl-N-[2-[2-[[3-(trifluoromethyl)phenyl]sulfonyl]-ethylsulfanyl]-pyridin-3-yl]-butyramide;
**26** 3-Thiophen-2-yl-N-[2-[2-[[3-(trifluoromethyl)phenyl]sulfonyl]-ethylsulfanyl]-pyridin-3-yl] propionamide;
**27** 3-Phenyl-N-[2-[2-[[3-(trifluoromethyl)phenyl]sulfonyl]-ethylsulfanyl]-pyridin-3-yl] propionamide;
**28** 3-Cyclopentyl-N-[2-[2-[[3-(trifluoromethyl)phenyl]sulfonyl]-ethylsulfanyl]-pyridin-3-yl] propionamide;
**29** 2-Cyclohexyl-N-[2-[2-[[3-(trifluoromethyl)phenyl]sulfanyl]-ethylsulfanyl]-pyridin-3-yl] acetamide;
**31** ((E)-3-(4-Fluorophenyl)-N-[2-[2-[[3-(trifluoromethyl)phenyl]sulfonyl]-ethylsulfanyl]-pyridin-3-yl] acrylamide;
**33** N-[2-(3-Phenyl-propylsulfanyl)-pyridin-3-yl]-2-thiophen-2-yl acetamide;
**35** 6-Chloro-N-[2-[2-[[3-(trifluoromethyl)phenyl]sulfonyl]-ethylsulfanyl]-pyridin-3-yl]-pyridine-3-carboxylic acid amide;
**36** 2-Pyridin-4-yl-N-[2-[2-[[3-(trifluoromethyl)phenyl]sulfonyl]-ethylsulfanyl]-pyridin-3-yl] acetamide;
**37** 2-(3-Hydroxyphenyl)-N-[2-[2-[[3-(trifluoromethyl)phenyl]sulfonyl]-ethylsulfanyl]-pyridin-3-yl] acetamide;
**38** N-[2-[2-[[3-(Trifluoromethyl)phenyl]sulfonyl]-ethylsulfanyl]-pyridin-3-yl]-tetrahydropyrane-3-carboxylic acid amide;
**39** 2-Tetrahydropyran-2-yl-N-[2-[2-[[3-(trifluoromethyl)phenyl]sulfonyl]-ethylsulfanyl]-pyridin-3-yl] acetamide;
**40** 2-Tetrahydropyran-4-yl-N-[2-[2-[[3-(trifluoromethyl)phenyl]sulfonyl]-ethylsulfanyl]-pyridin-3-yl] acetamide;
**41** 3-Cyclohexyl-N-[2-[2-[[3-(trifluoromethyl)phenyl]sulfonyl]-ethylsulfanyl]-pyridin-3-yl] propionamide;
**44** 2-Cyclohexyl-N-[2-(2-phenylsulfanyl-ethylsulfanyl)-pyridin-3-yl] acetamide;
**45** N-[2-(2-Phenoxy-ethylsulfanyl)-pyridin-3-yl]-2-thiophen-2-yl acetamide;
**46** 2-Pyridin-3-yl-N-[2-[2-[[3-(trifluoromethyl)phenyl]sulfonyl]-ethylsulfanyl]-pyridin-3-yl] acetamide;
**47** 3-Hydroxy-N-[2-[2-[[3-(trifluoromethyl)phenyl]sulfonyl]-ethylsulfanyl]-pyridin-3-yl] benzamide;
**48** N-[2-[2-[[3-(Trifluoromethyl)phenyl]sulfonyl]-ethylsulfanyl]-pyridin-3-yl]-tetrahydropyrane-2-carboxylic acid amide;
**49** 2-Cycloheptyl-N-[2-[2-[[3-(trifluoromethyl)phenyl]sulfonyl]-ethylsulfanyl]-pyridin-3-yl] acetamide;
**50** 4-Fluoro-2-methoxy-N-[2-[2-[[3-(trifluoromethyl)phenyl]sulfonyl]-ethylsulfanyl]-pyridin-3-yl] benzamide;
**51** 4-Fluoro-2-hydroxy-N-[2-[2-[[3-(trifluoromethyl)phenyl]sulfonyl]-ethylsulfanyl]-pyridin-3-yl] benzamide;
**52** 2-(1,2,3,4-Tetrahydronaphthalen-2-yl)-N-[2-[2-[[3-(trifluoromethyl)phenyl]sulfonyl]-ethylsulfanyl]-pyridin-3-yl] acetamide;
**53** 2-Hydroxy-N-[2-[2-[[3-(trifluoromethyl)phenyl]sulfonyl]-ethylsulfanyl]-pyridin-3-yl] benzamide;
**55** 2-(3-Oxocyclohexyl)-N-[2-[2-[[3-(trifluoromethyl)phenyl]sulfonyl]-ethylsulfanyl]-pyridin-3-yl] acetamide;
**56** N-[4-Methyl-2-[2-[[3-(trifluoromethyl)phenyl]sulfonyl]-ethylsulfanyl]-pyridin-3-yl]-2-thiophen-2-yl acetamide;
**57** 3-(1,2,3,4-Tetrahydronaphthalen-2-yl)-N-[2-[2-[[3-(trifluoromethyl)phenyl]sulfonyl]-ethylsulfanyl]-pyridin-3-yl] propionamide;
**58** 3-Cycloheptyl-N-[2-[2-[[3-(trifluoromethyl)phenyl]sulfonyl]-ethylsulfanyl]-pyridin-3-yl] propionamide;
**59** 2-(Benzo[b]thiophen-2-yl)-N-[2-[2-[[3-(trifluoromethyl)phenyl]sulfonyl]-ethylsulfanyl]-pyridin-3-yl] acetamide;
**60** 2-Pyridin-2-yl-N-[2-[2-[[3-(trifluoromethyl)phenyl]sulfonyl]-ethylsulfanyl]-pyridin-3-yl] acetamide;
**61** N-[2-[2-[(4-Fluorophenyl)sulfanyl]-ethylsulfanyl]-pyridin-3-yl]-3,3-dimethyl butyramide;
**62** 2-(5-Bicyclo[2.2.1]heptanyl)-N-[2-[2-[[3-(trifluoromethyl)phenyl]sulfonyl]-ethylsulfanyl]-pyridin-3-yl] acetamide;
**63** N-[2-[2-(4-Fluorophenoxy)-ethylsulfanyl]-pyridin-3-yl]-3,3-dimethyl butyramide;
**64** 3,4-Difluoro-N-[2-[2-(4-fluorophenoxy)-ethylsulfanyl]-pyridin-3-yl] benzamide;
**65** 3,4-Difluoro-N-[2-[3-(4-fluorophenyl)-propylsulfanyl]-pyridin-3-yl] benzamide;
**66** N-[2-[3-(4-Fluorophenyl)-propylsulfanyl]-pyridin-3-yl]-3,3-dimethyl butyramide;
**67** 2-Cycloheptyl-N-[2-[2-[(4-fluorophenyl)sulfonyl]-ethylsulfanyl]-pyridin-3-yl] acetamide;
**68** 2-Cyclohexyl-2,2-difluoro-N-[2-[2-[[3-(trifluoromethyl)phenyl]sulfonyl]-ethylsulfanyl]-pyridin-3-yl] acetamide;

or the physiologically acceptable salts thereof.

**13.** Medicament containing at least one substituted aminopyridine according to one of claims 1 to 12, in the form of an individual stereoisomer or a mixture thereof, the free compounds and/or the physiologically acceptable salts thereof, and optionally suitable additives and/or auxiliary substances and/or optionally further active ingredients.

**14.** Use of at least one substituted aminopyridine according to one of claims 1 to 12,

each in the form of an individual stereoisomer or a mixture thereof, the free compound and/or the physiologically acceptable salts thereof, for the preparation of a medicament for the treatment of pain, epilepsy, urinary incontinence, anxiety states, dependency, mania, bipolar disorders, migraine, cognitive diseases, dystonia-associated dyskinesias and/or urinary incontinence.

**15.** Substituted aminopyridine according to one of claims 1 to 12 in the form of an individual stereoisomer or a mixture thereof, the free compound and/or the physiologically acceptable salts thereof, for the treatment of pain, epilepsy, urinary incontinence, anxiety states, dependency, mania, bipolar disorders, migraine, cognitive diseases, dystonia-associated dyskinesias and/or urinary incontinence.

## Revendications

**1.** 2-mercapto-3-aminopyridines substituées de formule générale (1),

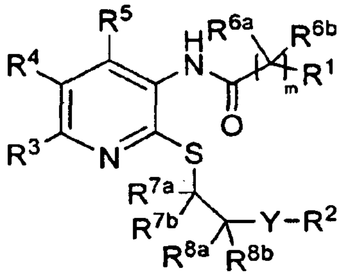

(1)

dans laquelle :

$R^1$ représente un groupe alkyle en $C_1$ à $C_6$, saturé ou insaturé, ramifié ou non ramifié, non substitué ou mono- ou polysubstitué ; cycloalkyle en $C_3$ à $C_{10}$ ou hétérocyclyle, respectivement saturé ou insaturé, non substitué ou mono-ou polysubstitué ; aryle ou hétéroaryle respectivement non substitué ou mono- ou polysubstitué ;
à la condition que, si $R^1$ représente un groupe hétérocyclyle, la liaison du groupe hétérocyclyle à la structure générale subordonnée se fasse par le biais d'un atome de carbone du groupe hétérocyclyle ;
$R^2$ représente un groupe aryle ou hétéroaryle respectivement non substitué ou mono- ou polysubstitué ;
Y est choisi dans le groupe consistant en des groupes $-(CR^{9a}R^{9b})-$, $SO_2$, S(=O), -S- , -O- , C(=O) ;
$R^3$, $R^4$, $R^5$, $R^{6a}$, $R^{6b}$, $R^{7a}$, $R^{7b}$, $R^{8a}$, $R^{8b}$, $R^{9a}$ et $R^{9b}$, représentent, respectivement indépendamment les uns des autres, H ; F ; Cl ; Br ; I ; un groupe $NO_2$ $CF_3$ ; CN ; OH ; $OCF_3$ ; SH ; $SCF_3$ ; $NH_2$ ; alkyle en $C_1$ à $C_6$, O-alkyle en $C_1$ à $C_6$, O-C(=O)alkyle en $C_1$ à $C_6$, S-alkyle en $C_1$ à $C_6$, NH(alkyle en $C_1$ à $C_6$), N(alkyle en $C_1$ à $C_6)_2$, NH-C(=O)alkyle en $C_1$ à $C_6$, N(C(=O)alkyle en $C_1$ à $C_6)_2$ ou C(=O)alkyle en $C_1$ à $C_6$, respectivement saturé ou insaturé, ramifié ou non ramifié, non substitué ou mono- ou polysubstitué ; cycloalkyle en $C_3$ à $C_7$ ou hétérocyclyle, respectivement saturé ou insaturé, ramifié ou non ramifié, non substitué ou mono- ou polysubstitué ;
$R^{7a}$ pouvant former avec $R^{8a}$ un radical cycloalkyle en $C_3$ à $C_7$, qui peut être saturé ou insaturé, non substitué ou mono- ou polysubstitué ;
dans laquelle « alkyle substitué » représente la substitution d'un ou de plusieurs atomes d'hydrogène, respectivement indépendamment les uns des autres, par F ; Cl ; Br ; I ; un groupe $NO_2$ ; $CF_3$ ; CN ; alkyle en $C_1$ à $C_8$ ; hétéroalkyle en $C_2$ à $C_8$ ; aryle ; hétéroaryle ; cycloalkyle en $C_3$ à $C_{10}$ ; hétérocyclyle ; aryle, hétéroaryle, cycloalkyle en $C_3$ à $C_{10}$ ou hétérocyclyle ponté par le biais d' un groupe alkyle en $C_1$ à $C_8$ ou hétéroalkyle en $C_2$ à $C_8$ ; CHO ; C(=O)alkyle en $C_1$ à $C_8$ ; C(=O)aryle ; C(=O)-hétéroaryle ; CONH ; C (=O) O-alkyle en $C_1$ à $C_8$ ; C(=O)O-aryle ; C(=O)O-hétéroaryle ; $CONH_2$ ; C(=O)NH-(alkyle en $C_1$ à $C_8$) ; C(=O)N(alkyle en $C_1$ à $C_8)_2$ ; C(=O)NH-aryle ; $C(=O)N(aryle)_2$ ; C(=O)NH-hétéroaryle ; C(=O)N(hétéro-aryle) $_2$ ; C(=O)N(alkyle en $C_1$ à $C_8$) (aryle) ; C(=O)N(alkyle en $C_1$ à $C_8$) (hétéroaryle) ; C(=O)N(hétéroaryle)(aryle) ; OH ; O-alkyle en $C_1$ à $C_8$ ; $OCF_3$ ; O-(alkyle en $C_1$ à $C_8$)-OH ; O-(alkyle en $C_1$ à $C_8$)-O-alkyle en $C_1$ à $C_8$ ; O-benzyle ; O-aryle ; O-hétéroaryle ; O-C(=O)alkyle en $C_1$ à $C_8$ ; O-C(=O) aryle ; O-C (=O) hétéroaryle ; $NH_2$ ; NH-alkyle en $C_1$ à $C_8$ ;

N(alkyle en $C_1$ à $C_8$)$_2$ ; NH-C (=O) alkyle en $C_1$ à $C_8$ ; N(alkyle en $C_1$ à $C_8$)-C(=O)alkyle en $C_1$ à $C_8$ ; N(C(=O)alkyle en $C_1$ à $C_8$)$_2$ ; NH-C(=O) aryle ; NH-C(=O)-hétéroaryle ; SH ; S-alkyle en $C_1$ à $C_8$ ; $SCF_3$ ; S-benzyle, S-aryle, S-hétéroaryle ; $S(=O)_2$alkyle en $C_1$ à $C_8$ ; $S(=O)_2$aryle ; $S(=O)_2$hétéroaryle $S(=O)_2OH$ ; $S(=O)_2$O-alkyle en $C_1$ à $C_8$ ; $S(=O)_2$O-aryle ; $S(=O)_2$O-hétéroaryle ; $S(=O)_2$-NH-alkyle en $C_1$ à $C_8$ ; $S(=O)_2$-NH-aryle ; et $S(=O)_2$-NH-hétéroaryle en $C_1$ à $C_8$ ;

dans laquelle « hétérocyclyle substitué » et « cycloalkyle substitué » représentent la substitution d'un ou de plusieurs atomes d'hydrogène, respectivement indépendamment les uns des autres, par F ; i Cl ; Br ; I ; un groupe $NO_2$ ; $CF_3$ ; =O ; CN ; alkyle en $C_1$ à $C_8$ ; hétéroalkyle en $C_2$ à $C_8$ ; aryle ; hétéroaryle ; cycloalkyle en $C_3$ à $C_{10}$ ; hétérocyclyle ; aryle, hétéroaryle, cycloalkyle en $C_3$ à $C_{10}$ ou hétérocyclyle ponté par le biais d'un groupe alkyle en $C_1$ à $C_8$ ou hétéroalkyle en $C_2$ à $C_8$ ; CHO ; C (=O) alkyle en $C_1$ à $C_8$ ; C (=O) aryle ; C(=O)-hétéroaryle ; $CO_2H$ ; C(=O)O-alkyle en $C_1$ à $C_8$ ; C(=O)O-aryle ; C(=O)O-hétéroaryle ; $CONH_2$ ; C(=O)NH-(alkyle en $C_1$ à $C_8$) ; C(=O)N(alkyle en $C_1$ à $C_8$)$_2$ ; C(=O)NH-aryle ; C(=O)N(aryle)$_2$ ; C(=O)NH-hétéroaryle ; C(=O)N(hétéro-aryle)$_2$ ; C(=O)N(alkyle en $C_1$ à $C_8$) (aryle) ; C (=O) N (alkyle en $C_1$ à $C_8$) (hétéroaryle) ; C(=O)N(hétéroaryle)(aryle) ; OH ; O-alkyle en $C_1$ à $C_8$ ; $OCF_3$ ; O- (alkyle en $C_1$ à $C_8$) -OH ; O-(alkyle en $C_1$ à $C_8$)-O-alkyle en $C_1$ à $C_8$ ; O-benzyle ; 0-aryle ; O-hétéroaryle ; O-C(=O)(alkyle en $C_1$ à $C_8$) ; O-C(=O)aryle ; O-C(=O)hétéroaryle ; $NH_2$ ; NH-alkyle en $C_1$ à $C_8$ ; N(alkyle en $C_1$ à $C_8$) $_2$ ; NH-C (=O) alkyle en $C_1$ à $C_8$ ; N(alkyle en $C_1$ à $C_8$) -C (=O) alkyle en $C_1$ à $C_8$ ; N (C (=O) alkyle en $C_1$ à $C_8$) $_2$ ; NH-C (=O) aryle ; NH-C(=O)-hétéroaryle ; SH ; S-alkyle en $C_1$ à $C_8$ ; $SCF_3$ ; S-benzyle, S-aryle, S-hétéroaryle ; $S(=O)_2$alkyle en $C_1$ à $C_8$ ; $S(=O)_2$aryle ; $S(=O)_2$hétéroaryle ; $S(=O)_2OH$ ; $S(=O)_2$O-alkyle en $C_1$ à $C_8$ ; $S(=O)_2$O-aryle ; $S(=O)_2$O-hétéroaryle ; $S(=O)_2$-NH-alkyle en $C_1$ à $C_8$ ; S (=O) $_2$-NH-aryle ; et $S(=O)_2$-NH-hétéroaryle en $C_1$ à $C_8$ ;

dans laquelle « aryle substitué » et « hétéroaryle substitué » représentent la substitution d'un ou de plusieurs atomes d'hydrogène, respectivement indépendamment les uns des autres, par F ; Cl ; Br ; I ; un groupe $NO_2$ ; $CF_3$ ; CN ; alkyle en $C_1$ à $C_8$ ; ou hétéroalkyle en $C_2$ à $C_8$ ; aryle ; hétéroaryle ; cycloalkyle en $C_3$ à $C_{10}$ ; hétérocyclyle ; aryle, hétéroaryle, cycloalkyle en $C_3$ à $C_{10}$ ou hétérocyclyle ponté par le biais d'un groupe alkyle en $C_1$ à $C_8$ ou hétéroalkyle en $C_2$ à $C_8$ ; CHO ; C(=O)alkyle en $C_1$ à $C_8$ ; C(=O)aryle ; C(=O)hétéroaryle ; $CO_2H$ ; C(=O)O-alkyle en $C_1$ à $C_8$ ; C(=O)O-aryle ; C(=O)O-hétéroaryle ; $CONH_2$ ; C(=O)NH-$CH_3$ ; C(=O)NH-$C_2H_5$ ; C(=O)N(alkyle en $C_1$ à $C_a$)$_2$ ; C(=O)NH-aryle ; C(=O)N(aryle)$_2$ ; C(=O)NH-hétéroaryle ; C(=O)N(hétéroaryle)$_2$ ; C(=O)N(alkyle en $C_1$ à $C_8$) (aryle) ; C(=O)N(alkyle en $C_1$ à $C_8$)(hétéroaryle) ; C(=O)N(hétéroaryle)(aryle) ; OH ; O-alkyle en $C_1$ à $C_8$ ; $OCF_3$ ; O- (alkyle en $C_1$ à $C_8$)-OH ; O-(alkyle en $C_1$ à $C_8$)-O-alkyle en $C_1$ à $C_8$ ; O-benzyle ; O-aryle ; O-hétéroaryle ; O-C(=O)alkyle en $C_1$ à $C_8$ ; O-C (=O) aryle ; O-C(=O)hétéroaryle ; $NH_2$ ; NH-alkyle en $C_1$ à $C_8$ ; N(alkyle en $C_1$ à $C_8$)$_2$ ; NH-C(=O)alkyle en $C_1$ à $C_8$ ; N(alkyle en $C_1$ à $C_8$) -C (=O) alkyle en $C_1$ à $C_8$ ; N (C (=O) alkyle en $C_1$ à $C_8$)$_2$ ; NH-C (=O) aryle ; NH-C (=O) -hétéroaryle ; SH ; S-alkyle en $C_1$ à $C_8$ ; $SCF_3$ ; S-benzyle ; S-aryle ; S-hétéroaryle ; $S(=O)_2$alkyle en $C_1$ à $C_8$ ; $S(=O)_2$aryle ; $S(=O)_2$hétéroaryle ; $S(=O)_2OH$ ; $S(=O)_2$O-alkyle en $C_1$ à $C_8$ ; $S(=O)_2$O-aryle ; $S(=O)_2$O-hétéroaryle ; $S(=O)_2$-NH-alkyle en $C_1$ à $C_8$ ; $S(=O)_2$-NH-aryle ; $S(=O)_2$-NH-hétéroaryle en $C_1$ à $C_8$ ;

sous la forme de composés libres ou de sels d'acides ou de bases physiologiquement acceptables.

**2.** Aminopyridines substituées selon la revendication 1, **caractérisées en ce que** $R^1$ représente un groupe phényle, pyridyle ou thiényle, respectivement non substitué ou mono-ou polysubstitué par un ou plusieurs substituants choisis respectivement indépendamment les uns des autres dans le groupe consistant en F, Cl, Br, I, un groupe $NO_2$, $CF_3$, CN, méthyle, éthyle, n-propyle, isopropyle, cyclopropyle, n-butyle, sec.-butyle, tertiobutyle, $CH_2CF_3$, C(=O)-méthyle, C(=O)-éthyle, C(=O)-OH, C(=O)-O-méthyle, C(=O)-O-éthyle, C(=O)-$NH_2$, C(=O)-N(méthyle)$_2$, C(=O)-N(éthyle)$_2$, C(=O)-NH-méthyle, C(=O)-NH-éthyle, C(=O)-N(méthyle)(éthyle), OH, 0-méthyle, O-éthyle, O-$(CH_2)_2$-O-$CH_3$, O-$(CH_2)_2$-OH, $OCF_3$, O-C(=O)-méthyle, O-C(=O)-éthyle, $NR^aR^b$, $R^a$ et $R^b$ étant choisis, indépendamment l'un de l'autre, dans le groupe consistant en H, un groupe méthyle, éthyle, $(CH_2)_2$-O-$CH_3$ et $(CH_2)_2$-OH ou $R^a$ et $R^b$, conjointement avec l'atome d'azote auquel ils sont reliés, forment un noyau pyrrolidinyle, pipéridinyle, 4-méthylpipérazinyle ou morpholinyle, NHC(=O)-méthyle, NHC(=O)-éthyle, SH, $SCF_3$, S-méthyle, S-éthyle, $S(=O)_2OH$, $S(=O)_2$O-méthyle, benzyle, phényle, pyridyle, où les groupes benzyle, phényle, pyridyle sont respectivement non substitués ou mono-, di- ou trisubstitués par un, deux ou trois substituants choisis, respectivement indépendamment les uns des autres, dans le groupe consistant en F, Cl, Br, I, un groupe CN, méthyle, éthyle, $CF_3$, OH, O-méthyle et $OCF_3$.

**3.** Aminopyridines substituées selon la revendication 1 ou 2, **caractérisées en ce que** $R^1$ représente un groupe phényle, pyridyle ou thiényle, respectivement non substitué ou mono- ou di- ou trisubstitués par un, deux ou trois substituants choisis respectivement indépendamment les uns des autres dans le groupe consistant en F, Cl, Br, I, un groupe $NO_2$, $CF_3$, CN, méthyle, éthyle, C(=O)-méthyle, OH, 0-méthyle, O-$(CH_2)_2$-O-$CH_3$, $OCF_3$, O-C(=O)-méthyle, $NH_2$, NH-C(=O)-méthyle, N(méthyle)$_2$, morpholinyle, S-méthyle, $SCF_3$, benzyle et phényle.

4. Aminopyridines substituées selon l'une des revendications 1 à 3, **caractérisées en ce que** $R^2$ représente un groupe aryle ou hétéroaryle, respectivement non substitué ou mono- ou polysubstitué par un ou plusieurs substituants choisis respectivement indépendamment les uns des autres dans le groupe consistant en F, Cl, Br, I, un groupe $NO_2$, CN, OH, O-alkyle en $C_1$ à $C_8$, $OCF_3$, alkyle en $C_1$ à $C_8$, C(=O)-OH, $CF_3$, $NH_2$, NH(alkyle en $C_1$ à $C_8$), N(alkyle en $C_1$ à $C_8$)$_2$, SH, S-alkyle en $C_1$ à $C_8$, $SCF_3$, $S(=O)_2OH$, benzyle, phényle, pyridyle et thiényle, où les groupes benzyle, phényle, pyridyle, thiényle peuvent être respectivement non substitués ou mono- ou polysubstitués par un ou plusieurs substituants choisis respectivement indépendamment les uns des autres dans le groupe consistant en F, Cl, Br, I, un groupe $NO_2$, CN, OH, O-alkyle en $C_1$ à $C_8$, $OCF_3$, alkyle en $C_1$ à $C_8$, C(=O)-OH, $CF_3$, $NH_2$, NH(alkyle en $C_1$ à $C_8$), N(alkyle en $C_1$ à $C_8$) $_2$, SH, S-alkyle en $C_1$ à $C_8$, $SCF_3$, $S(=O)_2OH$.

5. Aminopyridines substituées selon l'une des revendications 1 à 4, **caractérisées en ce que** le radical $R^2$ représente un groupe aryle ou hétéroaryle, respectivement non substitué ou mono- ou polysubstitué par un ou plusieurs substituants choisis respectivement indépendamment les uns des autres dans le groupe consistant en F, Cl, Br, I, un groupe $NO_2$, CN, OH, O-alkyle en $C_1$ à $C_8$, $OCF_3$, alkyle en $C_1$ à $C_8$, C(=O)-OH, $CF_3$, $NH_2$, NH(alkyle en $C_1$ à $C_8$), N(alkyle en $C_1$ à $C_8$) $_2$, SH, S-alkyle en $C_1$ à $C_8$, $SCF_3$, $S(=O)_2OH$, benzyle, phényle, pyridyle et thiényle, où les groupes benzyle, phényle, pyridyle, thiényle peuvent être respectivement non substitués ou mono- ou polysubstitué par un ou plusieurs substituants choisis respectivement indépendamment les uns des autres dans le groupe consistant en F, Cl, Br, I, un groupe $NO_2$, CN, OH, O-alkyle en $C_1$ à $C_8$, $OCF_3$, alkyle en $C_1$ à $C_8$, C(=O)-OH, $CF_3$, $NH_2$, NH(alkyle en $C_1$ à $C_8$), N(alkyle en $C_1$ à $C_8$)$_2$, SH, S-alkyle en $C_1$ à $C_8$, $SCF_3$, $S(=O)_2OH$.

6. Aminopyridines substituées selon l'une des revendications 1 à 5, **caractérisées en ce que** $R^{7a}$, $R^{7b}$, $R^{Ba}$, $R^{8b}$, $R^{9a}$ et $R^{9b}$, représentent, respectivement indépendamment les uns des autres, H ; F ; Cl ; Br ; I ; un groupe $NO_2$ ; $CF_3$ ; $CH_2CF_3$ ; CN ; OH ; $OCF_3$ ; $NH_2$ ; alkyle en $C_1$ à $C_4$, 0-alkyle en $C_1$ à $C_4$, O-alkyle en $C_1$ à $C_4$-OH, O-alkyle en $C_1$ à $C_4$-O-$CH_3$, NH(alkyle en $C_1$ à $C_4$), N (alkyle en $C_1$ à $C_4$)$_2$, respectivement saturé ou insaturé, ramifié ou non ramifié, non substitué ; cycloalkyle en $C_3$ à $C_{10}$, saturé ou insaturé, non substitué ou mono- ou polysubstitué par un ou plusieurs substituants choisis respectivement indépendamment les uns des autres dans le groupe consistant en F, Cl, Br, I, un groupe alkyle en $C_1$ à $C_4$, OH, O-alkyle en $C_1$ à $C_4$.

7. Aminopyridines substituées selon l'une des revendications 1 à 6, **caractérisées en ce que** $R^{7a}$ forme avec $R^{8a}$ un radical cycloalkyle à 6 membres, qui peut être saturé ou insaturé, non substitué ou mono- ou polysubstitué.

8. Aminopyridines substituées selon l'une des revendications 1 à 7, **caractérisées en ce que** m = 0 ou 1.

9. Aminopyridines substituées selon l'une des revendications 1 à 8, **caractérisées en ce que** Y représente un groupe -($CR^{9a}R^{9b}$)-.

10. Aminopyridines substituées selon l'une des revendications 1 à 8, **caractérisées en ce que** $R^3$, $R^4$ et $R^5$ représentent, respectivement indépendamment les uns des autres, H ; F ; Cl ; Br ; I ; un groupe $NO_2$ ; $CF_3$ ; CN ; OH ; $OCF_3$ ; SH ; $SCF_3$ ; méthyle ; éthyle ; n-propyle ; isopropyle ; butyle ; sec.-butyle ; tertiobutyle ; $CH_2CF_3$ ; O-méthyle ; O-éthyle ; O-n-propyle ; O-isopropyle ; O-butyle ; O-sec.-butyle ; O-tertiobutyle ; O-$(CH_2)_2$-O-méthyle ; O-$(CH_2)_2$-OH ; O-(C=O)-méthyle ; O-(C=O)-éthyle ; S-méthyle ; S-éthyle ; cyclopropyle ; cyclobutyle ; $NR^aR^b$, $R^a$ et $R^b$ étant choisis, indépendamment l'un de l'autre, dans le groupe consistant en H, un groupe méthyle, éthyle, $(CH_2)_2$-O-méthyle, $(CH_2)_2$-OH, (C=O)-méthyle, (C=O)-éthyle ou $R^a$ et $R^b$, conjointement avec l'atome d'azote auquel ils sont reliés, forment un noyau pyrrolidinyle, pipéridinyle, 4-méthylpipérazinyle ou morpholinyle.

11. Aminopyridines substituées selon l'une des revendications 1 à 10, **caractérisées en ce que** :

m représente le chiffre 0 et $R^1$ représente un groupe aryle ou hétéroaryle ;
m représente le chiffre 1 et $R^1$ représente un groupe thiényle, phényle, un radical alkyle en $C_3$ à $C_8$, qui peut être saturé ou insaturé, non substitué ou mono- ou polysubstitué, ou représente un radical cycloalkyle en $C_3$ à $C_8$ mono- ou bicyclique, qui peut être saturé ou insaturé (mais pas aromatique), non substitué ou mono- ou polysubstitué ; ou
m représente le chiffre 2 et $R^1$ représente un groupe phényle, cycloalkyle ou alkyle.

12. Aminopyridines substituées selon l'une des revendications 1 à 11, choisies dans le groupe consistant en :

1 N-[2-[2-(benzènesulfonyl)-éthylsulfanyl]-pyridine-3-yl]-2-cyclohexyl-acétamide ;
2 2-cyclohexyl-N-[2-[2-[[3-(trifluorométhyl)-phényl]-sulfonyl]-éthylsulfanyl]-pyridine-3-yl]-acétamide ;

3 2-thiophène-2-yl-N-[2-[2-[[3-(trifluorométhyl)-phényl]-sulfonyl]-éthylsulfanyl]-pyridine-3-yl]-acétamide ;
4 N-[2-[2-(benzènesulfonyl)-éthylsulfanyl]-pyridine-3-yl]-benzamide ;
5 N-[2-[2-(benzènesulfonyl)-éthylsulfanyl]-pyridine-3-yl]-3,4-difluoro-benzamide ;
6 N-[2-[2-(benzènesulfonyl)-éthylsulfanyl]-pyridine-3-yl]-3-cyclohexyl-propionamide ;
7 N-[2-[2-(benzènesulfonyl)-éthylsulfanyl]-pyridine-3-yl]-2-thiophène-2-yl-acétamide ;
8 N-[2-[2-(benzènesulfonyl)-éthylsulfanyl]-pyridine-3-yl]-2-(3,5-diméthylphényl)-propionamide ;
9 2-(2-méthoxyphényl)-N-[2-[2-[[3-(trifluorométhyl)-phényl]-sulfonyl]-éthylsulfanyl]-pyridine-3-yl]-acétamide ;
10 2-(4-méthoxyphényl)-N-[2-[2-[[3-(trifluorométhyl)-phényl]-sulfonyl]-éthylsulfanyl]-pyridine-3-yl]-acétamide ;
11 2-(3-méthoxyphényl)-N-[2-[2-[[3-(trifluorométhyl)-phényl]-sulfonyl]-éthylsulfanyl]-pyridine-3-yl]-acétamide ;
12 2-(2-hydroxyphényl)-N-[2-[2-[[3-(trifluorométhyl)-phênyl]-sulfonyl]-éthylsulfanyl]-pyridine-3-yl]-acétamide ;
13 2-(4-hydroxyphényl)-N-[2-[2-[[3-(trifluorométhyl)-phényl]-sulfonyl]-éthylsulfanyl]-pyridine-3-yl]-acétamide ;
14 2-cyclopentyl-N-[2-[2-[[3-(trifluorométhyl)-phényl]-sulfonyl]-éthylsulfanyl]-pyridine-3-yl]-acétamide ;
18 amide d'acide N-[2-[2-[[3-(trifluorométhyl)-phényl]-sulfonyl]-éthylsulfanyl]-pyridine-3-yl]-thiophène-2-carboxylique ;
19 amide d'acide N-[2-[2-[[3-(trifluorométhyl)-phényl]-sulfonyl]-éthylsulfanyl]-pyridine-3-yl]-cyclohexane-carboxylique ;
20 2-thiophène-2-yl-N-[2-[2-[[3-(trifluorométhyl)-phényl]-sulfanyl]-éthylsulfanyl]-pyridine-3-yl]-acétamide ;
21 N-[2-(2-phénylsulfanyl-éthylsulfanyl)-pyridine-3-yl]-2-thiophène-2-yl-acétamide ;
22 2-thiophène-2-yl-N-[2-[2-[3-(trifluorométhyl)-phénoxy]-éthylsulfanyl]-pyridine-3-yl]-acétamide ;
23 2-thiophène-2-yl-N-[2-[3-[3-(trifluorométhyl)-phényl]-propylsulfanyl]-pyridine-3-yl]-acétamide ;
24 2-naphthalène-2-yl-N-[2-[2-[[3-(trifluorométhyl)-phényl]-sulfonyl]-éthylsulfanyl]-pyridine-3-yl]-acétamide ;
25 4-phényl-N-[2-[2-[[3-(trifluorométhyl)-phényl]-sulfonyl]-éthylsulfanyl]-pyridine-3-yl]-butyramide ;
26 3-thiophène-2-yl-N-[2-[2-[[3-(trifluorométhyl)phé-nyl]sulfonyl]-éthylsulfanyl]-pyridine-3-yl]-propionamide ;
27 3-phényl-N-[2-[2-[[3-(trifluorométhyl)-phényl]-sulfonyl]-éthylsulfanyl]-pyridine-3-yl]-propionamide ;
28 3-cyclopentyl-N-[2-[2-[[3-(trifluorométhyl)-phényl]-sulfonyl]-éthylsulfanyl]-pyridine-3-yl]-propionamide ;
29 2-cyclohexyl-N-[2-[2-[[3-(trifluorométhyl)-phényl]-sulfanyl]-éthylsulfanyl]-pyridine-3-yl]-acétamide ;
31 (E)-3-(4-fluorophényl)-N-[2-[2-[[3-(trifluorométhyl)-phênyl]-sulfonyl]-éthylsulfanyl]-pyridine-3-yl]-acrylamide ;
33 N-[2-(3-phényl-propylsulfanyl)-pyridine-3-yl]-2-thiophène-2-yl-acétamide ;
35 6-chloro-N-[2-[2-[[3-(trifluoromêthyl)-phényl]-sulfonyl]-éthylsulfanyl]-pyridine-3-yl]-pyridine-3-carbonamide ;
36 2-pyridine-4-yl-N-[2-[2-[[3-(trifluorométhyl)-phênyl]-sulfonyl]-éthylsulfanyl]-pyridine-3-yl]-acétamide ;
37 2-(3-hydroxyphényl)-N-[2-[2-[[3-(trifluorométhyl)-phényl]-sulfonyl]-éthylsulfanyl]-pyridine-3-yl]-acétamide ;
38 amide d'acide N-[2-[2-[[3-(trifluorométhyl)-phényl]-sulfonyl]-éthylsulfanyl]-pyridine-3-yl]-tétrahydro-pyranne-3-carboxylique ;
39 2-tétrahydro-pyranne-2-yl-N-[2-[2-[[3-(trifluoro-mêthyl)-phényl]-sulfonyl]-êthylsulfanyl]-pyridine-3-yl]-acétamide ;
40 2-tétrahydro-pyranne-4-yl-N-[2-[2-[[3-(trifluoro-méthyl)-phényl]-sulfonyl]-éthylsulfanyl]-pyridine-3-yl]-acétamide ;
41 3-cyclohexyl-N-[2-[2-[[3-(trifluorométhyl)-phényl]-sulfonyl]-éthylsulfanyl]-pyridine-3-yl]-propionamide ;
44 2-cyclohexyl-N-[2-(2-phénylsulfanyl-éthylsulfanyl)-pyridine-3-yl]-acétamide ;
45 N-[2-(2-phénoxy-éthylsulfanyl)-pyridine-3-yl]-2-thiophène-2-yl-acétamide ;
46 2-pyridine-3-yl-N-[2-[2-[[3-(trifluorométhyl)-phényl]-sulfonyl]-éthylsulfanyl]-pyridine-3-yl]-acétamide ;
47 3-hydroxy-N-[2-[2-[[3-(trifluorométhyl)-phényl]-sulfonyl]-éthylsulfanyl]-pyridine-3-yl]-benzamide ;
48 amide d'acide N-[2-[2-[[3-(trifluorométhyl)-phényl]-sulfonyl]-éthylsulfanyl]-pyridine-3-yl]-tétrahydro-pyranne-2-carboxylique ;
49 2-cycloheptyl-N-[2-[2-[[3-(trifluorométhyl)-phényl]-sulfonyl]-éthylsulfanyl]-pyridine-3-yl]-acétamide ;
50 4-fluoro-2-méthoxy-N-[2-[2-[[3-(trifluorométhyl)-phényl]-sulfonyl]-éthylsulfanyl]-pyridine-3-yl]-benzamide ;
51 4-fluoro-2-hydroxy-N-[2-[2-[[3-(trifluorométhyl)-phényl]-sulfonyl]-éthylsulfanyl]-pyridine-3-yl]-benzamide ;
52 2-(1,2,3,4-tétrahydro-naphthalène-2-yl)-N-[2-[2-[[3-(trifluorométhyl)-phényl]-sulfonyl]-éthylsulfanyl]-pyridine-3-yl]-acétamide ;
53 2-hydroxy-N-[2-[2-[[3-(trifluorométhyl)-phényl]-sulfonyl]-éthylsulfanyl]-pyridine-3-yl]-benzamide ;
55 2-(3-oxo-cyclohexyl)-N-[2-[2-[[3-(trifluorométhyl)-phényl]-sulfonyl]-éthylsulfanyl]-pyridine-3-yl]-acétamide ;
56 N-[4-méthyl-2-[2-[[3-(trifluorométhyl)-phényl]-sulfonyl]-éthylsulfanyl]-pyridine-3-yl]-2-thiophène-2-yl-acétamide ;
57 3-(1,2,3,4-tétrahydro-naphthalène-2-yl)-N-[2-[2-[[3-(trifluorométhyl)-phényl]-sulfonyl]-éthylsulfanyl]-pyridine-3-yl]-propionamide ;
58 3-cycloheptyl-N-[2-[2-[[3-(trifluorométhyl)-phényl]-sulfonyl]-éthylsulfanyl]-pyridine-3-yl]-propionamide ;
59 2-(benzo[b]thiophène-2-yl)-N-[2-[2-[[3-(trifluoro-méthyl)-phényl]-sulfonyl]-éthylsulfanyl]-pyridine-3-yl]-acétamide ;
60 2-pyridine-2-yl-N-[2-[2-[[3-(trifluorométhyl)-phényl]-sulfonyl]-éthylsulfanyl]-pyridine-3-yl]-acétamide ;
61 N-[2-[2-[(4-fluorophényl)-sulfanyl]-éthylsulfanyl]-pyridine-3-yl]-3,3-diméthyl-butyramide ;
62 2-(5-bicyclo[2.2.1]heptanyl)-N-[2-[2-[[3-(trifluoro-méthyl)-phényl]-sulfonyl]-éthylsulfanyl]-pyridine-3-yl]-acétamide ;
63 N-[2-[2-(4-fluorophénoxy)-éthylsulfanyl]-pyridine-3-yl]-3,3-diméthyl-butyramide ;
64 3,4-difluoro-N-[2-[2-(4-fluorophénoxy)-éthylsulfanyl]-pyridine-3-yl]-benzamide ;
65 3,4-difluoro-N-[2-[3-(4-fluorophényl)-propylsulfanyl]-pyridine-3-yl]-benzamide ;
66 N-[2-[3-(4-fluorophényl)-propylsulfanyl]-pyridine-3-yl]-3,3-diméthyl-butyramide ;
67 2-cycloheptyl-N-[2-[2-[(4-fluorophényl)sulfonyl]-éthylsulfanyl]-pyridine-3-yl]-acétamide ;
68 2-cyclohexyl-2,2-difluoro-N-[2-[2-[[3-(trifluoro-méthyl)-phényl]-sulfonyl]-éthylsulfanyl]-pyridine-3-yl]-acétamide ;

ou leurs sels physiologiquement acceptables.

**13.** Médicament contenant au moins une aminopyridine substituée selon l'une des revendications 1 à 12, sous la forme d'un stéréoisomère unique ou de leur mélange, des composés libres et/ou de leurs sels physiologiquement acceptables, ainsi que contenant, le cas échéant, des additifs et/ou auxiliaires et/ou le cas échéant d'autres principes actifs.

**14.** Utilisation d'au moins une aminopyridine substituée selon l'une des revendications 1 à 12, sous la forme d'un stéréoisomère unique ou de leur mélange, du composé libre et/ou de leurs sels physiologiquement acceptables, pour la production d'un médicament pour le traitement de la douleur, de l'épilepsie, de l'incontinence urinaire, d'états d'anxiété, de la dépendance, de la manie, de troubles bipolaires, de la migraine, de maladies cognitives, de dyskinésies associées à une dystonie et/ou de l'incontinence urinaire.

**15.** Aminopyridine substituée selon l'une des revendications 1 à 12 sous la forme d'un stéréoisomère unique ou de leur mélange, du composé libre et/ou de leurs sels physiologiquement acceptables pour le traitement de la douleur, de l'épilepsie, de l'incontinence urinaire, d'états d'anxiété, de la dépendance, de la manie, de troubles bipolaires, de la migraine, de maladies cognitives, de dyskinésies associées à une dystonie et/ou de l'incontinence urinaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20020128277 A
- WO 2006092143 A
- WO 2009018466 A
- WO 2004058704 A
- WO 01010380 A
- WO 2006051311 A


### In der Beschreibung aufgeführte Nicht-Patentliteratur


- **PASSMORE et al.** *J Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Naunyn Schmfedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **GRIBKOFF.** *Expert Opin Ther Targets,* 2008, vol. 12 (5), 565-81
- **MICELI et al.** *Curr Opin Pharmacol,* 2008, vol. 8 (1), 65-74
- **STRENG et al.** *J Urol,* 2004, vol. 172, 2054-2058
- **HANSEN et al.** *Eur J Pharmacol,* 2007, vol. 570 (1-3), 77-88
- **DENCKER et al.** *Epilepsy Behav,* 2008, vol. 12 (1), 49-53
- **RICHTER et al.** *Br J Pharmacol,* 2006, vol. 149 (6), 747-53
- **BENNETT, G.J. ; XIE, Y.K.** A peripheral mononeuropathy in rat that produces disorders of pain sensation like those seen in man. *Pain,* 1988, vol. 33 (1), 87-107
- **KIM, S.H. ; CHUNG, J.M.** An experimental model for peripheral neuropathy produced by segmental spinal nerve ligation in the rat. *Pain,* 1992, vol. 50 (3), 355-363
- **DE SARRO et al.** *Naunyn-Schmiedeberg's Arch. Pharmacol.,* 2001, vol. 363, 330-336
- **J. MARCH.** Advanced Organic Chemistry. Wiley & Sons, 2007
- **F. A. CAREY ; R. J. SUNDBERG.** Advanced Organic Chemistry, Parts A and B. Springer, 2007
- **AUTORENKOLLEKTIV.** Compendium of Organic Synthetic Methods. Wiley & Sons
- Experimentelle Vorschriften können beispielweise in der Reaxys® Datenbank der Firma. Elsevier
- **DUBUISSON, D. ; DENNIS, S.G.** *Pain,* 1977, vol. 4, 161-174
- **LITCHFIELD, J.T. ; WILCOXON, J.J.** *J. Pharmacol. Exp. Ther.,* 1949, vol. 96, 99-113